(19) Europäisches Patentamt / European Patent Office / Office européen des brevets

(11) **EP 2 216 415 B2**

(12) **NEW EUROPEAN PATENT SPECIFICATION**
After opposition procedure

(45) Date of publication and mention of the opposition decision:
**04.01.2017 Bulletin 2017/01**

(45) Mention of the grant of the patent:
**26.12.2012 Bulletin 2012/52**

(21) Application number: **10150568.3**

(22) Date of filing: **30.07.2004**

(51) Int Cl.:
*C12Q 1/68* (2006.01) *C07H 21/00* (2006.01)
*C07H 21/02* (2006.01) *C07H 21/04* (2006.01)

(54) **Methods for preparing short RNA molecules**

Zusammensetzungen und Verfahren zur Aufreinigung kurzer RNA-Moleküle und anderer Nukleinsäuren

Compositions et procédés de préparation de courtes molécules d'ARN et d'autres acides nucléiques

(84) Designated Contracting States:
**AT BE BG CH CY CZ DE DK EE ES FI FR GB GR HU IE IT LI LU MC NL PL PT RO SE SI SK TR**
Designated Extension States:
**AL HR LT LV MK**

(30) Priority: **01.08.2003 US 491758 P**
**17.11.2003 US 520383 P**

(43) Date of publication of application:
**11.08.2010 Bulletin 2010/32**

(62) Document number(s) of the earlier application(s) in accordance with Art. 76 EPC:
**04779463.1 / 1 660 631**

(73) Proprietor: **Life Technologies Corporation**
**Carlsbad, CA 92008 (US)**

(72) Inventors:
- **Madden, Knut R.**
  **Carlsbad, CA 92008 (US)**
- **Harris, Adam N.**
  **Oceanside, CA 92057 (US)**
- **Hecker, Karl H.**
  **San Diego, CA 92130 (US)**
- **Lee, Byung-In**
  **Encinitas, CA 92024 (US)**

(74) Representative: **HGF Limited**
**1 City Walk**
**Leeds LS11 9DX (GB)**

(56) References cited:
**WO-A1-2005/012523** **DE-U- 20 003 080**
**US-A1- 2003 138 828** **US-B1- 6 180 778**

- **INVITROGEN LIFE TECHNOLOGIES: "CATALOG NOS. K3600-01 AND K3650-01" 29 July 2003 (2003-07-29), XP002585666 Retrieved from the Internet: URL:http://web.archive.org/web/20031208060410/http://www.invitrogen.com/content/sfs/manuals/blockit_dicer_man.pdf> [retrieved on 2008-04-28]**
- **GEHRING H H ET AL: "An improved RNA isolation method for succulent plant species rich in polyphenols and polysaccharides" PLANT MOLECULAR BIOLOGY REPORTER, NEW YORK, NY, US, vol. 18, 1 January 2000 (2000-01-01), pages 369-376, XP002353937 ISSN: 0735-9640**
- **JOHANSEN LISA K ET AL: "Silencing on the spot. Induction and suppression of RNA silencing in the Agrobacterium-mediated transient expression system" PLANT PHYSIOLOGY (ROCKVILLE), vol. 126, no. 3, July 2001 (2001-07), pages 930-938, XP002478469 ISSN: 0032-0889**
- **ANDERSON A C ET AL: "HPLC purification of RNA for crystallography and NMR." RNA (NEW YORK, N.Y.) FEB 1996, vol. 2, no. 2, February 1996 (1996-02), pages 110-117, XP002478471 ISSN: 1355-8382**
- **Lewis, A.C.: "Chromatographic Techniques" John Wiley & Sons, Ltd Encyclopedia of Life Sciences 3 May 2005 (2005-05-03), XP002585667 Chichester DOI: 10.1038/npg.els.0002705 Retrieved from the Internet: URL:http://mrw.interscience.wiley.com/emrw/9780470015902/els/article/a0002705/current/abstract> [retrieved on 2010-05-28]**

- Cuatrecasas, P., Wilchek, M.: "Affinity Chromatography" In: Lennarz, W., Lane, M., et al.: "Encyclopedia of Biological Chemistry" October 2004 (2004-10), Academic Press , New York , XP002585668 ISBN: 9780124437104 vol. 1, , pages 51-56 * the whole document *
- MYERS JASON W ET AL: "Recombinant Dicer efficiently converts large dsRNAs into siRNAs suitable for gene silencing.", NATURE BIOTECHNOLOGY MAR 2003 LNKD-PUBMED:12592410, vol. 21, no. 3, March 2003 (2003-03), pages 324-328, XP002302300, ISSN: 1087-0156
- ’Block-iT Dicer RNAi Kits’ INVITROGEN INSTRUCTION MANUAL, VERSION A (D7) 29 July 2003,

## Description

BACKGROUND OF THE INVENTION

Field of the Invention

The present invention is related to the fields of molecular biology, developmental biology, biochemistry and medicine. The invention provides methods of preparing nucleic acids, such as RNA molecules, of a defined size or range of sizes. More specifically, the invention provides compositions and methods for use in the preparation of small RNA molecules and other nucleic acids of various sizes. The invention also provides kits comprising solutions and compositions for preparing Short RNA molecules or other nucleic acids. Further provided are devices and methods for high throughput screening of nucleic acids.

Related Art

This summary is not meant to be complete but is provided only for understanding of the invention that follows. The citation of any reference herein should not be construed as an admission that such reference is available as "Prior Art" to the instant application.

Nucleic Acid Purification

Methods of purifying nucleic acids are known in the art. Such methods typically involve separating a nucleic acid of interest from other nucleic acids. The separation process is based on differences in parameters such as topology (*e.g.*, supercoiled DNA separated from linear DNA), length (in nucleotides or base pairs for, respectively, single-stranded or double-stranded nucleic acids), chemical differences, and the like. Although size differences have been used to separate nucleic acids in gels, the methods involved in recovering the separated material in solution phase are time-consuming, as the portion of the gel containing the nucleic acid of interest must be extracted and then treated to degrade the gel or otherwise extract the nucleic acid therefrom, and introduce contaminants from the gel. Such methods are also not easily adapted to high throughput (HTS) screening. The separation of small nucleic acids in solution presents other difficulties.

Methods of purifying certain types of ribonucleic acid (RNA) molecules are known in the art. Such methods include those described in the following publications.

Methods of purifying mRNA from cellular extracts are known in the art. See, *e.g.*, Chapter 7, "Extraction, Purification and Analysis of mRNA from Eukaryotic Cells" in: Molecular Cloning: A Laboratory Manual. Sambrook et al. Cold Spring Harbor Laboratory Press, Cold Spring Harbor, NY, 2001.

Methods of purifying RNA produced by *in vitro* transcription are described by Clarke in "Labeling and Purification of RNA Synthesized by In Vitro Transcription," in: RNA-Protein Interaction Protocols. Haynes, ed. Humana Press, Totowa, NJ, 1999.

Puttaraju et al., Nucleic Acids Symp Ser. 33:49-51 (1995) describe the purification of circular RNA molecules generated by a modified self-splicing intron-exon sequence.

McLaughlin et al., J Chromatogr. 418:51-72 (1987) describe the separation of complex mixtures of tRNAs using high-performance liquid chromatography.

Mandell et al., Anal Biochem 1:66 (1960) describe "MAK (methylated albumen on kieselguhr) columns" on which DNA sticks irreversibly, and various sized RNA's can be eluted with higher and higher salt concentrations (kieselguhr is diatomaceous earth). Loeser et al., Biochemistry 9:2364-6 (1970) describe the separation of 5S RNA from other nucleic acids by polyamino acid kieselguhr column chromatography. Modak et al., Anal Biochem. 34:284-6 (1970) describe a MAK column procedure for separation of RNA subtractions, [insert page 3b]

These and other methods of nucleic acid isolation are tedious and not readily adaptable to certain applications, such as high throughput screening (HTS) and the purification of small nucleic acids, such as Short RNA (as defined herein). The present invention fulfills this need by providing methods, compositions and kits for the preparation of small nucleic acids. The methods and compositions may also be adapted for use in HTS applications.

RNA Interference

One example of a methodology that would benefit from methods of preparing relatively pure small nucleic acids is RNA interference (RNAi). RNAi was originally described as a naturally-occuring process in the model organism *C. elegans* (Fire et al., Nature 391:806-811, 1998). In brief, the process involves application of double stranded RNA (dsRNA) that represents a complementary sense and antisense strand of a portion of a target gene within the region that encodes mRNA, with the result being posttranscriptional down-regulation of the target gene.

Initially, RNAi technology did not appear to be readily applicable to mammalian systems. This is because, in mammals, dsRNA activates dsRNA-activated protein kinase (PKR) resulting in an apoptotic cascade and cell death (Der et al, Proc. Natl. Acad. Sci. USA 94:3279-3283, 1997). In addition, it has long been known that dsRNA activates the interferon cascade in mammalian cells, which can also lead to altered cell physiology (Colby et al, Annu. Rev. Microbiol. 25:333, 1971; Kleinschmidt et al., Annu. Rev. Biochem. 41:517, 1972; Lampson et al., Proc. Natl. Acad. Sci. USA 58L782, 1967; Lomniczi et al., J. Gen. Virol. 8:55, 1970; and Younger et al., J. Bacteriol. 92:862, 1966). However, dsRNA-mediated activation of the PKR and interferon cascades requires dsRNA longer than about 30 base pairs. In contrast, dsRNA less than 30 base pairs in length has been demonstrated to cause RNAi in mammalian US 6 180 778 B1 discloses a method for the separation of double-stranded nucleic acids from single-stranded nucleic acids in a sample using a lysing solution containing guanidine and ethanol at 10 to 50% final concentration to bind nucleic acids differentially to a silica matrix. Fig. 1 shows binding of nearly a 100% of RNA to the matrix at an ethanol concentration of around 30%. The size of the single-stranded RNA is 0.9 kb while the double-stranded DNA is a pTZ plasmid of around 2.800 bp. The two purification steps involving the silica matrix serve to bind either double-stranded nucleic acids or single-stranded nucleic acids. Throughout the examples of D2 whole genomic DNA and whole RNA is isolated. The document is silent regarding nucleic acids smaller than 100 base pairs or nucleotides.

Gehrig et al. (Plant Molecular Biology Reporter, 2000, 18, 369-376) discloses a method for isolation of RNA from plant tissue using as the first column a Qiagen shredder column which does not bind any nucleic acids but serves only to homogenise the sample and a silica based second column which serves to bind total RNA However, the Document does not teach the separation of high molecular weight RNA from RNA molecules smaller than 100 nt.

Johansen et al. (Plant Physiol., 2001, 2, 110-117) discloses method for the purification of small DNA molecules. It involves differential elution from a Qiagen column with binding and elution buffers of unknown composition.

Meyers et al. (Nature Biotech.2003, 21, 324-328) teaches the purification of small RNA by gel purification or a series of three spin columns involving a G-25 column (Amersham), an EZ-pure column (Millipore), and a Microcon-100 column (Millipore). cells (Caplen et al., Proc. Natl. Acad. Sci. USA 98:9742-9747, 2001). Thus, it is expected that undesirable, non-specific effects associated with longer dsRNA molecules can be avoided by preparing Short RNA that is substantially free from longer dsRNAs.

The biochemistry of RNAi involves generation of active small interfering RNA (siRNA) through the action of a ribonuclease, DICER, which digests long double stranded RNA molecules (dsRNA) into shorter fragments. The small interfering RNAs (siRNAs) produced through the action of DICER mediate degradation of the complementary homologous RNA. Since the primary products of DICER are 21-23 base pair fragments of dsRNA, one can circumvent the adverse or undesired mammalian responses to dsRNA and still elicit an interfering RNA effect via siRNA (Elbashir et al., Nature 411:494-498, 2001) if the "DICING" reaction goes to completion. Incomplete "DICING," however, results in a mixture of longer RNA molecules, which may trigger undesirable and/or non-specific responses, along with the desired 21-23 bp RNA (i.e., diced siRNA or d-siRNA) molecules. It is thus desirable to separate Short RNA of the desired narrow size range (from about 21 to about 23) from other dsRNA (e.g., dsRNA substrate, incompletely "diced" dsRNA, contaminating RNA, and the like) in order to prepare d-siRNA compositions having a higher specific RNAi activity.

Another enzyme that has been used to catalyze the *in vitro* processing of long dsRNA substrates to shorter siRNA molecules is RNase III, particularly prokaryotic RNase III, *e.g., Escherichia coli* RNase III (Yang et al., Proc Natl Acad Sci USA 99: 9942-7, 2002; Calegari et al., Proc Natl Acad Sci USA 99:14236-40, 2002). Complete digestion of dsRNA with RNase III results in Short RNA averaging from about 12 to about 15 bp in length, but these short dsRNA molecules have been reported to not be as effective at triggering an RNAi response in mammalian cells (Paddison et al., Proc. Natl. Acad. Sci. USA 99:1443-8, 2002). Limited RNase III digestion of dsRNA is used to obtain Short RNA having a length of from about 20 to about 25 bp. These Short RNA molecules, which have been called-endoribonuclease-prepared siRNA (e-siRNA) molecules, mediate RNAi in mammalian cells (Yang et al., Proc Natl Acad Sci USA 99: 9942-7, 2002). However, as the RNase III reaction is not allowed to go to completion, some unreacted dsRNA may be present, as well as shorter, inactive RNA products. Both of these are undesirable as they can reduce the specific activity of the desired e-siRNA products.

The final, desired d-siRNA or e-siRNA end-products of RNase (Dicer or RNase III, respectively) digestion of a dsRNA substrate, and siRNA molecules formed by annealing two oligonucleotides to each other, typically have the following general structure, which includes both double-stranded and single-stranded portions:

```
                                          |-m-|      (Overhang)
          |------x-------|                           ("Core")
     5'-XXXXXXXXXXXXXNNNNN-3'
        : : : : : : : : : : : : :
3'-NNNNNYYYYYYYYYYYYY-5'
   |-n-|                                             (Overhang)
```

Wherein N, X and Y are nucleotides; X hydrogen bonds to Y; ":" signifies a hydrogen bond between two bases; x is a natural integer having a value between 1 and about 100; and m and n are whole integers having, independently, values between 0 and about 100. In some embodiments, N, X and Y are independently A, G, C and T or U. Non-naturally occurring bases and nucleotides can be present, particularly in the case of synthetic siRNA (*i.e.*, the product of annealing two oligonucleotides). The double-stranded central section is called the "core" and has base pairs (bp) as units of measurement; the single-stranded portions are overhangs, having nucleotides (nt) as units of measurement. The overhangs shown are 3' overhangs, but molecules with 5' overhangs are also within the scope of the invention. Also within the scope of the invention are siRNA molecules with no overhangs (*i.e.*, m = 0 and n = 0), and those having an overhang on one side of the core but not the other (*e.g.*, m = 0 and n ≥ 1, or vice-versa).

In some embodiments of the invention, the siRNA that is desired to be prepared has 3' overhangs having from about 1 to about 5 nt. In these and other embodiments, the siRNA comprises a core having from about 10 to about 30 bp; from about 15 to about 25 bp; or 16, 17, 18, 19, 20, 21, 22, 23, 24, 25, 26, 27, 28, or 29 bp.

There is a need for methods, compositions and kits by which one can prepare nucleic acids, particularly small nucleic acids, more particularly Short RNA (as defined herein) including without limitation micro-RNA, siRNA, d-siRNA and e-siRNA. There is also a need for devices (*e.g.*, filter blocks) comprising such compositions that can be used for high throughput screening of nucleic acids, particularly small nucleic acids, more particularly Short RNA molecules.

## SUMMARY OF THE INVENTION

The present invention is defined in the claims, and provides methods and compositions for preparing one or more Short RNA molecules

The term "preparing one or more nucleic acids" is meant to encompass methods in which a composition comprising a population of nucleic acids is treated in order to produce a composition comprising a subpopulation of nucleic acids. In general, the subpopulation is defined by differences in size among the population of nucleic acids, although other characteristics may also be of interest. Other characteristics include, by way of non-limiting example, the chemical structure of the nucleic acid (*i.e.*, the chemical differences between DNA, RNA and PNA, as well as chemical modifications of nucleic acids); secondary structure (*e.g.*, stem-loop sequences, double-strandedness vs. single-strandedness, circular vs. linear); topology (supercoiled vs. relaxed); and the like.

As an example of the meaning of the term "preparing a nucleic acid", in the phrase "preparing Short RNA", the term "preparing" includes but is not limited to (i) fractionating a sample to produce fractions thereof that comprise a given size or range of sizes of RNA molecules, (ii) enriching for RNA molecules of a given size or range of sizes, (iii) separating RNA molecules of a given size or range of sizes from other components of a biochemical reaction (*e.g.*, enzymes, buffer components such as salts, cofactors and/or unreacted substrates, including by way of non-limiting example unreacted nucleic acids), (iv) purifying one or more Short RNA molecules of a given size or range of sizes, and (v) isolating RNA molecules of a given size or range of sizes.

The method is described in the following sections in the context of purifying short (21-23 bp) double-stranded siRNA molecules (d-siRNA) from a reaction in which a longer template dsRNA has been treated with an enzyme (an RNase) that cleaves the template dsRNA into the short d-siRNA molecules. The RNase is selected from the group consisting of ribonuclease A, nuclease S1, ribonuclease T1, RNase III, and DICER.

In particular embodiments of the invention, the RNase is DICER. In these exemplary embodiments, the desired Short siRNA (d-siRNA) has a length of from about 16 bp to about 30 bp, preferably from about 20 to about 25 bp, and most preferably from 21 bp to 23 bp. The desired Short d-siRNA is desirably separated from (i) the DICER enzyme, (ii) reaction mix components (salts, triphosphates, etc.), (iii) non-nucleic acid products resulting from digestion of the RNA (bases, sugars, etc.), (iv) unreacted (template) dsRNA, and (v) partially reacted dsRNA. The last of these undesirable components is often the most difficult to separate from the desired 21-23 bp d-siRNA, as they can be as small as about 30 bp in length.

In particular embodiments of the invention, the RNase is a member of the RNase III family of ribonucleases (Lamontagne et al., Curr Issues Mol Biol. 3:71-78, 2001), including without limitation a prokaryotic RNase III (*e.g.*, RNase III from *E. coli*). In these exemplary embodiments, the desired Short RNA (e-siRNA) has a length of from about 18 bp to about 28 bp, and most preferably from 20 to 25 bp. The desired Short e-siRNA is desirably separated from (i) the RNase III enzyme, (ii) reaction mix components (salts, triphosphates, etc.), (iii) non-nucleic acid products resulting from digestion of the RNA (bases, sugars, etc.), (iv) unreacted (template) dsRNA, (v) partially reacted dsRNA roughly equal to or greater than about 30 bp in length, and (vi) over-digested RNA products equal to or less than about 18 bp. The last two of these undesirable components is often the most difficult to separate from the desired 21-23 bp d-siRNA.

In some embodiments, the preparative methods of the present invention utilize affinity columns that comprise one or more glass fiber segments.

The method for purifying short RNA molecules from about 18 to about 50 nucleotides or base pairs in length comprises:

(a) adding a first fluid mixture or, in any order, fluids or combinations of fluids that contain the components of said first fluid mixture, to a sample comprising said short RNA molecules and other nucleic acids having a size other than said short RNA molecules to produce a first binding mixture,
wherein the first fluid mixture or fluids or combinations of fluids that contain the components of the fluid mixture, comprises guanidine isothiocyanate, and
wherein the first binding mixture comprises m% (v/v) ethanol or isopropanol wherein m is any whole integer from 30 to 45;
(b) filtering said first binding mixture through a first silica based column, wherein said short RNA molecules pass through a composition within the first column, and said other nucleic acids are retained, to produce a first flow-through solution;
(c) adding a second fluid mixture or, in any order, fluids or combinations of fluids that contain the components of said second fluid mixture to said first flow-through solution, to produce a second binding mixture,
wherein the second binding mixture comprises m% (v/v) ethanol or isopropanol wherein m is any whole integer from 65 to 75;
(d) filtering the second binding mixture through a second silica based column, wherein said short RNA molecules bind to a composition within the second column and, optionally, washing the bound nucleic acids; and
(e) eluting the said short RNA molecules with a third fluid mixture, to produce an eluate, wherein said short RNA molecules are present in said eluate.

One or more of the affinity columns can, but need not, comprise one or more glass fiber segments.

The 2-column modality is particularly useful for the purification of nucleic acids prepared by RNase digestion in vitro. In general, a long dsRNA molecule is prepared by methods known in the art, and is treated with RNase in order to generate a reaction mixture comprising, among other things, Short dsRNA molecules. This reaction mixture is a non-limiting example of a sample comprising Short RNA, and the undesirable partial products that are preferably removed therefrom include unincorporated nucleotides and unhybridized oligonucleotides.

As a non-limiting example, a substrate dsRNA that is about 500 bp long is separated from diced Short RNA molecules. As another example, the method is used to separate a completely diced RNA fragment (e.g., a 22-mer) from incompletely diced Short RNA molecules (e.g., 44-mers, 66-mers, 88-mers, etc.). Small molecules (e.g., nucleotides removed from RNA molecules by DICER, salts, ions, nucleotides and mono-, di- and tri-phosphates thereof) and proteins (enzymes, e.g., ribonucleases), and components of enzyme "stop solutions" can also be separated from the desired Short RNA molecules. A "stop solution" for DICER or another RNase may comprise EDTA, EGTA or some other chelating agent, KCl, and/or an RNase inhibitor (such as RNasin® Ribonuclease Inhibitor from Promega, Madison, WI).

Optionally, the method further comprises (f) precipitating the RNA in the eluate with an alcohol with a coprecipitant such as, for example, glycogen, which is, as explained above, preferred in some instances and embodiments. The precipitated RNA can be dried and stored or resuspended in a buffer.

In this and various other embodiments of the invention, the first and second affinity columns are identical; in other embodiments, they are different.

In modalities involving alcohol gradient fractionation, the methods comprise:

(a) adding a first fluid mixture to a nuclease reaction mix, to produce a first binding mixture, wherein the first fluid mixture is essentially free of alcohol;
(b) filtering the first binding mixture through a first affinity column, wherein longer RNA molecules in the first binding mixture (i.e., RNA having a length of about "y" bp to about "z" or more bp) bind to, and shorter RNA molecules (i.e., RNA having a length of about 5 bp to about "y" bp) pass through, a composition within the first affinity column, to produce a first flow-through solution comprising RNA having a length of about 5 bp to about "y" bp;
(c) eluting the longer RNA from the first affinity column, to produce a first eluate comprising longer RNA molecules having lengths in the range of about "y" bp to about "z" or more bp;
(d) adding one or more alcohol(s) to the first flow-through solution to bring the final concentration to about 10% (v/v), to produce a second binding mixture;

(e) filtering the second binding mixture through a second affinity column, wherein longer RNA molecules in the second binding mixture (*i.e.*, RNA having a length of about "x" bp to about "y" or more bp) bind to, and shorter RNA molecules (*i.e.*, RNA having a length of about 5 bp to about "x" bp) pass through, a composition within the second affinity column, to produce a second flow-through solution comprising RNA having a length of about 5 bp to about "x" bp;

(f) eluting the longer RNA from the second affinity column, to produce a second eluate comprising longer RNA molecules having lengths in the range of about "x" bp to about "y" bp;

(g) adding one or more alcohol(s) to the second flow-through solution to bring the final concentration to about 10% (v/v) more than the first flow-through solution, to produce a third binding mixture;

(h) filtering the third binding mixture through a third affinity column, wherein longer RNA molecules in the second binding mixture (*i.e.*, RNA having a length of about "w" bp to about "x" bp) bind to, and shorter RNA molecules (*i.e.*, RNA having a length of about 5 bp to about '"w" bp) pass through, a composition within the third affinity column, to produce a third flow-through solution comprising RNA having a length of about 5 bp to about "w" bp; and

(i) eluting the longer RNA from the second affinity column, to produce a third eluate comprising longer RNA molecules having lengths in the range of about "w" bp to about "x" bp;

wherein "w" < "x" < "y" < "z", and wherein said first eluate comprises RNA molecules having an average length greater than that of the RNA molecules in the second eluate, and the second eluate comprises RNA molecules having an average length greater than that of the RNA molecules in the third eluate.

In some embodiments, steps (g) through (i) are repeated for as many cycles as is necessary to achieve the desired degree of size-based fractionation. In this modality, the % (v/v) alcohol in each flow-through solution is increased by about 10% until RNA of the desired (short) length is retained by and then eluted from an affinity column. Additionally or alternatively, the alcohol may be added at more discrete concentrations (*e.g.*, about 5%, followed by about 10%, followed by about 15%, etc.) in order to produce a series of eluates comprising RNA molecules, each eluate having narrower ranges of length (bp) than eluates produced with less discrete concentrations of alcohol.

Optionally, the method further comprises precipitating the RNA in the eluate with an alcohol as described above. The precipitated RNA can be dried and stored or can be resuspended in a buffer.

In various embodiments of this aspect of the invention, the first and/or second affinity column(s) are glass fiber columns.

In various embodiments of this and other aspects of the invention, isopropanol is substituted for ethanol.

The methods, kits and compositions of the invention can be used to separate a monomeric form of a Short RNA molecule from a solution comprising both monomeric and multimeric forms of the Short RNA molecule. For example, an RNase may be a processive enzyme that cuts fragments of short length (*e.g.*, 20-25 nt or bp) from a longer template RNA molecule. In such a situation, if the RNase reaction does not go to completion, a series of incompletely digested template RNA molecules is generated.

For example, an RNA template comprising 5 repeats of a Short RNA sequence will yield, if its digestion is incomplete, the desired monomeric form as well as multimeric forms (*e.g.*, dimers, trimers, tetramers and the template RNA, which can be thought of as a "5-mer"). The methods, compositions and kits of the invention are used to separate monomers from the multimers. For example, a Short RNA molecule 21 bp in length is separated from incompletely digested (multimeric) molecules, *i.e.*, Short RNA molecules having lengths of 42, 63, 84, or 105 bp; a Short RNA 22 bp in length is separated from Short RNA molecules having lengths of 44, 66, 88, or 110 bp; and a Short RNA 23 bp in length is separated from Short RNA molecules having lengths of 46, 69, 92, or 115 bp.

Once separated from template and incompletely digested RNA molecules, the monomeric form of the Short RNA molecules have enhanced activity. Non-limiting examples of enhanced activity of Short RNA molecules include greater specificity (*i.e.*, the regulation of the target sequences and genes occurs with less background and/or fewer spurious effects); higher specific activity (whereby a lower dose of the purified Short RNA molecule is required to achieve the same result as with a higher dose of unpurified Short RNA molecules); reduced toxicity on the subcellular, cellular and/or organismal level; increased stability *in vitro* or *in vivo,* which may include an enhanced shelf life; and the like. The present invention also provides kits useful for carrying out the methods of the invention.

Other preferred embodiments of the present invention will be apparent to one or ordinary skill in light of the following drawings and description of the invention, and of the claims.

BRIEF DESCRIPTION OF THE DRAWINGS

Figure 3 shows the application of the "2 column" modality of the invention to siRNA. Panel A: Schematic of purification method. Panel B: a 20% polyacrylamide TBE gel showing (i) 10 bp ladder (leftmost lane); (ii) dsRNA Dicer substrate, unpurified dicing reactions, partially purified and purified d-siRNA molecules for GFP (lanes 2-5); (iii) dsRNA Dicer substrate, unpurified dicing reactions, partially purified and purified d-siRNA molecules for luc (lanes 6-9), and (iv) synthetic siRNA (lane 10).

Figure 4 shows the "alcohol gradient fractionation" modality of the invention applied to short dsDNA molecules in a DNA "ladder" (L). Panel A: Schematic of purification method. Ethanol concentrations are systematically increased in flow-through before binding to subsequent columns. Panel B: 20% polyacrylamide TBE gel showing 10 bp ladder (L) and eluates from Micro-to-Midi RNA purification columns bound with the percent ethanol indicated (i.e., 0%, 10%, 20%, 30%, 40%, 50%, 60%, 70% and 80% EtOH).

Figure 5 shows a Western analysis for detecting an endogenous gene (lamin A/C) in cells contacted with siRNA targeted to lamin A/C or control siRNA targeted to lacZ.

Figure 8 shows a flow digram illustrating the d-siRNA purification process.

Figures 19A-19C show: 19A) gel analysis results of crude lacZ siRNA, siRNA purified using the two-column protocol, various fractions of the single-column purification protocol, as well as chemically synthesized siRNA analyzed on a 4% E-Gel, which were used for functional testing; 19B) measurements of luciferase activities after transfection of cells with lacZ siRNA; 19C) measurements of β-galactosidase activities after transfection of cells with lacZ siRNA

Figures 20A-20B show purification of siRNA generated with Dicer and RNaseIII.

Figures 23A-23B show determination of column capacity and recovery efficiency. A) Recovery of tRNA after binding to the column matrix with a single 100-μl or two 50-μl elutions. B) Recovery of a 1-kb dsRNA fragment after binding to the column matrix with a single 100-μl or two 50-μl elutions.

## DETAILED DESCRIPTION OF THE INVENTION

### I. Definitions

Unless defined otherwise, all technical and scientific terms used herein have the same meanings as commonly understood by one of ordinary skill in the art to which this invention belongs.

As used herein, the term "animal" is meant to include any animal, including but not limited to worms *(e.g.*, C. *elegans*), insects (including but not limited to, *Drosophila* spp., *Trichoplusa* spp., and *Spodoptera* spp.), fish, reptiles, amphibians, birds (including, but not limited to chickens, turkeys, ducks, geese and the like), marsupials, mammals and the like. Mammals include without limitation cats, large felines (lions, tigers, etc.), dogs, wolves, mice, rats, rabbits, deer, mules, bears, cows, pigs, horses, oxen, zebras, elephants, primates, and humans.

As used herein, the term "gene" refers to a nucleic acid comprising an open reading frame encoding a polypeptide (a structural gene), or a sequence that is the reverse complement of a gene product that is a nucleic acid, typically an RNA molecule (including without limitation ribosomal RNA, tRNA, Micro-RNAs and the like), including both exon and (optionally) intron sequences.

As used herein, the term "regulation of gene expression" refers to the act of controlling the ability of a gene to produce a biologically active protein. Regulation may result in increased expression of a gene, decreased expression of a gene or maintenance of expression of a gene, as described herein.

As used herein, the term "plurality" refers to more than one.

As used herein when referring to any numerical value, the term "about" means a value of ±10% of the stated value. For example, "about 50°C encompasses a range of temperatures from 45°C to 55°C, inclusive; similarly, "about 100 mM" encompasses a range of concentrations from 90 mM to 110 mM, inclusive.

A liquid solution that is "substantially free of" a substance comprises less than about 5 to 10% of the substance, preferably less than about 1 to 5%, more preferably less than about 0.1 to 1%, most preferably less than about 0.1%, by volume. A solid that is "substantially free of" a substance comprises less than about 5% of the substance, preferably less than about 1%, more preferably less than about 0.1 %, by weight.

The terms "separate", "isolate" and "purify" have the following meanings herein. A compound of interest is said to have been separated from a mixture of other compounds if the separation process results in the mixture being enriched for the compound of interest or substantially free of at least one of the other compounds. Separation can be partial (e.g., as in fractionation). Purification signifies that the compound of interest is substantially free of other, chemically dissimilar types of compounds; for example, nucleic acids are purified from mixtures comprising proteins, lipids, carbohydrates, etc. Isolation results in a compound that is in pure form, *i.e.*, free or substantially free from all other compounds, whether chemically similar or not. It should be noted that these processes are not mutually exclusive and need not occur in any particular order or association linked. For example, an isolated compound of interest can be prepared by separating the compound from, e.g., 10 other compounds in a mixture; if the compound of interest is separated from compounds of different types, it is said to have been purified (or partially purified). Separation, followed by various degrees of purification, is one way to effect isolation of a compound of interest. However, distinct steps are not always used, as it may be possible to prepare in some instances to isolate an isolated compound from a mixture of compounds in a single step. The term "preparing" includes but is not limited to separating, isolating, purifying, enriching and fractionating, whether performed as a method *per se,* a step in a method, or in combination with other methods.

A "weak buffer" is a buffer that has low electrical conductivity and/or low ionic strength. Conductivity is reciprocal of electrical resistivity, which may be measured using a conductivity meter including without limitation commercially meters such as those sold by Hanna Instruments (Bedfordshire, U.K.), ICM (Hillsboro, OR), and Orion meters (MG Scientific, Pleasant Prairie, WI).

As used herein, "low electrical conductivity" indicates a conductivity of from about 0.1 mS.cm-1 to about 1,000 mS.cm-1; from about 0.1 mS.cm-1 to about 500 mS.cm-1; from about 0.1 mS.cm-1 to about 250 mS.cm-1; from about 0.1 mS.cm-1 to about 100 mS.cm-1; from about 0.1 mS.cm-1 to about 50 mS.cm-1; from about 0.1 mS.cm-1 to about 10 mS.cm-1; from about 0.1 mS.cm-1 to about 5 mS.cm-1; from about 0.1 mS.cm-1 to about 1 mS.cm-1; and from about 0.1 mS.cm-1 to about 0.5 mS.cm-1.

Ionic strength (I) is calculated according to the following formula and rules:

$$\underline{I} = \tfrac{1}{2} \sum_{i} z_i^2 \, [x_i]$$

wherein $z_i$ is the charge on the ion *i* at a molar concentration $[X_i]$. Uncharged species do not contribute to ionic strength. If the solution comprises more than one type of salt or buffering species, the ionic strength contributions of each species must be summed in order to determine I for the solution.

As used herein, "low ionic strength" indicates an ionic strength of from about 1 micromho/cm to about 10,000 micromho/cm; from about 1 micromho/cm to about 5,000 micromho/cm; from about 1 micromho/cm to about 1,000 micromho/cm; from about 1 micromho/cm to about 500 micromho/cm; from about 1 micromho/cm to about 100 micromho/cm; from about 1 micromho/cm to about 50 micromho/cm; from about 1 micromho/cm to about 10 micromho/cm; or from about 1 micromho/cm to about 5 micromho/cm.

Examples of weak buffers include without limitation TE buffer (10 mM Tris-HCl and 1 mM EDTA), 2xTE buffer (20 mM Tris-HCl and 2 mM EDTA), 3xTE buffer (30 mM Tris-HCl and 3 mM EDTA), TE-plus buffer (from about 30 to about 100 mM Tris-HCl, and from about 1 to about 10 mM EDTA), and from about 2-fold to about 100-fold dilutions thereof. Another example is phosphate-buffered saline (PBS) buffer (*e.g.*, from about 0.7% to about 01% NaCl and from about 1 to about 10 mM sodium phosphate), such as D-PBS (Dulbecco's Phosphate-Buffered Saline), and from about 2-fold to about 100-fold dilutions thereof. Such buffers preferably have a pH of from about 6 to about 8, from about 6.5 to about 7.5, or about 6.0, about 6.1, about 6.2, about 6.3, about 6.4, about 6.5, about 6.6, about 6.7, about 6.8, about 6.9, about 7.0, about 7.1, about 7.2, about 7.3, about 7.4, about 7.5, about 7.6, about 7.7, about 7.8, about 7.9, or about 8.0.

## II. Exemplary Embodiments

The present invention provides methods of separating, isolating and/or purifying Short RNA molecules. In various embodiments, the RNA molecules are double-stranded (ds). RNAi molecules are one type of Short RNA molecule, and typically comprise from 18 to 22 bp. That is, the Short RNA may comprise 18, 19, 20, 21, 22, 23, 24, 25, 26, 27, 28, 29, 30, or 31 bp. Preferred Short RNA molecules comprise 19 to 23 bp. Particularly preferred are Short RNA molecules generated by the action of an enzyme called DICER; these Short RNA molecules typically comprise 21, 22 or 23 bp. A Short RNA molecule may comprise both double-stranded (ds) and single-stranded (ss) portions. It should be understood that the invention can be practiced in such a manner so as to yield Short RNA having a narrow range of sizes (*e.g.*, 21-23 bp), or to yield RNA having a broader range. The range of sizes of RNA that can be prepared according to the invention can be described as from about "q" to about "r" bp, wherein "q" is any whole integer ≥ 10 and "r" is any whole integer > 15, with the proviso that q > r, q < 1,000,000 and r < 1,000,000. Thus, the range of nucleic acids that are prepared according to the invention can range, by way of non-limiting example, from about 30 to about 40 bp, about 50 bp,

In various embodiments of the invention, the Short RNA molecules that are desirably prepared result from the enzymatic digestion of a larger template nucleic acid. For example, a dsRNA template molecule of 230 bp may be treated to produce ten Short RNA molecules, each of which comprises 23 bp. Enzymes suitable for use in digesting RNA molecules into a plurality of fragments, include, but are not limited to ribonucleases such as DICER, ribonuclease A, nuclease S1, ribonuclease T1, and the like. In particular embodiments, DICER is selected as the digestion enzyme to produce a plurality of Short RNA molecules. Ribonucleases particularly useful in practicing the invention include, without limitation, those described in Table 1 (entitled "Non-limiting Examples of Ribonucleases") and members of the RNase III family of ribonucleases (for reviews, see Lamontagne et al., Curr Issues Mol Biol. 3:71-78, 2001; Conrad et al., Int JBiochem Cell Biol. 34:116-29, 2002; and Srivastava et al., Indian JBiochem Biophys. 33:253-60, 1996).

TABLE 1: NON-LIMITING EXAMPLES OF RIBONCLEASES

| | ENZYME | ORGANISM | CITATION / SOURCE / ACCESSION NOS. |
|---|---|---|---|
| | DICER | *S. pombe* | Provost *et al.*, 2002a (i) |
| | DICER | *Giardia intestinalis* | Accession No.<br><br>gi\|27652061\|gb\|AA017549.1\|[27 652061] |
| | DICER (a.k.a. CARPEL FACTORY, SHORT INTEGUMENTS1; SUSPENSOR1; CARPEL FACTORY; DCL1) | *Arabidopsis thaliana* | Park *et al.,* 2002 (ii); Golden *et al.,* 2002 (iii); Schauer *et al.,* 2002 (iv); Accession No. (v) |
| | DICER (a.k.a. K12H4.8; dcr-1) | *Caenorhabditis elegans* | Ketting *et al.,* 2001 (vi); Accession Nos.<br><br>gi\|25145329\|ref\|NP_501019.2\|[25 145329]; gi\|17552834\|ref\|NP_498761.1\|[17 552834]; gi\|17539846\|ref\|NP_501018.1\|[17 539846]; and gi\|21431882\|sp\|P34529\|DCR1_C AEEL[21431882] |
| | DICER | *Mus musculus* | Nicholson *et al.,* 2002 (vii); Accession Nos.<br>gi\|22507359\|ref\|NP_683750.1\|[22 507359]; gi\|28522452\|ref\|XP_127160.3\|[28 522452]; gi\|19072784\|gb\|AAL84637.1 \|AF4 84523_1[19072784]; gi\|24418363\|sp\|Q8R418\|DICE_M OUSE[24418363]; gi\|22830885\|dbj\|BAC15765.1\|[22 830885]; and gi\|20385913\|gb\|AAM21495.1\|AF |
| | | | 430845_1[20385913] |
| | DICER | *Mus musculus x Mus spretus* | Accession No.<br><br>gi\|19072786\|gb\|AAL84638.1\|AF4 84524_1[19072786] |
| | DICER | *Rattus norvegicus* | Accession Nos.<br><br>gi\|27719453\|ref\|XP_235831.1\|[27 719453]; gi\|27668581\|ref\|XP_216776.1\|[27 668581 ] |
| | DICER | *Drosophila melanogaster* | Bernstein *et al.*, 2001 (viii); Accession Nos.<br><br>gi\|16215719\|dbj\|BAB69959.1\|[16 215719] |
| | DICER | *Homo sapiens* | Matsuda *et al.*(ix); Accession No. gi\|29294651\|ref\|NP_803187.1\|[29 294651]; gi\|29294649\|ref\|NP_ 085124.2\|[ 29294649; gi\|24418367\|sp\|Q9UPY3\|DICE _HUMAN[24418367] |
| | DICER | Human -recombinant | Provost *et al.,* 2002b (x); Meyers, 2003 (xi) Kawasaki *et al.,* 2003 (xii) |
| | RNase III | *Escherichia coli* | Yang *et al.,* 2002 (xiii) |
| | RNase III | *Mus musculus* | Fortin *et al.,* 2002 (xiv) |
| | RNase III | *Rhodobacter capsulatus* | Rauhut et al., 1996 (xv) |

References and notes for Table 1: (i) Provost et al., Proc Natl Acad Sci USA 99:16648-53, 2002; (ii) Park et al., Curr Biol 12:1484-95, 2002; (iii) Golden et al., Plant Physiol. 130:808-22, 2002; (iv) Schauer et al., Trends Plant Sci.

7:487-91, 2002; (v) Accession Nos. for the Entrez Nucleotides database, which is a collection of sequences from several sources, including the GenBank, RefSeq, and PDBGenBank databases, which can be accessed on-line at http://www.ncbi.nlm.nih.gov/entrez/query.fcgi; (vi) Ketting et al., Genes Dev. 15:2654-9, 2001; (vii) Nicholson et al., Mamm Genome 13:67-73, 2002; (viii) Bernstein et al., Nature 409:363-6, 2001; (ix) Matsuda et al., Biochim Biophys Acta 1490:163-9, 2000; (x) Provost et al., EMBO J. 21:5864-74, 2002; (xi) Myers et al., Nat Biotechnol. 21:324-8, 2003; (xii) Kawasaki et al., Nucleic Acids Res. 31:981-7, 2003; (xiii) Yang et al., Proc Natl Acad Sci USA 99:9942-9947, 2002; (xiv) Fortin et al., BMC Genomics 3:26, 2002; and Rauhut et al., Nucleic Acids Res. 24:1246-1251, 1996.

Affinity columns that may be used in the methods of the invention include, but are not limited to glass fiber RNA purification columns (*e.g.*, such as those in the Micro-to-Midi Total RNA Purification kits, Invitrogen Corp., Carlsbad, CA),

Alcohols that may be used in the present invention include, ethanol; isopropanol; . Combinations of alcohols can also be used. In some applications, including but not limited to those in which it is desirable to keep the volume of the sample low, isopropanol is preferably used in place of ethanol.

In some embodiments, the alcoholic solution is an azeotrope, a liquid mixture of two or more substances that retains the same composition in the vapor state as in the liquid state when distilled or partially evaporated under a certain pressure. An azeotrope is thus a mixture that has its own unique boiling point that is different (lower) than those of its components. For example, an azeotrope of ethanol and water comprises about 4% water and about 96% ethanol, depending on the pressure; under ambient conditions, the ethanol:water azeotrope is about 4.4% water and about 95.6% ethanol. As another example, an azeotrope of isopropanol and water comprises about 13% water and about 87% isopropanol, depending on the pressure; under ambient conditions, the isopropanol:water azeotrope is about 12.6% water and about 87.4% ethanol. As is known in the art, the composition of an azeotrope may change depending on what other compounds are present in the solution. For example, a mixture of a buffer with ethanol may form an azeotrope having a composition different from about 4% water and about 96% ethanol. Other parameters (*e.g.*, atmospheric pressure) may also influence the composition of any given azeotrope. One skilled in the art will be able to determine, either empirically or by calculation using known formulae, the composition of a specific azeotrope under any particular set of circumstances, and will also be able to prepare azeotropes directly (*e.g.*, by distillation).

In some embodiments of the invention, the first fluid mixture comprises ethanol. In other particular embodiments, the second fluid mixture comprises ethanol.

In various embodiments, the first and/or second fluid mixtures may also comprise a suitable physiologic buffer. Physiologic buffers that may be used in the methods of the present invention include, but are not limited to, those comprising saline, Tris-(hydroxymethyl)aminomethane-HCl (TRIS-HCl), Ethylene-diaminetetraacetic acid (EDTA) disodium salt, Phosphate Buffered Saline (PBS), N-(2-Hydroxyethyl)piperazine-N'-(2-ethanesulfonic acid) (HEPES), 3-(N-Morpholino)propanesulfonic acid (MOPS), 2-bis(2-Hydroxyethylene)amino-2-(hydroxymethyl)-1,3-propanediol (bis-TRIS), potassium phosphate ($KPO_4$), sodium phosphate ($NaPO_4$), dibasic sodium phosphate ($Na_2HPO_4$), monobasic sodium phosphate ($NaH_2PO_4$), monobasic sodium potassium phosphate ($NaKHPO_4$), magnesium phosphate ($Mg_3(PO_4)_2$-$4H_2O$), potassium acetate ($CH_3COOH$), D(+)-$\alpha$-sodium glycerophosphate ($HOCH_2CH(OH)CH_2OPO_3Na_2$) and other physiologic buffers known to those skilled in the art. These first and second fluid mixtures may also comprise additional reagents including, but not limited to, guanidine isothiocyanate, [3-mercaptoethanol and other reducing agents, and the like.

In various embodiments of the methods of the invention, the first fluid mixture comprises isopropanol. In other embodiments, the second fluid mixture comprises isopropanol, In various embodiments, the first and/or second fluid mixtures may also comprise a suitable physiologic buffer and/or additional reagents as described above.

The third fluid mixture used in the preparative methods of the invention is substantially free of alcohol (*e.g.*, ethanol or isopropanol). In various embodiments, the third fluid mixture will not contain any alcohol. In additional embodiments, the third fluid mixture may contain a weak and/or dilute physiologic buffer. Suitable physiologic buffers include those described above.

In preferred embodiments, the third fluid mixture will be a weak buffer as that term is herein described.

Varying the ethanol concentrations at each binding step will cause different sized Short RNA molecules to bind or pass through the membranes. These concentrations can be optimized to obtain Short RNA molecules of a preferred purity and/or concentration.

Suitable digestion enzymes for use in this embodiment of the invention include, but are not limited to DICER, ribonuclease A, nuclease S1, ribonuclease T1, and the like. In particular embodiments, DICER is selected as the digestion enzyme to produce a plurality of Short RNA molecules, including but not limited to RNAi molecules.

Affinity columns that may be used in this embodiment of the invention include, but are not limited to glass fiber RNA purification columns (Micro-to-Midi Total RNA Purification kit, Invitrogen Corp., Carlsbad, California), and the like. In other embodiments of this method of the invention, isopropanol, may be substituted for ethanol.

Elution of Short RNA molecules and solutions through the affinity columns may occur simply via gravity, may be facilitated though the use of a centrifuge to spin the columns, or by positive or negative (vacuum) pressure. In embodiments using glass fiber filters, centrifugation and/or positive or negative pressure are preferred.

In certain embodiments of the invention, step (h), which may be described as a "washing" step, may be repeated multiple times (*i.e.* two, five, 10, 20, etc. times) prior to eluting the bound Short RNA molecules with water or a dilute buffer in step (i). In other embodiments of the invention, the isolation methods do not have to comprise the RNA digestion step (b) as described above and may simply comprise the isolation of Short RNA molecules from a plurality of RNAi molecules.

In suitable embodiments of the invention, the methods are used to prepare Short RNA molecules for use as interfering RNA. Suitable nucleic acid molecules are Short RNA molecules, including without limitation RNAi molecules, which can be separated, isolated and/or purified using the methods of the present invention.

III. Short RNA Molecules and Other Nucleic Acids

As used herein, the term "nucleic acids" (which is used herein interchangeably and equivalently with the term "nucleic acid molecules") refers to nucleic acids (including DNA, RNA, and DNA-RNA hybrid molecules) that are isolated from a natural source; that are prepared *in vitro,* using techniques such as PCR amplification or chemical synthesis; that are prepared *in vivo, e.g.,* via recombinant DNA technology; or that are prepared or obtained by any appropriate method. Nucleic acids used in accordance with the invention may be of any shape (linear, circular, etc.) or topology (single-stranded, double-stranded, linear, circular, supercoiled, torsional, nicked, etc.). The term "nucleic acids" also includes without limitation nucleic acid derivatives such as peptide nucleic acids (PNAs) and polypeptide-nucleic acid conjugates; nucleic acids having at least one chemically modified sugar residue, backbone, internucleotide linkage, base, nucleotide, nucleoside, or nucleotide analog or derivative; as well as nucleic acids having chemically modified 5' or 3' ends; and nucleic acids having two or more of such modifications. Not all linkages in a nucleic acid need to be identical.

Nucleic acids can be synthesized *in vitro,* prepared from natural biological sources (*e.g.*, cells, organelles, viruses and the like), or collected as an environmental or other sample. Examples of nucleic acids include without limitation oligonucleotides (including but not limited to antisense oligonucleotides), ribozymes, aptamers, polynucleotides, artificial chromosomes, cloning vectors and constructs, expression vectors and constructs, gene therapy vectors and constructs, PNA (peptide nucleic acid) DNA and RNA.

RNA includes without limitation rRNA, mRNA, and Short RNA. As used herein, the term "Short RNA" encompasses RNA molecules described in the literature as "tiny RNA" (Storz, Science 296:1260-3, 2002; Illangasekare et al., RNA 5:1482-1489, 1999); prokaryotic "small RNA" (sRNA) (Wassarman et al., Trends Microbiol. 7:37-45, 1999); eukaryotic "noncoding RNA (ncRNA)"; "micro-RNA (miRNA)"; "small non-mRNA (snmRNA)"; "functional RNA (fRNA)"; "transfer RNA (tRNA)"; "catalytic RNA" [*e.g.*, ribozymes, including self-acylating ribozymes (Illangaskare et al., RNA 5: 1482-1489, 1999]; "small nucleolar RNAs (snoRNAs)"; "tmRNA" (a.k.a. "10S RNA", Muto et al., Trends Biochem Sci. 23:25-29, 1998; and Gillet et al., Mol Microbiol. 42:879-885, 2001); RNAi molecules including without limitation "small interfering RNA (siRNA)", "endoribonuclease-prepared siRNA (e-siRNA)", "short hairpin RNA (shRNA)", and "small temporally regulated RNA (stRNA)"; "diced siRNA(d-siRNA)", and aptamers, oligonucleotides and other synthetic nucleic acids that comprise at least one uracil base.

III.A. Oligonucleotides

As used in the present invention, an oligonucleotide is a synthetic or biologically produced molecule comprising a covalently linked sequence of nucleotides which may be joined by a phosphodiester bond between the 3' position of the pentose of one nucleotide and the 5' position of the pentose of the adjacent nucleotide. As used herein, the term "oligonucleotide" includes natural nucleic acid molecules (*i.e.*, DNA and DNA) as well as non-natural or derivative molecules such as peptide nucleic acids, phosphorothioate-containing nucleic acids, phosphonate-containing nucleic acids and the like. In addition, oligonucleotides of the present invention may contain modified or non-naturally occurring sugar residues (*e.g.*, arabinose) and/or modified base residues. The term oligonucleotide encompasses derivative molecules such as nucleic acid molecules comprising various natural nucleotides, derivative nucleotides, modified nucleotides or combinations thereof. Oligonucleotides of the present invention may also comprise blocking groups which prevent the interaction of the molecule with particular proteins, enzymes or substrates.

Oligonucleotides include without limitation RNA, DNA and hybrid RNA-DNA molecules having sequences that have minimum lengths of e nucleotides, wherein "e" is any whole integer from about 2 to about 15, and maximum lengths of about f nucleotides, wherein "f" is any whole integer from about 2 to about 200. In general, a minimum of about 6 nucleotides, preferably about 10, and more preferably about 12 to about 15 nucleotides, is desirable to effect specific binding to a complementary nucleic acid strand.

In general, oligonucleotides may be single-stranded (ss) or double-stranded (ds) DNA or RNA, or conjugates (*e.g.*, RNA molecules having 5' and 3' DNA "clamps") or hybrids (*e.g.*, RNA:DNA paired molecules), or derivatives (chemically modified forms thereof). Single-stranded DNA is often preferred, as DNA is less susceptible to nuclease degradation than RNA. Similarly, chemical modifications that enhance the specificity or stability of an oligonucleotide are preferred in some applications of the invention.

Certain types of oligonucleotides are of particular utility in the compositions and complexes of the present invention, including but not limited to RNAi molecules, antisense oligonucleotides, ribozymes, and aptamers.

III.A.1. Antisense Oligonucleotides

Nucleic acid molecules suitable for use in the present invention include antisense oligonucleotides. In general, antisense oligonucleotides comprise nucleotide sequences sufficient in identity and number to effect specific hybridization with a preselected nucleic acid. Antisense oligonucleotides are generally designed to bind either directly to mRNA transcribed from, or to a selected DNA portion of, a targeted gene, thereby modulating the amount of protein translated from the mRNA or the amount of mRNA transcribed from the gene, respectively. Antisense oligonucleotides may be used as research tools, diagnostic aids, and therapeutic agents.

Antisense oligonucleotides used in accordance with the present invention typically have sequences that are selected to be sufficiently complementary to the target mRNA sequence so that the antisense oligonucleotide forms a stable hybrid with the mRNA and inhibits the translation of the mRNA sequence, preferably under physiological conditions. It is preferred but not necessary that the antisense oligonucleotide be 100% complementary to a portion of the target gene sequence. However, the present invention also encompasses the production and use of antisense oligonucleotides with a different level of complementarity to the target gene sequence, *e.g.*, antisense oligonucleotides that are at least about g% complementary to the target gene sequence, wherein g is any whole integer from 50 to 100. In certain embodiments, the antisense oligonucleotide hybridizes to an isolated target mRNA under the following conditions: blots are first incubated in prehybridization solution (5x SSC; 25 mM $NaPO_4$, pH 6.5; 1x Denhardt's solution; and 1% SDS) at 42°C for at least 2 hours, and then hybridized with radiolabelled cDNA probes or oligonucleotide probes (1x 106 cpm/ml of hybridization solution) in hybridization buffer (5x SSC; 25 mM $NaPO_4$, pH 6.5; 1x Denhardt's solution; 250 ug/ml total RNA; 50% deionized formamide; 1% SDS; and 10% dextran sulfate). Hybridization for 18 hours at 30-42°C is followed by washing of the filter in 0.1-6x SSC, 0.1% SDS three times at 25-55°C. The hybridization temperatures and stringency of the wash will be determined by the percentage of the GC content of the oligonucleotides in accord with the guidelines described by Sambrook et al. (Molecular Cloning: A Laboratory Manual, 2nd edition, 1989, Cold Spring Harbor Laboratory Press, Plainview, New York), including but not limited to Table 11.2 therein.

Representative teachings regarding the synthesis, design, selection and use of antisense oligonucleotides include without limitation U.S. Patent No. 5,789,573, Antisense Inhibition of ICAM-1, E-Selectin, and CMV IE1/IE2, to Baker et al.; U.S. Patent No. 6,197,584, Antisense Modulation of CD40 Expression, to Bennett *et al.;* and Ellington, 1992, Current Protocols in Molecular Biology, 2nd Ed., Ausubel et al., eds., Wiley Interscience, New York, Units 2.11 and 2.12.

III.A.2. Ribozymes

Nucleic acid molecules suitable for use in the present invention also include ribozymes. In general, ribozymes are RNA molecules having enzymatic activities usually associated with cleavage, splicing or ligation of nucleic acid sequences. The typical substrates for ribozymes are RNA molecules, although ribozymes may catalyze reactions in which DNA molecules (or maybe even proteins) serve as substrates. Two distinct regions can be identified in a ribozyme: the binding region which gives the ribozyme its specificity through hybridization to a specific nucleic acid sequence (and possibly also to specific proteins), and a catalytic region which gives the ribozyme the activity of cleavage, ligation or splicing. Ribozymes which are active intracellularly work in cis, catalyzing only a single turnover, and are usually self-modified during the reaction. However, ribozymes can be engineered to act in trans, in a truly catalytic manner, with a turnover greater than one and without being self-modified. Owing to the catalytic nature of the ribozyme, a single ribozyme molecule cleaves many molecules of target RNA and therefore therapeutic activity is achieved in relatively lower concentrations than those required in an antisense treatment (see published PCT patent application WO 96/23569).

Representative teachings regarding the synthesis, design, selection and use of ribozymes include without limitation U.S. Patent No. 4,987,071 (RNA ribozyme polymerases, dephosphorylases, restriction endoribonucleases and methods) to Cech *et al.;* and U.S. Patent No. 5,877,021 (B7-1 Targeted Ribozymes) to Stinchcomb *et al.*; the disclosures of all of which are incorporated herein by reference in their entireties.

3. Nucleic Acids for RNAi (RNAi Molecules)

Nucleic acid molecules suitable for use in the present invention also include nucleic acid molecules, particularly oligonucleotides, useful in RNA interference (RNAi). In general, RNAi is one method for analyzing gene function in a sequence-specific manner. For reviews, see Tuschl, Chembiochem. 2:239-245 (2001), and Cullen, Nat Immunol. 3: 597-599 (2002). RNA-mediated gene-specific silencing has been described in a variety of model organisms, including nematodes (Parrish et al., Mol Cell 6:1077-1087, 2000; Tabara et al., Cell 99:123-132, 1999); in plants, *i.e.,* "co-suppression" (Napoli et al., Plant Cell 2:279-289, 1990) and posttranscriptional or homologous gene silencing (Hamilton et al., Science 286:950-952, 1999; Hamilton et al., EMBO J 21:4671-4679, 2002) (PTGS or HGS, respectively) in plants; and in fungi, *i.e.,* "quelling" (Romano et al., Mol Microbiol 6:3343-3353, 1992). Examples of suitable interfering RNAs include siRNAs, shRNAs and stRNAs. As one of ordinary skill will readily appreciate, however, other RNA molecules having analogous interfering effects are also suitable for use in accordance with this aspect of the present invention.

III.A.3.a. Small Interfering RNA (siRNA)

RNAi is mediated by double stranded RNA (dsRNA) molecules that have sequence-specific homology to their "target" mRNAs (Caplen et al., Proc Natl Acad Sci USA 98:9742-9747, 2001). Biochemical studies in Drosophila cell-free lysates indicates that the mediators of RNA-dependent gene silencing are 21-25 nucleotide "small interfering" RNA duplexes (siRNAs). Accordingly, siRNA molecules are advantageously used in the compositions, complexes and methods of the present invention. The siRNAs are derived from the processing of dsRNA by an RNase known as DICER (Bernstein et al., Nature 409:363-366, 2001). It appears that siRNA duplex products are recruited into a multi-protein siRNA complex termed RISC (RNA Induced Silencing Complex). Without wishing to be bound by any particular theory, it is believed that a RISC is guided to a target mRNA, where the siRNA duplex interacts sequence-specifically to mediate cleavage in a catalytic fashion (Bernstein et al., Nature 409:363-366, 2001; Boutla et al., Curr Biol 11: 1776-1780, 2001).

RNAi has been used to analyze gene function and to identify essential genes in mammalian cells (Elbashir et al., Methods 26:199-213, 2002; Harborth et al., J Cell Sci 114:4557-4565, 2001), including by way of non-limiting example neurons (Krichevsky et al., Proc Natl Acad Sci USA 99:11926-11929, 2002). RNAi is also being evaluated for therapeutic modalities, such as inhibiting or block the infection, replication and/or growth of viruses, including without limitation poliovirus (Gitlin et al., Nature 418:379-380, 2002) and HIV (Capodici et al., J Immunol 169:5196-5201, 2002), and reducing expression of oncogenes (*e.g.*, the bcr-abl gene; Scherr et al., Blood Sep 26 epub ahead of print, 2002). RNAi has been used to modulate gene expression in mammalian (mouse) and amphibian *(Xenopus)* embryos (respectively, Calegari et al., Proc Natl Acad Sci USA 99:14236-14240, 2002; and Zhou, et al., Nucleic Acids Res 30:1664-1669; 2002), and in postnatal mice (Lewis et al., Nat Genet 32:107-108, 2002), and to reduce trangsene expression in adult transgenic mice (McCaffrey et al., Nature 418:38-39, 2002). Methods have been described for determining the efficacy and specificity of siRNAs in cell culture and *in vivo* (see, *e.g.*, Bertrand et al., Biochem Biophys Res Commun 296: 1000-1004, 2002; Lassus et al., Sci STKE 2002(147):PL13, 2002; and Leirdal et al., Biochem Biophys Res Commun 295:744-748, 2002).

Molecules that mediate RNAi, including without limitation siRNA, can be produced *in vitro* by chemical synthesis (Hohjoh, FEBS Lett 521:195-199, 2002), hydrolysis of dsRNA (Yang et al., Proc Natl Acad Sci USA 99:9942-9947, 2002), by *in vitro* transcription with T7 RNA polymerase (Donzeet et al., Nucleic Acids Res 30:e46, 2002; Yu et al., Proc Natl Acad Sci USA 99:6047-6052, 2002), and by hydrolysis of double-stranded RNA using a nuclease such as E. coli RNase III (Yang et al., Proc Natl Acad Sci USA 99:9942-9947, 2002).

References regarding siRNA: Bernstein et al., Nature 409:363-366, 2001; Boutla et al., Curr Biol 11:1776-1780, 2001; Cullen, Nat Immunol. 3:597-599, 2002; Caplen et al., Proc Natl Acad Sci USA 98:9742-9747, 2001; Hamilton et al., Science 286:950-952, 1999; Nagase et al., DNA Res. 6:63-70, 1999; Napoli et al., Plant Cell 2:279-289, 1990; Nicholson et al., Mamm. Genome 13:67-73, 2002; Parrish et al., Mol Cell 6:1077-1087, 2000; Romano et al., Mol Microbiol 6:3343-3353, 1992; Tabara et al., Cell 99:123-132, 1999; and Tuschl, Chembiochem. 2:239-245, 2001.

III.A.3.b. Short Hairpin RNAs (shRNAs)

Paddison et al. (Genes & Dev. 16:948-958, 2002) have used small RNA molecules folded into hairpins as a means to effect RNAi. Accordingly, such short hairpin RNA (shRNA) molecules are also advantageously used in the methods, compositions and kits of the invention. The length of the stem and loop of functional shRNAs varies; stem lengths can range anywhere from about 25 to about 30 nt, and loop size can range between 4 to about 25 nt without affecting silencing activity. While not wishing to be bound by any particular theory, it is believed that these shRNAs resemble the dsRNA products of the DICER RNase and, in any event, have the same capacity for inhibiting expression of a specific gene.

IILA.3.c. Small Temporally Regulated RNAs (stRNAs)

Another group of small RNAs suitable for use in the compositions, complexes and methods of the present invention are the small temporally regulated RNAs (stRNAs). In general, stRNAs comprise from about 20 to about 30 nt (Banerjee et al., Bioessays 24:119-129, 2002). Unlike siRNAs, stRNAs downregulate expression of a target mRNA after the initiation of translation without degrading the mRNA.

III.A.3.d. Design and Synthesis of siRNA, shRNA, stRNA, Antisense and Other Oligonucleotides

One or more of the following guidelines may be used in designing the sequence of siRNA and other nucleic acids designed to bind to a target mRNA, *e.g.*, shRNA, stRNA, antisense oligonucleotides, ribozymes, and the like, that are advantageously used in accordance with the present invention.

In the sequence of the target mRNA, select a region located from about 50 to about 100 nt 3' from the start codon. In this region, search for the following sequences: AA(N19)TT or AA(N21), where N = any nucleotide. The GC content of the selected sequence should be from about 30% to about 70%, preferably about 50%. In order to maximize the specificity of the RNAi, it may be desirable to use the selected sequence in a search for related sequences in the genome of interest; sequences absent from other genes are preferred. The secondary structure of the target mRNA may be determined or predicted, and it may be preferable to select a region of the mRNA that has little or no secondary structure, but it should be noted that secondary structure seems to have little impact on RNAi. When possible, sequences that bind transcription and/or translation factors should be avoided, as they might competitively inhibit the binding of an siRNA, shRNA or stRNA (as well as other antisense oligonucleotides) to the mRNA. Thus, in general, it is preferred to select regions that do not overlap the start codon, and to also avoid the 5' and 3' untranslated regions (UTRs) of an mRNA transcript.

Nucleic acids that mediate RNAi may be synthesized in vitro using methods to produce oligonucleotides and other nucleic acids, as is described elsewhere herein, and as described in published international Patent Application No. WO 02/061034; U.S. Provisional Patent Application No. 60/254,510, filed December 8, 2000; U.S. Provisional Patent Application No. 60/326,092, filed September 28, 2001; U.S. Patent Application No. 10/014,128, filed December 7, 2001; and U.S. Provisional Patent Application No. 60/520,946, filed November 17, 2003, entitled "Compositions and Methods for Rapidly Generating Recombinant Nucleic Acid Molecules," attorney docket No. INVIT1290-3; the disclosures of which applications are incorporated by reference herein in their entireties. In addition, dsRNA and other molecules that mediate RNAi are available from commercial vendors, such as Ribopharma AG (Kulmach, Germany), Eurogentec (Seraing, Belgium) and Sequitur (Natick, MA). Eurogentec offers siRNA that has been labeled with fluorophores (*e.g.*, HEX/TET; 5' Fluorescein, 6-FAM; 3' Fluorescein, 6-FAM; Fluorescein dT internal; 5' TAMRA, Rhodamine; 3' TAMRA, Rhodamine), and these are examples of fluorescent dsRNA that can be used in the invention.

III.A.4. Aptamers

Traditionally, techniques for detecting and purifying target molecules have used polypeptides, such as antibodies, that specifically bind such targets. Nucleic acids have long been known to specifically bind other nucleic acids (*e.g.*, ones having complementary sequences). However, nucleic acids that bind non-nucleic target molecules have been described and are generally referred to as aptamers (see, *e.g.*, Blackwell et al., Science 250:1104-1110, 1990; Blackwell et al., Science 250:1149-1152, 1990; Tuerk et al., Science 249:505-510, 1990; and Joyce, Gene 82:83-87, 1989. Accordingly, nucleic acid molecules suitable for use in the present invention also include aptamers.

As applied to aptamers, the term "binding" specifically excludes the "Watson-Crick"-type binding interactions (*i.e.*, A:T and G:C base-pairing) traditionally associated with the DNA double helix. The term "aptamer" thus refers to a nucleic acid or a nucleic acid derivative that specifically binds to a target molecule, wherein the target molecule is either (i) not a nucleic acid, or (ii) a nucleic acid or structural element thereof that is bound by the aptatmer through mechanisms other than duplex- or triplex-type base pairing.

In general, techniques for identifying aptamers involve incubating a preselected non-nucleic acid target molecule with mixtures (2 to 50 members), pools (50 to 5,000 members) or libraries (50 or more members) of different nucleic acids that are potential aptamers under conditions that allow complexes of target molecules and aptamers to form. By "different nucleic acids" it is meant that the nucleotide sequence of each potential aptamer may be different from that of any other member, that is, the sequences of the potential aptamers are random with respect to each other. Randomness can be introduced in a variety of manners such as, *e.g.*, mutagenesis, which can be carried out in vivo by exposing cells harboring a nucleic acid with mutagenic agents, in vitro by chemical treatment of a nucleic acid, or in vitro by biochemical replication (*e.g.*, PCR) that is deliberately allowed to proceed under conditions that reduce fidelity of replication process; randomized chemical synthesis, *i.e.*, by synthesizing a plurality of nucleic acids having a preselected sequence that, with regards to at least one position in the sequence, is random. By "random at a position in a preselected sequence" it is meant that a position in a sequence that is normally synthesized as, *e.g.*, as close to 100% A as possible (*e.g.*, 5'-C-T-T-A-G-T-3'), is allowed to be randomly synthesized at that position (C-T-T-N-G-T, wherein N indicates a randomized position. At a randomized position, for example, the synthesizing reaction contains 25% each of A,T,C and G; or x% A, w% T, y% C and z%G, wherein x + w + y + z =100. The randomization at the position may be complete (*i.e.*, x = y = w = z = 25%) or stoichastic (*i.e.*, at least one of x, w, y and z is not 25%).

In later stages of the process, the sequences are increasingly less randomized and consensus sequences may appear; in any event, it is preferred to ultimately obtain an aptamer having a unique nucleotide sequence.

Aptamers and pools of aptamers are prepared, identified, characterized and/or purified by any appropriate technique, including those utilizing in vitro synthesis, recombinant DNA techniques, PCR amplification, and the like. After their formation, target:aptamer complexes are then separated from the uncomplexed members of the nucleic acid mixture, and the nucleic acids that can be prepared from the complexes are candidate aptamers (at early stages of the technique, the aptamers generally being a population of a multiplicity of nucleotide sequences having varying degrees of specificity for the target). The resulting aptamer (mixture or pool) is then substituted for the starting apatamer (library or pool) in repeated iterations of this series of steps. When a limited number (*e.g.*, a pool or mixture, preferably a mixture with less than 10 members, most preferably 1) of nucleic acids having satisfactory specificity is obtained, the aptamer is sequenced and characterized. Pure preparations of a given aptamer are generated by any appropriate technique (*e.g.*, PCR amplification, in vitro chemical synthesis, and the like).

For example, Tuerk and Gold (Science 249:505-510, 1990) describe the use of a procedure termed "systematic evolution of ligands by exponential enrichment" (SELEX). In this method, pools of nucleic acid molecules that are randomized at specific positions are subjected to selection for binding to a nucleic acid-binding protein (see, *e.g.*, PCT International Publication No. WO 91/19813 and U.S. Pat. No. 5,270,163). The oligonucleotides so obtained are sequenced and otherwise characterization. Kinzler et al. (Nucleic Acids Res. 17:3645-3653, 1989) used a similar technique to identify synthetic double-stranded DNA molecules that are specifically bound by DNA-binding polypeptides. Ellington et al. (Nature 346:818-822, 1990) describe the production of a large number of random sequence RNA molecules and the selection and identification of those that bind specifically to specific dyes such as Cibacron blue.

Another technique for identifying nucleic acids that bind non-nucleic target molecules is the oligonucleotide combinatorial technique described by Ecker et al. (Nuc. Acids Res. 21:1853, 1993) known as "synthetic unrandomization of randomized fragments" (SURF), which is based on repetitive synthesis and screening of increasingly simplified sets of oligonucleotide analogue libraries, pools and mixtures (Tuerk et al., Science 249:505, 1990). The starting library consists of oligonucleotide analogues of defined length with one position in each pool containing a known analogue and the remaining positions containing equimolar mixtures of all other analogues. With each round of synthesis and selection, the identity of at least one position of the oligomer is determined until the sequences of optimized nucleic acid ligand aptamers are discovered.

Once a particular candidate aptamer has been identified through a SURF, SELEX or any other technique, its nucleotide sequence can be determined (as is known in the art), and its three-dimensional molecular structure can be examined by nuclear magnetic resonance (NMR). These techniques are explained in relation to the determination of the three-dimensional structure of a nucleic acid ligand that binds thrombin in Padmanabhan et al., J. Biol. Chem. 24:17651 (1993); Wang et al., Biochemistry 32:1899 (1993); and Macaya et al., Proc. Nat'l. Acad. Sci. USA 90:3745 (1993). Selected aptamers may be resynthesized using one or more modified bases, sugars or backbone linkages. Aptamers consist essentially of the minimum sequence of nucleic acid needed to confer binding specificity, but may be extended on the 5' end, the 3' end, or both, or may be otherwise derivatized or conjugated.

III.A.5. Oligonucleotide Synthesis

The oligonucleotides used in accordance with the present invention can be conveniently and routinely made through the well-known technique of solid-phase synthesis. Equipment for such synthesis is sold by several vendors including, for example, Applied Biosystems (Foster City, CA). Other methods for such synthesis that are known in the art may additionally or alternatively be employed. It is well known to use similar techniques to prepare oligonucleotides such as the phosphorothioates and alkylated derivatives. By way of non-limiting example, see, *e.g.*, U.S. Patent No. 4,517,338 (Multiple reactor system and method for polynucleotide synthesis) to Urdea et al., and 4,458,066 (Process for preparing polynucleotides) to Caruthers *et al.;* Lyer et al., Modified oligonucleotides--synthesis, properties and applications. Curr Opin Mol Ther. 1:344-358, 1999; Verma et al., Modified oligonucleotides: synthesis and strategy for users. Annu Rev Biochem. 67:99-134, 1998; Pfleiderer et al., Recent progress in oligonucleotide synthesis. Acta Biochim Pol. 43:37-44, 1996; Warren et al., Principles and methods for the analysis and purification of synthetic deoxyribonucleotides by high-performance liquid chromatography. Mol Biotechnol. 4:179-199, 1995; Sproat, Chemistry and applications of oligonucleotide analogues. JBiotechnol. 41:221-238, 1995; De Mesmaeker et al., Backbone modifications in oligonucleotides and peptide nucleic acid systems. Curr Opin Struct Biol. 5:343-355, 1995; Charubala et al., Chemical synthesis of 2',5'-oligoadenylate analogues. Prog Mol Subcell Biol. 14:114-138, 1994; Sonveaux, Protecting groups in oligonucleotide synthesis. Methods Mol Biol. 26:1-71, 1994; Goodchild, Conjugates of oligonucleotides and modified oligonucleotides: a review of their synthesis and properties. Bioconjug Chem. 1:165-187, 1990; Thuong et al., Chemical synthesis of natural and modified oligodeoxynucleotides. Biochimie 67:673-684, 1985; Itakura et al., Synthesis and use of synthetic oligonucleotides. Annu Rev Biochem. 53:323-356, 1984; Caruthers et al., Deoxyoligonucleotide synthesis via the phosphoramidite method. Gene Amplif Anal. 3:1-26, 1983; Ohtsuka et al., Recent developments in the chemical synthesis of polynucleotides. Nucleic Acids Res. 10:6553-6560, 1982; and Kossel, Recent advances in polynucleotide synthesis. Fortschr Chem Org Naturst. 32:297-508, 1975.

III.A.6. Micro-RNAs

MicroRNAs (miRNAs) are short non-coding RNAs that play a role in the control of gene expression. It has been estimated that as much as 1% of the human genome may encode miRNA (Lim et al., Science 299:1540, 2003). RNAi molecules, such as those described herein, are one type of miRNA; others are known in the art (see, for example, Meli et al., Int Microbiol. 4:5-11, 2001; Wassarman et al., Trends Microbiol. 7:37-45, 1999; and The Small RNA Database at http://mbcr.bcm.1mc.edu/smallRNA/smallma.html), and include without limitation tRNAs, snoRNAs and tmRNAs.

IILA.6.a. Small Nucleolar RNAs (snoRNAs)

Small nucleolar RNAs (snoRNAs) are stable RNA species localized in the eukaryotic nucleoli of a broad variety of eukaryotes including fungi, protists, plants and animals. (For reviews, see Peculis et al., Curr. Opin. Cell Biol. 6:1413-1415, 1996; Gerbi, Biochem. Cell. Biol. 73:845-858, 1995; and Maxwell et al., Annu. Rev. Biochem., 35:897-934, 1995; see also The snoRNA Database at http://rna.wustl.edu/snoRNAdb/). SnoRNAs have been demonstrated to define sites of nucleotide modifications in rRNA, specifically 2'-O-ribose methylation and formation of pseudouridines, and a few snoRNAs are required for cleavage of precursor rRNA.

Generally, snoRNAs fall into two groups: the box C/D family and the box H/ACA family (Balakin et al., Cell 86:823-834, 1996; Ganot et al., Genes Dev. 11:941-956, 1997). (One exception is the MRP RNA, which is involved in a specific pre-rRNA cleavage reaction, perhaps as a ribozyme; see Maxwell et al., Annu. Rev. Biochem. 35:897-934, 1995; Tollervey et al., Curr. Opin. Cell Biol. 9:337-342, 1997.) A small number of box C/D snoRNAs are involved in rRNA processing; most, however, are known or predicted to serve as guide RNAs in ribose methylation of rRNA.

The DNA coding units for the snoRNAs occur in both traditional and novel genetic arrangements. Some are transcribed from independent promoters which serve mono- or polycistronic snoRNA coding units. Others are encoded within introns of protein (or protein-like) genes. Regardless of the diverse genomic organization, snoRNA synthesis appears to involve a number of pathways with some common steps: (1) folding of the precursor to form a box C/D or box H/ACA protein binding motif; (2) binding of one or more proteins to this motif; (3) processing of the precursor to the mature RNA; (4) partial or complete assembly of the snoRNP particle; and (5) transport to the nucleolus (Samarsky et al., EMBO J. 17:3747-3757, 1998, and references cited therein).

III.A.6.b. tmRNA

As the name implies, tmRNA (earlier called "10S RNA") has properties of tRNA and mRNA combined in a single molecule. Acting both as a tRNA and an mRNA, in a process known as trans-translation, tmRNA adds a short peptide tag to undesirable proteins. Trans-translation plays at least two physiological roles: removing ribosomes stalled upon mRNA, and targeting the resulting truncated proteins for degradation by proteases. For a review of tmRNA, see Muto et al., Trends Biochem Sci. 23:25-29 (1998). Sequences of tmRNA molecules may be found in the tmRNA website, http://www.indiana.edu/~tmrna/; see also Williams, Nucleic Acids Res 27:165-166, 1999; and Williams et al., Nucleic Acids Res 26:163-165, 1998.

III.A.7. Chemical Modifications of Nucleic Acids

In certain embodiments, particularly those involving synthetic nucleic acids, oligonucleotides used in accordance with the present invention may comprise one or more chemical modifications. By way of non-limiting example, Braasch et al. (Biochemistry 42:7967-75, 2003) report that RNAi molecules at least tolerate, and may be enhanced by, phosphorothioate linkages and/or the incorporation of 2'-deoxy-2'-fluorouridine. Chemical modifications include with neither limitation nor exclusivity base modifications, sugar modifications, and backbone modifications. In addition, a variety of molecules, including by way of non-limiting example fluorophores and other detectable moieties, can be conjugated to the oligonucleotides or incorporated therein during synthesis. Other suitable modifications include but are not limited to base modifications, sugar modifications, backbone modifications, and the like.

III.A.7.a. Base Modifications

In certain embodiments, the oligonucleotides used in the present invention can comprise one or more base modifications. For example, the base residues in aptamers may be other than naturally occurring bases (*e.g.*, A, G, C, T, U, and the like). Derivatives of purines and pyrimidines are known in the art; an exemplary but not exhaustive list includes aziridinylcytosine, 4-acetylcytosine, 5-fluorouracil, 5-bromouracil, 5-carboxymethylaminomethyl-2-thiouracil, 5-carboxymethylaminomethyluracil, inosine (and derivatives thereof), N6-isopentenyladenine, 1-methyladenine, 1-methylpseudouracil, 1-methylguanine, 1-methylinosine, 2,2-dimethylguanine, 2-methyladenine, 2-methylguanine, 7-methylguanine, 3-methylcytosine, 5-methylcytosine (5MC), N6-methyladenine, 5-methylaminomethyluracil, 5-methoxyaminomethyl-2-thiouracil, beta-D-mannosylqueosine, 5-methoxyuracil, 2-methylthio-N-6-isopentenyladenine, uracil-5-oxyacetic acid methylester, pseudouracil, queosine, 2-thiocytosine, 5-methyl-2-thiouracil, 2-thiouracil, 4-thiouracil, 5-methyluracil, uracil-5-oxyacetic acid, and 2,6-diaminopurine. In addition to nucleic acids that incorporate one or more of such base derivatives, nucleic acids having nucleotide residues that are devoid of a purine or a pyrimidine base may also be included in oligonucleotides and other nucleic acids.

III.A.7.b. Sugar Modifications

The oligonucleotides used in the present invention can also (or alternatively) comprise one or more sugar modifications. For example, the sugar residues in oligonucleotides and other nucleic acids may be other than conventional ribose and deoxyribose residues. By way of non-limiting example, substitution at the 2'-position of the furanose residue enhances nuclease stability. An exemplary, but not exhaustive list, of modified sugar residues includes 2' substituted sugars such as 2'-O-methyl-, 2'-O-alkyl, 2'-O-allyl, 2'-S-alkyl, 2'-S-allyl, 2'-fluoro-, 2'-halo, or 2'-azido-ribose, carbocyclic sugar analogs, alpha-anomeric sugars, epimeric sugars such as arabinose, xyloses or lyxoses, pyranose sugars, furanose sugars, sedoheptuloses, acyclic analogs and abasic nucleoside analogs such as methyl riboside, ethyl riboside or propylriboside.

III.A.7.c. Backbone Modifications

The oligonucleotides used in the present invention can also (or alternatively) comprise one or more backbone modifications. For example, chemically modified backbones of oligonucleotides and other nucleic acids include, by way of non-limiting example, phosphorothioates, chiral phosphorothioates, phosphorodithioates, phosphotriesters, aminoalkylphos-photriesters, methyl and other alkyl phosphonates including 3'-alkylene phosphonates and chiral phosphonates, phosphinates, phosphoramidates including 3'-amino phosphoramidate and aminoalkylphosphoramidates, thionophosphoramidates, thionoalkylphosphonates, thionoalkylphosphotri-esters, and boranophosphates having normal 3'-5' linkages, 2'-5' linked analogs of these, and those having inverted polarity wherein the adjacent pairs of nucleoside units are linked 3'-5' to 5'-3' or 2'-5' to 5'-2'. Chemically modified backbones that do not contain a phosphorus atom have backbones that are formed by short chain alkyl or cycloalkyl internucleoside linkages, mixed heteroatom and alkyl or cycloalkyl internucleoside linkages, or one or more short chain heteroatomic or heterocyclic internucleoside linkages, including without limitation morpholino linkages; siloxane backbones; sulfide, sulfoxide and sulfone backbones; formacetyl and thioformacetyl backbones; methylene formacetyl and thioformacetyl backbones; alkene containing backbones; sulfamate backbones; methyleneimino and methylenehydrazino backbones; sulfonate and sulfonamide backbones; and amide backbones.

IV. DICER Reactions

As reported by Zhang et al. (EMBO J 21:5875-5885, 2002) and Provost et al. (EMBO J 21:5864-5874, 2002), activity of recombinantly-produced human DICER is stimulated by limited proteolysis, and the proteolysed enzyme becomes active at 4°C. Cleavage of dsRNA by purifed DICER is ATP independent. Complexes of DICER and dsRNA formed at high KCl concentrations are catalytically inactive, which suggests that ionic interactions are involved in dsRNA cleavage. Zhang *et al.* (2002) report that the maximal activity was found at pH 6.5-6.9, 1-5 mM $Mg^{++}$, and 50-100 mM NaCl, although it should be noted increasing the NaCl to 0.2 M inhibited the reactions. Other reaction conditions are described in Tuschl et al. (Genes Dev. 13:3191-3197, 1999) and Zamore et al. (Cell 101:25-33, 2000).

Binding of a dsRNA substrate to the enzyme can be uncoupled from the cleavage step by omitting $Mg^{++}$ or by performing the reaction at 4°C. Thus, it is possible to set up DICER reaction mixes in which the dsRNA substrate is bound to the enzyme (*e.g.*, due the absence of $Mg^{++}$), but the reaction does not initiate until the DICER is made active (*e.g.*, by the addition of $Mg^{++}$). Similarly, DICER reactions can be terminated by the addition of a chelating agent (*e.g.*, EDTA) in an amount sufficient to lower the concentration of $Mg^{++}$ to a level insufficient to support the enzymatic reaction, or by addition of an amount of KCl sufficient to inhibit the reaction.

Conditions for reactions using RNase III have been described. For example, Li et al. (Nucleic Acids Res 21:1919-1925, 1993) describe reaction conditions for RNase III purified to homogeneity from an overexpressing bacterial strain. For example, one set of reaction conditions is 37°C, in buffer containing 250 mM potassium glutamate and 10 mM $MgCl_2$). The magnesium ion ($Mg^{++}$) can be replaced by $Mn^{++}$ or $Co^{++}$, whereas neither $Ca^{++}$ nor $Zn^{++}$ support RNase III activity. Li *et al.* (1993) further report that RNase III does not require a monovalent salt for its activity; however, the *in vitro* reactivity pattern is influenced by the monovalent salt concentration. Franch et al. (J Biol Chem 274: 26572-26578, 1999) describe assays of RNase III activity in 1x TMK-glutamate buffer (20 mM Tris acetate, 10 mM magnesium acetate and 200 mM potassium glutamate), which may be supplemented with 1 mM dithiothreitol and 1 ug tRNA, in a reaction volume of 20 ul.

One skilled in the art will know how to prepare, characterize and assay other RNases form other biological systems. For example, Bellofatto et al. (J Biol Chem 258:5467-5476, 1983) describe the purification and characterization of an RNA processing enzyme from *Caulobacter crescentus* that has an absolute requirement for monovalent cations. Methods for assaying ribonuclease activity are known. For example, March et al. (Nucleic Acids Res 18:3293-3298, 1990) describe experiments in which enzyme activity was monitored by assaying fractions for the ability to correctly process exogenous RNA containing specific RNase III cleavage sites

Following the completion of a dicing reaction, the reaction mixture is diluted with a suitable amount (which may be none) of water or an aqueous solution, such as a buffer, which may be a weak buffer. A suitable amount encompasses an amount of solution determined to be appropriate for the purposes of carrying on the filtration and isolation methods. Such amounts can be readily determined by one skilled in the art, and are encompassed by the present invention. In one embodiment, water or an aqueous solution, such as a buffer, which may be a weak buffer, is added to the reaction mixture to produce a buffered RNA fragment solution. Suitable solutions and buffers include those buffers described throughout this application. In one such embodiment, this solution comprises about 1 to about 10 M guanidine isothiocyanate, about 10 to about 100 mM Tris-HCl (pH 7.0 to 8.0, preferably 7.5), about 1 to about 50mM EDTA (pH 7.5 to 8.5, preferably 8.0), and about 1 to about 10% β-mercaptoethanol. In certain such embodiments, this solution comprises about 4 M guanidine isothiocyanate, about 50 mM Tris-HCl (pH 7.0 to 8.0, preferably 7.5), about 25 mM EDTA (pH 8.0), and about 1% β-mercaptoethanol. This solution typically also comprises ethanol. In a certain such embodiment, this solution comprises about 33% ethanol by volume. In other suitable embodiments, isoproanol may be substituted for ethanol.

The RNA fragment solution is then loaded on one or more first affinity columns. In a suitable embodiment, these one or more first affinity columns are glass fiber RNA purification columns (Micro-to-Midi Total RNA Purification kit, Invitrogen). The elutate comprising Short (*i.e.*, diced) RNA molecules is then allowed to pass through the column and is collected. This eluate may pass through the column via gravity, the column may be centrifuged to facilitate elution or by vacuum or pressure, and combinations of such methods. In one embodiment, the first affinity columns are centrifuged for 2 minutes at about 10,000 revolutions per minute in a microcentrifuge.

The eluate comprising the Short RNA molecules is then diluted with a suitable volume of ethanol. In a certain such embodiment, the volume of ethanol added to the eluate comprising Short RNA molecules is such that the final concentration of ethanol is about 70% by volume. In other suitable embodiments, isoproanol may be substituted for ethanol.

This diluted eluate comprising the Short RNA molecules is then loaded onto one or more second affinity columns. In a suitable embodiment, these one or more second affinity columns are glass fiber RNA purification columns (Micro-to-Midi Total RNA Purification kit, Invitrogen). The elutate comprising Short RNA molecules is then allowed to pass through the second affinity columns. This eluate may pass the second affinity columns via gravity, positive or negative (vacuum) pressure, or the columns may be centrifuged to facilitate elution, or combinations of such methods can be used. In a suitable embodiment, the second affinity columns are centrifuged for about 15 seconds at about 10,000 revolutions per minute in a microcentrifuge.

The one or more second affinity columns are then washed with a suitable buffer comprising between from about 50 to about 100% ethanol by volume. For example, the final ethanol concentration may be about 50 to about 95%, about 60 to about 90%, or about 70 to about 90% ethanol by volume. Suitable buffers include those buffers described throughout this application. In other suitable embodiments, isoproanol may be substituted for ethanol. In suitable embodiments, this wash buffer comprises between 10 to about 50 mM Tris-HCl (pH 7.5), about 0.01 to about 1 mM EDTA (pH 8.0) and about 60 to about 90% ethanol. In one such embodiment, this was buffer comprises 5mM Tris-HCl (pH 7.5), 0.1 mM EDTA (pH 8.0) and 80% ethanol. In other suitable embodiments, isoproanol may be substituted for ethanol. The eluate is then allowed to pass through the one or more second affinity columns. This eluate may pass through the one or more second affinity columns via gravity, positive or negative (vacuum) pressure, or the columns may be centrifuged to facilitate elution, or combinations of such methods can be used. In a suitable embodiment, the one or more second affinity columns are centrifuged for about 2 minutes at about 10,000 revolutions per minute in a microcentrifuge. This washing step may be repeated multiple times (i.e. two, five, 10, 20, etc. times). The one or more second affinity columns are then dried. The columns may be dried via heat, gravity ("drip-dry"), positive or negative (vacuum) pressure, or the columns may be centrifuged, or combinations of such methods can be used. In a suitable embodiment, the second affinity columns are centrifuged for about 2 minutes at about 10,000 revolutions per minute in a microcentrifuge.

One or more Short RNA molecules is then collected from the second one or more affinity columns. A suitable volume of water or an aqueous solution, such as a buffer, which may be a weak buffer, is added to the second one or more affinity columns and the eluate comprising the one or more Short RNA molecules is collected. This eluate may pass the second affinity columns via gravity, positive or negative (vacuum) pressure, or the columns may be centrifuged to facilitate elution, or combinations of such methods can be used. In a suitable embodiment, the one or more second affinity columns are centrifuged for about 2 minutes at about 10,000 revolutions per minute in a microcentrifuge.

V. Gene Regulation, Genomics, Proteomics and High Throughput Screening

In certain embodiments, RNAi molecules and other nucleic acids (including without limitation antisense nucleic acids) prepared according the present invention are used in various methods of and compositions for gene regulation. Decreased expression of a gene results in lesser amounts of the final gene product that is, in some embodiments, a protein; however, as in known in the art, some gene products are RNA molecules (*e.g.*, rRNA, tRNA, and the like).

In any event, RNAi molecules and other nucleic acids are prepared via the invention can be used to decrease expression of one or more target genes. The expression of known genes and proteins is down-regulated in order to observe the effects on a biological system (cells, organisms, etc.) on the loss of the function of a previously identified gene product. For example, if the target gene is a repressor of the expression of other genes, up-regulation of these genes would result from down-regulation of the target gene. In other embodiments, the nucleic acid sequence of a gene is known, but the function of the gene product is unknown; in this case, down-regulation is used to determine the function per se of the target gene. The latter embodiment describes, in general terms, the field of proteomics. In both of these embodiments, target nucleic acid sequences are identified and used to design RNAi molecules or other nucleic acids of the invention, and these nucleic acids are prepared using the methods of the invention.

Partial or complete down-regulation of target genes are possible using RNAi molecules or other nucleic acids prepared via the methods, compositions and kits of the invention. Any measurable degree of down-regulation can be achieved using the invention. The expression of the target gene can be reduced from about 1% to about 99%, with 100% being complete suppression/inhibition of the target gene, although a reduction of anything less than about 5% of expression may not produce a measurable response. In general, the range of down-regulation is any value in the range of from about 5% to 100%. That is, the degree of down-regulation is about n%, wherein n is any whole integer between 5 and 100. For example, about 10%, about 20%, about 30%, about 40%, about 50%, about 60%, about 70%, about 80%, about 90%, about 95%, or about 99% of gene expression of the target gene may be suppressed.

Depending on the assay, quantitation of the amount of gene expression allows one to determine the degree of inhibition (or enhancement) of gene expression in a cell or animal treated with one or more RNAi molecules, compared to a cell or animal not so treated according to the present invention. Lower doses of injected material and longer times after administration of dsRNA may result in inhibition or enhancement in a smaller fraction of cells or animals (*e.g.*, at least 10%, 20%, 50%, 75%, 90%, or 95% of targeted cells or animals). Quantitation of gene expression in a cell or animal may show similar amounts of inhibition or enhancement at the level of accumulation of target mRNA or translation of target protein. The efficiency of inhibition or enhancement may be determined by assessing the amount of gene product in the cell or animal using any method known in the art. For example, mRNA may be detected with a hybridization probe having a nucleotide sequence outside the region used for the interfering RNA, or translated polypeptide may be detected with an antibody raised against the polypeptide sequence of that region. Methods by which to quantitate mRNA and polypeptide sequences are well-known in the art can be found in, for example, Sambrook, J. et al., Molecular Cloning: A Laboratory Manual, 2nd edition, Cold Spring Harbor Laboratory Press, Plainview, New York (1989), and other similar manuals.

The methods of the present invention can be used to regulate expression of genes that are endogenous to a cell or animal using RNAi molecules prepared via the methods, compositions and kits of the invention. An endogenous gene is any gene that is heritable as an integral element of the genome of the animal species. Regulation of endogenous genes by methods of the invention can provide a method by which to suppress or enhance a phenotype or biological state of a cell or an animal. Examples of phenotypes or biological states that can be regulated include, but are not limited to, shedding or transmission of a virus, feed efficiency, growth rate, palatability, prolificacy, secondary sex characteristics, carcass yield, carcass fat content, wool quality, wool yield, disease resistance, post-partum survival and fertility. Additional endogenous genes that can also be regulated by the methods of the invention include, but are not limited to, endogenous genes that are required for cell survival, endogenous genes that are required for cell replication, endogenous genes that are required for viral replication, endogenous genes that encode an immunoglobulin locus, and endogenous genes that encode a cell surface protein. Other examples of endogenous genes include developmental genes (*e.g.*, adhesion molecules, cyclin kinase inhibitors, Writ family members, Pax family members, Winged helix family members, Hox family members, cytokines/lymphokines and their receptors, growth/differentiation factors and their receptors, neurotransmitters and their receptors), tumor suppressor genes (*e.g.*, APC, BRCA1, BRCA2, MADH4, MCC, NF 1, NF2, RB 1, TP53, and WTI) and enzymes (*e.g.*, ACC synthases and oxidases, ACP desaturases and hydroxylases, ADP-glucose pyrophorylases, ATPases, alcohol dehydrogenases, amylases, amyloglucosidases, catalases, cellulases, chalcone synthases, chitinases, cyclooxygenases, decarboxylases, dextrinases, DNA and RNA polymerases, galactosidases, glucanases, glucose oxidases, granule-bound starch synthases, GTPases, helicases, hemicellulases, integrases, inulinases, invertases, isomerases, kinases, lactases, lipases, lipoxygenases, lysozymes, nopaline synthases, octopine synthases, pectinesterases, peroxidases, phosphatases, phospholipases, phosphorylases, phytases, plant growth regulator synthases, polygalacturonases, proteinases and peptidases, pullanases, recombinases, reverse transcriptases, RUBISCOs, topoisomerases, and xylanases).

Methods by which to transfect cells with RNAi molecules and other nucleic acids are well known in the art and include, but are not limited to, electroporation, particle bombardment, microinjection, and through the use of transfection agents. Such transfection agents include without limitation those listed in Table 2 (entitled "Non-limiting Examples of Transfection Agents"), and can be used alone or in combination with each other.

Table 2: Non-limiting Examples of Transfection Agents

| TRANSFECTION AGENT | DESCRIPTION | PATENTS AND/OR CITATIONS | AVAILABLE FROM |
|---|---|---|---|
| BMOP | N-(2-bromoethyl)-N,N-dimethyl-2,3-bis(9-octadecenyloxy)-propana minimun bromide) | | |
| BMOP:DOPE | 1:1 (wt/wt) formulation of N-(2-bromoethyl)-N,N-dimethyl-2,3-bis(9-octadecenyloxy)-propana minimun bromide) (BMOP) and DOPE | Walzem et al., Poult Sci. 76:882-6, 1997. Transfection of avian LMH-2A hepatoma cells with cationic lipids. | |
| Cationic polysaccharides | Cationic polysaccharides | Published U.S. patent application 2002/0146826 | |
| CellFECTIN® | 1:1.5 (M/M) formulation of N, NI, NII, NIII-tetramethyl-N, NI, NII, NIII-tetrapalmitylspermine (TM-TPS) and dioleoyl phosphatidylethanolamine (DOPE) | U.S. Patents 5,674,908, 5,834,439 and 6,110,916 | Invitrogen (LTI) |
| CTAB:DOPE | formulation of cetyltrimethyl-ammonium bromide (CATB) and dioleoylphosphatidylethanol -amine (DOPE) | | |
| Cytofectin GSV | 2:1 (M/M) formulation of cytofectin GS* and dioleoyl phosphatidyl-ethanolamine (DOPE) | | (*Cytofectin GS corresponds to Gilead Sciences' GS 3815) |
| DC-Cholesterol (DC-Chol) | 3,β-N,(N',N'-dimethylaminoethane)-carbamo-yl]cholesterol | | |
| DC-Chol:DOPE | formulation of 3,β-N,(N',N'-dimethylaminoethane)-carbamo-yl] cholesterol (DC-Chol) and dioleoyl phosphatidyl-ethanolamine (DOPE) | Gao et al., Biochim. Biophys. Res. Comm. 179:280-285, 1991 | |
| DC-6-14 | O,O'-Ditetradecanoyl-N-(alpha-trimethylammonioacetyl)diet hanolamine chloride | Kikuchi et al., Hum Gene Ther 10:947-55, 1999. Development of novel cationic liposomes for efficient gene transfer into peritoneal disseminated tumor. | |
| DCPE | Dicaproylphosphtidylethanol -amine | | |

(continued)

| TRANSFECTION AGENT | DESCRIPTION | PATENTS AND/OR CITATIONS | AVAILABLE FROM |
|---|---|---|---|
| DDPES | Dipahnitoylphosphatidylethanolamine 5-carboxyspermylamide | Behr et al., Proc. Natl. Acad. Sci. USA 86:6982-6986, 1989. Efficient gene transfer into mammalian primary endocrine cells with lipopolyamine-coated DNA; EPO published patent application 0 394 111 | |
| DDAB | didoceyl methylammonium bromide | | |
| Dextran and dextran derivatives or conjugates | DEAE-Dextran; Dextran sulfate | Mai et al., J Biol Chem. 277:30208-30218, 2002. Efficiency of protein transduction is cell type-dependent and is enhanced by dextran sulfate. | |
| Diquaternary ammonium salts | (examples:) N,N'-dioleyl-N,N,N',N'-tetramethyl-1,2-ethanediamine (TmedEce), N,N'-dioleyl-N,N,N',N'-tetramethyl-1,3-propanediamine (PropEce), N,N'-dioleyl-N,N,N',N'-tetramethyl-1,6-hexanediamine (HexEce), and their corresponding N,N'-dicetyl saturated analogues (TmedAce, PropAce and HexAce) | Rosenzweig et al., Bioconjug Chem 12:258-63, 2001. Diquatemary ammonium compounds as transfection agents; U.S. Patent 5,994,317 | Vical |
| DLRIE | dilauryl oxypropyl-3-dimethylhydroxy ethylammonium bromide | Felgner et al., Ann N Y Acad Sci 772:126-39, 1995. Improved cationic lipid formulations for in vivo gene therapy. | Vical |
| DMAP | 4-dimethylaminopyridine | | |
| DMPE | Dimyristoylphospatidylethan ol-amine | | |
| DMRIE | N-[1-(2,3-dimyristyloxy)propyl]-N,N-dimethyl-N-(2-hydroxyethyl) ammonium bromide | Konopka et al., Biochim Biophys Acta 1312:186-96, 1996. Human immuno-deficiency virus type-1 (HIV-1) infection increases the sensitivity of macrophages and THP-1 cells to cytotoxicity by cationic liposomes. | |
| DMRIE-C | 1:1 formulation of N-[1-(2,3-dimyristyloxy)propyl]-N,N-dimethyl-N-(2-hydroxyethyl) ammonium bromide (DMRIE) and cholesterol | U.S. Patents 5,459,127 and 5,264,618, to Felgner, et al. (Vical) | Invitrogen (LTI) |

(continued)

| TRANSFECTION AGENT | DESCRIPTION | PATENTS AND/OR CITATIONS | AVAILABLE FROM |
|---|---|---|---|
| DMRIE:DOPE | formulation of 1,2-dimyristyloxypropyl-3-dimethyl-hydroxyethyl ammonium bromide and dioleoyl phosphatidyl-ethanolamine (DOPE) | San et al., Hum Gene Ther 4:781-8, 1993. Safety and short-term toxicity of a novel cationic lipid formulation for human gene therapy. | |
| DOEPC | dioleoylethylphosphocholine | | |
| DOHME | N-[1-(2,3-dioleoyloxy)propyl]-N-[1-(2-hydroxyethyl)]-N,N-dimethylammonium iodide | | |
| DOPC | dioleoylphosphatidylcholine | | |
| DOPC:DOPS | 1:1 (wt%) formulation of DOPC (dioleoylphosphatidylcholin e) and DOPS | | Avanti |
| DOSPA | 2,3-dioleoyloxy-N-[2-(sperminecarboxamidoethyl] -N,N-di-met- hyl-1-propanaminium trifluoroacetate | | |
| DOSPA:DOPE | Formulation of 2,3-dioleoyloxy-N-[2-(sperminecarboxamidoethyl] N,N-di-met-hyl-1-propanaminium trifluoroacetate (DOSPA) and dioleoyl phosphatidyl-ethanolamine (DOPE) | Baccaglini et al., J Gene Med 3:82-90, 2001. Cationic liposome-mediated gene transfer to rat salivary epithelial cells in vitro and in vivo. | |
| DOSPER | 1,3-Di-Oleoyloxy-2-(6-Carboxy-spermyl)-propylamid | Buchberger et al., Biochemica 2:7-10, 1996. DOSPER liposomal transfection reagent: a reagent with unique transfection properties. | Roche |
| DOTAP | N-[1-(2,3-dioleoyloxy)propyl]-N,N,N-trimethyl-ammonium methylsulfate | | |
| DOTMA | N-[1-(2,3-dioleyloxy)propyl]-n,n,n-trimethylammoniumchloride | | |
| DPEPC | Dipalmitoylethylphosphatid yl-choline | | |
| Effectene | (non-liposomal lipid formulation used in conjunction with a special DNA-condensing enhancer and optimized buffer) | Zellmer et al., Histochem Cell Biol 115:41-7, 2001. Long-term expression of foreign genes in normal human epidermal keratinocytes after transfection with lipid/DNA complexes. | Qiagen |
| FuGENE 6 | | Wiesenhofer et al., J Neurosci Methods 92: 145-52, 1999. Improved lipid-mediated gene transfer in C6 glioma cells and primary glial cells using FuGene. | Roche |

(continued)

| TRANSFECTION AGENT | DESCRIPTION | PATENTS AND/OR CITATIONS | AVAILABLE FROM |
|---|---|---|---|
| GAP-DLRIE:DOPE | N-(3-aminopropyl)-N, N-dimethyl-2,3-bis(dodecyloxy)-1-propaniminium bromide/dioleyl phosphatidylethanolamine | Stephan et al., Hum Gene Ther 7:1803-12, 1996. A new cationic liposome DNA complex enhances the efficiency of arterial gene transfer in vivo. | |
| GS 2888 cytofectin | | Lewis et al., Proc Natl Acad Sci USA 93:3176-81, 1996. A serum-resistant cytofectin for cellular delivery of antisense oligodeoxynucleotides and plasmid DNA. | Gilead Sciences |
| Lipofectin® | 1:1 (w/w) formulation of N-(1-2,3-dioleyloxypropyl)-N,N,N- triethylammonium (DOTMA) and dioleylphosphatidylethanola mine (DOPE) | U.S. Patents 4,897,355; 5,208,066; and 5,550,289. | Invitrogen (LTI) |
| LipofectACE™ | 1:2.5 (w/w) formulation of dimethyl dioctadecylammonium bromide (DDAB) and dioleoyl phosphatidylethanolamine (DOPE) | | Invitrogen (LTI) |
| LipofectAMINE TM | 3:1 (w/w) formulation of 2,3-dioleyloxy-N-2(sperminecarboxamido)ethyl]-N,N-dimethyl-1-propanaminium trifluoroacetate (DOSPA) and dioleoyl phosphatidylethanolamine (DOPE) | U.S. Patent 5,334,761; and U.S. Patents 5,459,127 and 5,264,618, to Felgner, et al. (Vical) | Invitrogen (LTI) |
| LipofectAMINE ™ 2000 | | | Invitrogen (LTI) |
| LipofectAMINE PLUS™ | PLUS and LipofectAMINE™ | U.S. Patents 5,736,392 and 6,051,429 | Invitrogen/LTI |
| LipoTAXI® | | | Stratagene |
| monocationic transfection lipids | (examples:) 1-deoxy-1-[dihexadecyl (methyl)ammonio]-D-xylitol; 1-deoxy-1-[methyl(ditetradecyl)ammonio]-D-arabinitol; 1-deoxy-1-[dihexadecyl (methyl)ammonio]-D-arabinitol; 1-deoxy-1-[methyl(dioctadecyl)ammonio]-D-arabinitol | Banerjee et al., J Med Chem 44:4176-85, 2001. Design, synthesis, and transfection biology of , novel cationic glycolipids for use in liposomal gene delivery. | of |
| O-Chol | 3 beta[1-ornithinamide-carbamoyl] cholesterol | Lee et al., Gene Ther 9:859-66, 2002. Intraperitoneal gene delivery mediated by a novel cationic liposome in a peritoneal disseminated ovarian cancer model. | |
| OliogfectAMIN E™ | | | Invitrogen (LTI) |

(continued)

| TRANSFECTION AGENT | DESCRIPTION | PATENTS AND/OR CITATIONS | AVAILABLE FROM |
|---|---|---|---|
| Piperazine based amphilic cationic lipids | Piperazine based amphilic cationic lipids | U.S. Patents 5,861,397 and 6,022,874 | Vical |
| PolyFect | (activated-dendrimer molecules with a defined spherical architecture) | | Qiagen |
| Protamine | Protamine mixture prepared from, *e.g.*, salmon, salt herring, etc.; can be supplied as, *e.g.*, a sulfate or phosphate. | Sorgi et al., Gene Ther 4:961-8, 1997. Protamine sulfate enhances lipid-mediated gene transfer. | Sigma |
| SuperFect | (activated-dendrimer molecules with a defined spherical architecture) | Tang etal., Bioconjugate Chem. 7:703, 1996. In vitro gene delivery by degraded polyamido-amine dendrimers.; published PCT applications WO 93/19768 and WO 95/02397 | Qiagen |
| Tfx™ | N,N,N',N'-tetramethyl-N,N'-bis(2-hydroxyethyl)-2,3-di(oleoyloxy)-1,4-butanediammonium iodide] and DOPE | | Promega |
| TransFast™ | N,N [bis (2-hydroxyethyl)-N-methyl-N-[2,3-di(tetradecanoyloxy) propyl] ammonium iodide and DOPE | | Promega |
| TransfectAce | | | Invitrogen (LTI) |
| TRANSFECTA™ | 5-carboxylspermylglycine dioctadecylamide (DOGS) | Behr et al., Proc. Natl. Acad. Sci. USA 86:6982-6986, 1989; EPO Publication 0 394 111 | Promega |
| TransMessenger | (lipid-based formulation that is used in conjunction with a specific RNA-condensing enhancer and an optimized buffer; particularly useful for mRNA transfection) | | Qiagen |
| Vectamidine | 3-tetradecylamino-N-tert-butyl-N'-tetradecylpropionamidine (a.k.a. diC14-amidine) | Ouahabi et al., FEBS Lett 414:187-92, 1997. The role of endosome destabilizing activity in the gene transfer process mediated by cationic lipids. | |
| X-tremeGENE Q2 | | | Roche |

High throughput screening (HTS) typically uses automated assays to search through large numbers of compounds for a desired activity. Typically HTS assays are used to find new drugs by screening for chemicals that act on a particular enzyme or molecule. For example, if a chemical inactivates an enzyme it might prove to be effective in preventing a process in a cell which causes a disease. High throughput methods enable researchers to try out thousands of different chemicals against each target very quickly using robotic handling systems and automated analysis of results.

As used herein, "high throughput screening" or "HTS" refers to the rapid in vitro screening of large numbers of compounds (libraries); generally tens to hundreds of thousands of compounds, using robotic screening assays. Ultra high-throughput Screening (uHTS) generally refers to the high-throughput screening accelerated to greater than 100,000 tests per day.

To achieve high-throughput screening, it is best to house samples on a multicontainer carrier or platform. A multicontainer carrier facilitates measuring reactions of a plurality of candidate compounds simultaneously. Multi-well microplates may be used as the carrier. Such multi-well microplates, and methods for their use in numerous assays, are both known in the art and commercially available. In HTS embodiments, multi-well plates are temporarily or permanently mated to a multi-well filter block comprising the same number of affinity columns of the invention as the number of wells in the mated multi-well plate. The affinity columns in the filter block are aligned with the wells in the multi-well plate, so that a fluid that is passed through a specified affinity column in the filter block winds up in the corresponding specified well. The wells may contain a target gene expression system, a control reporter system or a "blank" control sample that lacks either reporter system. The expression systems may comprise cellular extracts that can effect *in vitro* transcription and, optionally, translation. Alternatively, the expression systems can be cellular systems, *e.g.*, a cell line expressing a target gene of interest.

Various types of agents can be screened using the HTS embodiments of the invention. Using RNAi molecules that down-regulate expression of a target gene as a non-limiting example, such molecules are prepared using a filter block of the invention and then contacted with an expression system comprising the target gene. RNAi molecules having high specific activities are identified as those that cause the greatest reduction of expression of the target gene. These or other down-regulating molecules are used to observe the effect of down-regulating the target gene in a series of wells, each of which comprises the same target gene expression system and one or more test compounds unique to the well. In this arrangement, one can screen the test compounds for ones that enhance the down-regulation of the target gene or which compensate for the effects of the target gene down-regulation.

Screening assays may include controls for purposes of calibration and confirmation of proper manipulation of the components of the assay. Blank wells that contain all of the reactants but no member, of the chemical library are usually included. As another example, a known inhibitor (or activator) of an enzyme for which modulators are sought, can be incubated with one sample of the assay, and the resulting decrease (or increase) in the enzyme activity determined according to the methods herein. It will be appreciated that modulators can also be combined with the enzyme activators or inhibitors to find modulators which inhibit the enzyme activation or repression that is otherwise caused by the presence of the known the enzyme modulator.

VII. Kits

Also described herein is a kit comprising at least one affinity column, buffer, alcoholic solution, enzyme or any other composition useful for carrying out the invention. The enzyme may be a ribonuclease, such as DICER or RNase III, or a polymerase, such as an RNA polymerase, including without limitation RNA T7 and SP6 RNA polymerases. Kits according to the invention may further comprise one or more transfection agents, such as those listed in Table 2; one or more nucleic acids, such as a pair of primers, a dsRNA substrate or a vector for transcribing double-stranded RNA; an RNA polymerase; one or more co-factors for an enzyme, such as a nucleotide triphosphate (*e.g.*, ATP, GTP, CTP, TTP or UTP); one or more stop solutions, such as a solution comprising a chelating agent (*e.g.*, EDTA or EGTA), which terminates a reaction catalyzed by an enzyme; a nuclease inhibitor, such as an RNase inhibitor; and one or more set of instructions. The set of instructions can comprise instructions for optimizing ribonuclease reactions and/or instructions for preparing RNAi molecules such as siRNA and e-siRNA molecules.

A suitable buffer for storage of a substrate dsRNA is 10 mM Tris pH 8.0, 20 mM NaCl, and 1 mM EDTA. Human recombinant DICER (hDicer) or other RNases can be stored in 50 mM Tris pH 8.0, 500 mM NaCl, 20% Glycerol, 0.1% Triton X-100, and 0.1 mM EDTA, and is stable at 4°C for at least about four months.

Liquid components of kits are stored in containers, which are typically resealable. A preferred container is an Eppendorf tube, particularly a 1.5 ml Eppendorf tube. A variety of caps may be used with the liquid container. Generally preferred are tubes with screw caps having an ethylene propylene O-ring for a positive leak-proof seal. A preferred cap uniformly compresses the O-ring on the beveled seat of the tube edge. Preferably, the containers and caps may be autoclaved and used over a wide range of temperatures (*e.g.*, +120°C to -200°C) including use with liquid nitrogen. Other containers can be used.

In one embodiment, a kit is called a "Dicer RNAi Transfection Kit" and comprises 3 separate packages or "modules". (1) The BLOCK-iT™ Dicer Enzyme Module contains 300 ul of Dicer enzyme at 1 U/ul, 10x Dicer reaction buffer (*e.g.*, 500 mM Tris-HCl, pH 8.5, 1.5 mM NaCl and 30 mM $MgCl_2$), stop solution (*e.g.*, 0.5 M EDTA, pH 8.0), RNase-free water and optionally, Dicer Dilution Buffer (*e.g.*, 50 mM Tris-HCl, pH 8, 500 mM NaCl and 20% glycerol) and is stored at -20°C. (2) The BLOCK-iT™ siRNA Purification Module contains an RNA Binding Buffer, RNase-free water, a 5x solution of an RNA Wash Buffer, 50x RNA annealing buffer (*e.g.*, 500 mM Tris-HCl, pH 8.0, 1 M DEPC-treated NaCl, and 50 mM DEPC-treated EDTA), one or more RNA spin cartridges or columns, one or more eluate and flow-through recovery tubes, and one or more siRNA collection tubes, and is stored at ambient temperature. (3) Lipofectamine™ 2000 and/or one or more other transfection agents are optionally also included in this embodiment and are stored at 4°C.

In another embodiment, a kit is called a "Dicer RNAi Transcription Kit" and comprises 3 separate packages or "modules". (1) The BLOCK-iT™ RNAi Primer Module contains one or more primers for T7 RNA polymerase (*e.g.*, T7amp1, 5'-GATGACTCGTAATACGACTCACTA-3', SEQ ID NO.:1), RNase-free water and a control template for T7 transcription (*e.g.*, plasmid pcDNA1.2™/V5-GW/lacZ DNA). (2) The BLOCK-iT™ RNAi Transcription Module contains 10x Transcription Buffer (*e.g.*, 400 mM Tris-HCl, pH 8.0, 100 mM DTT, 20 mM Spermidine, and 100 mM $MgCl_2$), 75 mM dNTPs, T7 enzyme mix (*e.g.*, 4 parts T7 RNA Polymerase at 50 U/ul, 1 part RNaseOUT at 40 U/ul, and 1 part yeast inorganic pyrophosphatase at 0.6 U/ul), DNase I at 1 U/ul, BLOCK-iT T7 Enzyme Mix, and RNase-free water, and is stored at-20°C. (3) The BLOCK-iT™ Long RNAi Purification Module contains an RNA Binding Buffer, RNase-free water, a 5x solution of an RNA Wash Buffer, 50x RNA annealing buffer, one or more RNA spin cartridges or columns, one or more eluate and flow-through recovery tubes, and one or more RNA collection tubes, and is stored at ambient temperature.

In a related embodiment, a kit is called a "Dicer RNAi TOPO® Transcription Kit". In this embodiment, the TOPO® transcription system (Invitrogen) is used for high-yield RNA synthesis. The Dicer RNAi TOPO® Transcription Kit comprises three modules, which are as described above for the Dicer RNAi Transcription Kit, with the exception that the first module further comprises a T7 TOPO® Linker, 10x PCR buffer, PCR forward and reverse primers (*e.g.*, lacZ-fwd2, 5'-ACCAGAAGCGGTGCCGGAAA-3', SEQ ID NO.:2, and lacZ-rev2, 5'-CCACAGCGGATGGTTCGGAT-3', SEQ ID NO:3)., 40 mM dNTPs and, optionally, a thermostable polymerase suitable for PCR, *e.g.*, *Taq* polymerase. The T7 TOPO® Linker is a double-stranded oligonucleotide covalently bound to topoisomerase. A single copy of the T7 linker will join to either end of Taq-generated PCR products in a reaction that takes about 15 min, preferably from about 1 min to less than about 15 min, thus forming a template for secondary PCR and subsequent transcription. The sense and antisense RNA strands are transcribed by T7 RNA polymerase in separate reactions, purified, and annealed to each other. The resulting long dsRNA can be used as a template for an RNase, such as DICER, to generate short siRNA molecules, which can then be purified using the other components of the kit or otherwise.

When stored as indicated above, the kits and their components are stable for about from 1 month to about 18 months, from about 3 months to about 12 months, about 4 months, about 5 months, about 6 months, about 7 months, about 8 months, about 9 months, about 10 months or about 11 months.

The components of the above kit embodiments can also be packaged in a single kit.

Kit embodiments can further comprise nucleic acids (primers, vectors, etc.) and enzymes (ligase, Clonase™, topoisomerase, etc.) useful for cloning the dsRNA substrate and/or siRNA products.

It will be readily apparent to one of ordinary skill in the relevant arts that other suitable modifications and adaptations to the methods and applications described herein may be made without departing from the scope of the invention or any embodiment thereof. Having now described the present invention in detail, the same will be more clearly understood by reference to the following examples, which are included herewith for purposes of illustration only and are not intended to be limiting of the invention.

## EXAMPLES

### EXAMPLE 1

### DsRNA SUBSTRATE PREPARATION

The plasmids pcDNA1.2/V5/GW-LacZ and pcDNA5-FRT-luc were used as reporter plasmids for beta-galactosidase and luciferase, respectively, in co-transfection studies.

The pcDNA5-FRT-luc plasmid comprises a CMV promoter that drives expression of a luciferase gene that terminates with a BGH polyA sequence; it also contains a FRT recombination site. In brief, pcDNA5/FRT (Invitrogen) was digested with EcoRV and XhoI, and the 5048 bp vector fragment was gel purified. The luciferase gene came from pcDNA6T7EMC-luc (Invitrogen), digested with MscI and XhoI. This 1931 bp luciferase fragment was gel purified and ligated to the 5048 bp vector fragment to create pcDNA5/FRT/luc. The correct clone was verified by examining the products of restriction digests.

The LacZ expression control plasmid pcDNA1.2™/V5-GW/lacZ was made using Multi-site Gateway. The multi-site assembly format was B4-B1-B2-B3. Briefly, pENTR5'-CMV, pENTR-LacZ and pENTR/V5TKpolyA were mixed with the DEST R4R3 plasmid using LR Plus Clonase. The three plasmids in the Multi-site reaction were all created by standard Gateway recombination reactions: (1) the CMV promoter was amplified from pcDNA3.1 using primers flanked with attB4 and attB1 sequences and recombined with pDonr 5'(P4-P1R) to form pENTR5'-CMV; (2) the LacZ gene was amplified from pcDNA3.1-LacZ using attB1 and attB2 flanking primers and recombined with pDonr 221 to create pENTR-LacZ;

and (3) the V5-TKpolyA element was amplified from pcDNA3.2 using attB2 and attB3 primers and recombined with pDonr3'(P2-P3R). All the ENTR clones were verified by sequence as well as restriction digest. Additionally, the cloning junctions and portions of the genes in the final vector were verified by sequence analysis prior to the RNAi assays.

The respective forward and reverse primers used for the amplification of the beta-galactosidase transcription template for dsRNA synthesis are:

lacZ-fwd2 5'- ACCAGAAGCGGTGCCGGAAA-3' (SEQ ID NO:2), and
lacZ-rev2 5'-CCACAGCGGATGGTTCGGAT-3' (SEQ ID NO:3).

The respective forward and reverse primers used for the amplification of the luciferase transcription template for dsRNA synthesis are:

LucFor2 5'-TGAACATTTCGCAGCCTACC-3' (SEQ ID NO:4) and
LucRev2 5'-GGGGCCACCTGATATCCTTT-3' (SEQ ID NO:5).

Long dsRNA molecules, generated for use as substrates for DICER reactions, were generated using T7-mediated transcription of pcDNA1.2/V5/GW-LacZ and pcDNA5-FRT-luc and the above-described primers.

EXAMPLE 2

DICER REACTIONS

The conditions used were essentially those described by Myers et al. (Nat Biotechnol. 21:324-8, 2003). Briefly, His-tagged human recombinant DICER (hDicer) was prepared using an expression construct, pFastBac-HisT7 Dicer Baculovirus, essentially as described in Myers *et al.* (2003). The hDicer was incubated in a 20 ul reaction mix containing 1 ug of dsRNA substrate (prepared as in Example 1), 30 mM Hepes pH 8.0, 250 mM NaCl, and 2.5 mM $MgCl_2$. It should be noted that 50 mM Tris pH 8.5 can be used instead of 30 mM HEPES, and that a suitable 10X reaction buffer is 500 mM Tris pH 8.5, 1.5 mM NaCl, and 30 mM $MgCl_2$. The reactions were incubated at 37°C for either 6 hours or 14-16 hours, and stopped with the addition of 0.4 ul of 0.5 M EDTA pH 8.0 (final concentration, 1 mM EDTA). The dsRNA concentration was quantified by absorbance at 260 nm. Reaction products were examined by separation by gel elctrophoresis and staining (*e.g.*, PAGE in a 20% TBE gel stained with ethidium brodime).

EXAMPLE 3

2-COLUMN PREPARATION OF SHORT, DICED RNA

The 2-column modality of the invention is illustrated in Panel A of Figure 3. To produce a first binding solution, the following first fluid mixture was added to the dicing reaction: 1 volume of binding buffer (4M guanidine isothiocyanate; 50 mM Tris-HCl, pH 7.5; 25 mM EDTA, pH 8.0; and 1% β-mercaptoethanol) and 1 volume of 100% ethanol (~33% final ethanol concentration). The resulting first binding solution was mixed and loaded onto a first affinity column (a glass fiber RNA purification column from the Micro-to-Midi Total RNA Purification kit, Invitrogen, Carslbad, CA) and spunfor about 15 seconds in a microcentrifuge. The flow-through (FT), which contains short RNA, was collected for further steps. This first column, in which relatively long substrate RNA and/or partially diced RNA molecules are retained, was discarded.

To produce a second binding solution, the following second fluid mixture was added to the flow-through: 4x volumes (relative to the volume of the original dicing reaction) of 100% ethanol. The final ethanol concentration was ~70% ethanol. The solution was mixed and loaded onto a second glass fiber RNA purification column and spun 15s in a microcentrifuge, and the flow-through was discarded. As small volume columns were used in this experiment, the second binding solution was loaded and centrifuged 500 ul at a time. This second column, within which short RNA molecules are retained, was used in subsequent steps, whereas the flow-through from the second column was discarded.

The second affinity column was washed at this point with 500 μl of wash buffer (5mM Tris-HCl, pH 7.5; 0.1mM EDTA, pH 8.0; 80% ethanol). The wash buffer was loaded onto the column, which was then spun in a microfuge for 1 min, and the flow-through was discarded. The washing was repeated, and the second affinity column was spun as above for 1 min to dry the membrane.

The second affinity column was placed in a collection tube. An appropriate volume (30-50 ul) of the third fluid mixture (water, certified RNase-free) was loaded onto the column, which was then incubated at ambient temperature for 1 min. The second column was then spun for 1-2 min to produce an elute comprising diced RNA molecules.

The RNA molecules were brought to a final concentration of 10 mM Tris-HCl (pH 8.0), 20 mM NaCl, and 1 mM EDTA (pH 8.0) by adding 1.2 ul of a 50X stock buffer solution to 60 ul of pooled eluate. The concentration of the siRNAs was quantified by absorbance at 260 nm, and the adjusted eluate was then stored at -80°C.

The purification method was also performed and validated using isopropanol instead of ethanol. In the first step, the same volume of 100% isopropanol replaced the ethanol. However, after the first column, half as much isopropanol was added to the flow-through as ethanol (a volume equal to twice the original reaction size.

Representative results are shown in Panel B of Figure 3. The figure shows a 20% TBE gel comprising long dsRNA transcripts (dsRNA), which are a dsRNA substrate for DICER, Dicing reactions (not purified), partially purified Dicing reactions (long and short RNAs retained), or Micro-to-Midi purified diced siRNAs (purified d-siRNAs) targeted against luciferase (luc) and GFP. A synthetic siRNA, generated by synthesis rather than by enzymatic diegestion, is in the rightmost lane.

EXAMPLE 9

MULTIWELL FORMAT TRANSFECTION

The compounds, compositions and methods described herein can be used to transfect cells in a multiwell format, *e.g.*, a 24-, 48-, 96-, or 384-well plate. The following procedures describes the transfection of siRNA into cells using Lipofectamine™ 2000 or Oligofectamine™, and can be adapted to use with any other nucleic acids or transfection agents or combinations thereof.

In any procedure, one should have the following materials prepared beforehand: siRNA of interest (20 pmol/ul); prewarmed Opti-MEM® I Reduced Media (Invitrogen); and 24-well tissue culture plates and other tissue culture supplies. The cells to be transfected should be about 30 to about 50% confluent, and cell populations are preferably determined before transfection to comprise at least about 90% viable cells.

The following procedures are used to transfect mammalian cells in a 24-well format. To transfect cells in other tissue culture formats (*e.g.*, 48-, 96-or 384-well plates), optimal conditions for those formats might vary from those given herein for the 24-well format.

6.1. Lipofectamine™ 2000

For transfecting HEK293, BHK, CHO-1, or A549 cells, see Table 4 for suggested transfection conditions. Typically, in RNAi studies using these conditions, a decrease of >50%, preferably >70%, more preferably >80% decrease, most preferably >95% in the expression of a stably integrated reporter gene or an endogenous gene is observed by about 24 to about 48 hours after transfection.

Table 4: siRNA Transfection Conditions for exemplary Cell Lines

| ***ELL LINE*** | CELL DENSITY (CELLS/WELL) | AMOUNT OF LIPOFECTAMINE™ 2000 | AMOUNT OF sIRNA |
|---|---|---|---|
| HEK 293 | 1 x $10^5$ | 1 μl | 20 pmol |
| BHK | 1.5 x $10^4$ | 1 μl | 20 pmol |
| CHO-K1 | 4 x $10^4$ | 1 μl | 20 pmol |
| A549 | 1.5 x $10^4$ | 1 μl | 20 pmol |

1. One day before transfection, plate cells in 0.5 ml of growth medium without antibiotics so that they will be about 30 to about 50% confluent at the time of transfection.
2. For each transfection sample, prepare siRNA:Lipofectamine™ 2000 complexes as follows:

    (a) Dilute the appropriate amount of siRNA in 50 ul of Opti-MEM® Reduced Serum Medium without serum (or other medium without serum). Mix gently.
    (b) Mix Lipofectamine™ 2000 gently before use, then dilute the appropriate amount in 50 ul of Opti-MEM® Medium (or other medium without serum). Mix gently and incubate for 5 minutes at room temperature. Note: Combine the diluted Lipofectamine™ 2000 with the diluted siRNA within 30 minutes. Longer incubation times may decrease activity. If D-MEM is used as a diluent for the Lipofectamine™ 2000, mix with the diluted siRNA within 5 minutes.
    (c) After the 5 minute incubation, combine the diluted siRNA with the diluted Lipofectamine™ 2000 (total volume is 100 ul). Mix gently and incubate for 20 minutes at room temperature.

3. Add the 100 ul of the siRNA/Lipofectamine™ 2000 mixture to each well. Mix gently by, for example, rocking the plate back and forth.

4. Incubate the cells at 37°C in a $CO_2$ incubator for about 24 to about 72 hours until they are ready to be assayed for gene expression. It is generally not necessary to remove the complexes or change the medium; however, growth medium may be replaced after about 4 to about 6 hours without loss of tranfection activity.

6.2. Oligofectamine™

Typically, in RNAi studies of HeLa cells using the following conditions, a decrease of >50%, preferably >70%, more preferably >80% decrease, most preferably >95% in the expression of a stably integrated reporter gene or an endogenous gene is observed by about 24 to about 48 hours after transfection.

1. One day before transfection, plate cells in 0.5 ml of growth medium without antibiotics so that they will be about 50% confluent at the time of transfection.
2. For each transfection sample, prepare siRNA:Oligofectamine™ complexes as follows:

   (a) Dilute 60 pmol of siRNA in 50 ul of Opti-MEM® Reduced Serum Medium without serum (or other medium without serum). Mix gently.
   (b) Mix Oligofectamine™ gently before use, then dilute 3 ul in 12 ul of Opti-MEM® Medium (or other medium without serum). Mix gently and incubate for 5 minutes at room temperature.
   (c) After the 5 minute incubation, combine the diluted siRNA with the diluted Oligofectamine™ (total volume is 68 ul). Mix gently and incubate for 20 minutes at room temperature.

3. Add the 68 ul of the siRNA:Oligofectamine™ mixture to each well. Mix gently by, for example, rocking the plate back and forth.
4. Incubate the cells at 37°C in a CO2 incubator for about 24 to about 72 hours until they are ready to be assayed for gene expression. It is generally not necessary to remove the complexes or change the medium; however, growth medium may be replaced after about 4 to about 6 hours without loss of transfection activity.

Example 10

Exemplary product literature is provided below that describes the generation, purification, and transfection of gene-specific d-siRNA for use in RNA interference analysis, TOPO-mediated generation of templates and production of double-stranded RNA for use in RNA interference analysis. All catalog numbers provided below correspond to Invitrogen Corporation products, Carlsbad, CA, unless otherwise noted.

**D-SIRNA GENERATION AND TRANSFECTION PROCEDURE**

Produce dsRNA

Follow the guidelines to generate dsRNA. If you are using the BLOCK-iT™ Complete Dicer RNAi Kit, refer to the BLOCK-iT™ RNAi TOPO® Transcription Kit manual for instructions to generate dsRNA.

Perform the dicing reaction

1. Set up the following dicing reaction:

| | |
|---|---|
| 10X Dicer Buffer | 30 μl |
| RNase-Free Water | up to 210 μl |
| Purified dsRNA (60 μg) | 1-150 μl |
| BLOCK-iT™ Dicer Enzyme (1U/μl) | 60 μl |
| Total volume | 300 μl |

2. Mix reaction gently and incubate for 14-18 hours at 37°C.
3. Add 6 μl of 50X Dicer Stop Solution.

4. Check integrity of the d-siRNA, if desired. Proceed to purify d-siRNA.

Purify d-siRNA

1. To each 300 μl dicing reaction, add 300 μl of RNA Binding Buffer containing 1% (v/v) β-mercaptoethanol followed by 300 μl of isopropanol. Mix well by pipetting up and down 5 times.
2. Apply half the sample (~450 μl) to the RNA Spin Cartridge, and centrifuge at 14,000 x g for 15 seconds at room temperature. Save the flow-through.
3. Transfer the RNA Spin Cartridge to an siRNA Collection Tube and repeat Step 2, using the other half of the dicing reaction sample (-450 μl). Save the flow-through.
4. Transfer the flow-through from Step 2 to the siRNA Collection Tube containing the flow-through from Step 3. Add 600 μl of isopropanol and mix well by pipetting up and down 5 times.
5. Apply one-third of the sample (~500 μl) to a new RNA Spin Cartridge. Centrifuge at 14,000 x g for 15 seconds at room temperature. Discard the flow-through.
6. Repeat Step 5 twice, applying one-third of the remaining sample (~500 μl) to the RNA Spin Cartridge each time.
7. Add 500 μl of 1X RNA Wash Buffer to the RNA Spin Cartridge, and centrifuge at 14,000 x g for 15 seconds at room temperature. Discard the flow-through.
8. Repeat Step 7.
9. Centrifuge the RNA Spin Cartridge at 14,000 x g for 1 minute at room temperature.
10. Remove the RNA Spin Cartridge from the Wash Tube and place it in an RNA Recovery Tube.
11. Add 30 μl of RNase-Free Water to the RNA Spin Cartridge. Let stand at room temperature for 1 minute, then centrifuge the RNA Spin Cartridge at 14,000 x g for 2 mintues at room temperature to elute the d-siRNA.
12. Add 30 μl of RNase-Free Water to the RNA Spin Cartridge and repeat Step 11, eluting the d-siRNA into the same RNA Recovery Tube.
13. Add 1.2 μl of 50X RNA Annealing Buffer to the eluted d-siRNA.
14. Quantitate the yield of d-siRNA by spectrophotometry. Aliquot and store the d-siRNA at -80°C.

Transfect d-siRNA

Follow the procedure below to transfect cells using Lipofectamine™ 2000. Refer to later table for the appropriate reagent amounts and volumes to add for different tissue culture formats.

1. One day before transfection, plate cells in growth medium without antibiotics such that they will be 30-50% confluent at the time of transfection.
2. For each transfection sample, prepare d-siRNA:Lipofectamine™ 2000 complexes as follows:

    (a) Dilute d-siRNA in the appropriate amount of Opti-MEM® I Reduced Serum Medium without serum. Mix gently.
    (b) Mix Lipofectamine™ 2000 gently before use, then dilute the appropriate amount in Opti-MEM® I. Mix gently and incubate for 5 minutes at room temperature.
    (c) After the 5 minute incubation, combine the diluted d-siRNA with the diluted Lipofectamine™ 2000. Mix gently and incubate for 20 minutes at room temperature.

3. Add the d-siRNA:Lipofectamine™ 2000 complexes to each well containing cells and medium. Mix gently by rocking the plate back and forth.
4. Incubate the cells at 37°C in a CO2 incubator until they are ready to assay for gene knockdown.

Purifying Diced siRNA (d-siRNA)

Introduction

This section provides guidelines and instructions to purify the d-siRNA produced in the dicing reaction. Use the BLOCK-iT™ RNAi Purification reagents (Box 2) supplied with the kit.

Before proceeding to transfection, note that you should purify the d-siRNA produced in the dicing reaction to remove contaminating long dsRNA duplexes. Transfection of unpurified d-siRNA can trigger the interferon-mediated response and cause host cell shutdown and cellular apoptosis. When purifying d-siRNA, follow the purification procedure provided below exactly as instructed. This procedure is optimized to allow removal of contaminating long dsRNA and recovery of high yields of d-siRNA.

Experimental Outline

To purify d-siRNA, you will:

1. Add RNA Binding Buffer and isopropanol to the dicing reaction to denature the proteins and to enable the contaminating dsRNA to bind to the column.
2. Add half the volume of the sample to an RNA spin cartridge. The dsRNA binds to the silica-based membrane in the cartridge, and the d-siRNA and denatured proteins flow through the cartridge. Save the flow-through.
3. Transfer the RNA spin cartridge to an siRNA Collection Tube and add the remaining sample to the RNA spin cartridge. Repeat Step 2. Save the flow-through.
4. Pool the flow-throughs from Step 2 and Step 3 in the siRNA Collection Tube and add isopropanol to the sample to enable the d-siRNA to bind to the column.
5. Add the sample to a second RNA spin cartridge. The d-siRNA bind to the membrane in the cartridge.
6. Wash the membrane-bound d-siRNA to eliminate residual RNA Binding Buffer, isopropanol, and any remaining impurities.
7. Elute the d-siRNA from the RNA spin cartridge with water.
8. Add 50X RNA Annealing Buffer to the eluted d-siRNA to stabilize the d-siRNA for storage.

For an illustration of the d-siRNA purification process, see Figure 8.

Advance Preparation

Before using the BLOCK-iT™ RNA Purification reagents for the first time, add 10 ml of 100% ethanol to the entire amount of 5X RNA Wash Buffer to obtain a 1X RNA Wash Buffer (total volume = 12.5 ml). Place a check in the box on the 5 X RNA Wash Buffer label to indicate that the ethanol was added. Store the 1X RNA Wash Buffer at room temperature.
The RNA Binding Buffer contains guanidine isothiocyanate. This chemical is harmful if it comes in contact with the skin or is inhaled or swallowed. Always wear a laboratory coat, disposable gloves, and goggles when handling solutions containing this chemical.
Do not add bleach or acidic solutions directly to solutions containing guanidine isothiocyanate or sample preparation waste. Guanidine isothiocyanate forms reactive compounds and toxic gases when mixed with bleach or acids.

Materials Needed

Have the following materials on hand before beginning:

Dicing reaction (from Step 5)
RNA Binding Buffer (supplied with the kit, Box 2) β-mercaptoethanol
Isopropanol
RNA Spin Cartridges (supplied with the kit, Box 2; two for each sample)
siRNA Collection Tube (supplied with the kit, Box 2)
1X RNA Wash Buffer (see Advance Preparation, above)
RNase-Free Water (supplied with the kit, Box 2)
RNA Recovery Tube (supplied with the kit, Box 2)
50X RNA Annealing Buffer (supplied with the kit, Box 2)
RNase-free supplies

d-siRNA Purification Procedure

Use this procedure to purify d-siRNA produced from dicing 60 μg of dsRNA in a 300 μl reaction volume (see Step 5). If you have digested < 60 μg of dsRNA and have scaled down the volume of your dicing reaction, scale down the volume of your purification reagents proportionally. For example, if you have digested 30 μg of dsRNA in a 150 μl dicing reaction, scale down the volume of purification reagents used by half.
Before beginning, remove the amount of RNA Binding Buffer needed and add β-mercaptoethanol to a final concentration of 1% (v/v). Use fresh and discard any unused solution.

1. To each dicing reaction (~300 μl volume), add 300 μl of RNA Binding Buffer containing 1% (v/v) β-mercaptoethanol followed by 300 μl of isopropanol to obtain a final volume of 900 μl. Mix well by pipetting up and down 5 times.
2. Apply half of the sample (~450 μl) to the RNA Spin Cartridge.
Centrifuge at 14,000 x g for 15 seconds at room temperature.
3. Transfer the RNA spin cartridge to an siRNA Collection Tube. Save the flow-through containing d-siRNA from Step 2.
4. Apply the remaining half of the sample (-450 μl) to the RNA Spin Cartridge. Centrifuge at 14,000 x g for 2 minutes at room temperature.
5. Remove the RNA Spin Cartridge from the siRNA Collection Tube and discard. Save the flow-through containing d-siRNA.
6. Transfer the flow-through from Step 2 (~450 μl) to the siRNA Collection Tube containing the flow-through from Step 4 (~450 μl) to obtain a final volume of ~900 μl. Add 600 μl of isopropanol to the sample to obtain a final volume of 1.5 ml. Mix well by pipetting up and down.
7. Apply one-third of the sample (~500 μl) to a new RNA Spin Cartridge.
Centrifuge at 14,000 x g for 15 seconds at room temperature. Discard the flow-through.
8. Repeat Step 7 twice, applying one-third of the remaining sample (-500 μl) to the RNA Spin Cartridge each time.
9. Add 500 μl of 1X RNA Wash Buffer to the RNA Spin Cartridge containing bound d-siRNA. Centrifuge at 14,000 x g for 15 seconds at room temperature. Discard the flow-through.
10. Repeat the wash step (Step 9).
11. Centrifuge the RNA Spin Cartridge at 14,000 x g for 1 minute at room temperature to remove residual 1X RNA Wash Buffer from the cartridge and to dry the membrane.
12. Remove the RNA Spin Cartridge from the Wash Tube, and place it in an RNA Recovery Tube.
13. Add 30 μl of RNase-Free Water to the RNA Spin Cartridge. Let stand at room temperature for 1 minute, then centrifuge the RNA Spin Cartridge at 14,000 x g for 2 minutes at room temperature to elute the d-siRNA. Proceed to Step 14.
14. Add 30 μl of RNase-Free Water to the RNA Spin Cartridge and repeat Step 13, eluting the d-siRNA into the same RNA Recovery Tube. The total volume of eluted d-siRNA is 60 μl.
15. Add 1.2 μl of the 50X RNA Annealing Buffer to the eluted d-siRNA to obtain a final concentration of 1X RNA Annealing Buffer. Adding RNA Annealing Buffer to the sample increases the stability of the d-siRNA.
16. Proceed to quantitate the concentration of your purified d-siRNA (see Determining the Purity and Concentration of d-siRNA, below).
17. Store the purified d-siRNA at -80°C. Depending on the amount of d-siRNA produced and your downstream application, you may want to aliquot the d-siRNA before storage at -80°C.

When using the d-siRNA, avoid repeated freezing and thawing as d-siRNA can degrade with each freeze/thaw cycle.

Purifying d-siRNA

The table below lists some potential problems and possible solutions that may help you troubleshoot the purification procedure.

| Problem | Reason | Solution |
|---|---|---|
| Low yield of purified d-siRNA obtained | Eluted d-siRNA from the RNA Spin Cartridge using TE Buffer | Elute d-siRNA from the RNA Spin Cartridge using water. |
| | Concentration of d-siRNA incorrectly determined<br>• Sample diluted into water for spectrophoto metry<br>• Sample blanked against water | • Dilute sample in 1X RNA Annealing Buffer for spectrophotometry.<br>• Blank sample against 1X RNA Annealing Buffer. |
| No d-siRNA obtained | Forgot to add ethanol to the 5X RNA Wash Buffer | Add 10 ml of ethanol to the 5X RNA Wash Buffer (2.5 ml) to obtain a 1X RNA Wash Buffer. |

(continued)

| Problem | Reason | Solution |
|---|---|---|
| | Forgot to add isopropanol to the combined flow-throughs from the first RNA Spin Cartridge | You should add isopropanol to the combined flow-throughs from the first RNA Spin Cartridge to enable the d-siRNA to bind to the second RNA Spin Cartridge. |
| | Forgot to keep flow-throughs from the first RNA Spin Cartridge | Keep the flow-throughs from the first RNA Spin Cartridge (Steps 3 and 5). The flow-throughs contain the d-siRNA. |
| dsRNA present in purified d-siRNA sample | Forgot to add isopropanol to the dicing reaction | You should add RNA Binding Buffer containing 1% (v/v) β-mercaptoethanol and isopropanol to the dicing reaction to denature the proteins and enable the dsRNA to bind the first RNA Spin Cartridge. |
| | Added the mixture containing the flow-through and isopropanol from the first RNA Spin Cartridge (Step 6) back onto the first RNA Spin Cartridge | You should add the mixture containing the flow-through and isopropanol from the first RNA Spin Cartridge (Step 6) to a second RNA Spin Cartridge as the first RNA Spin Cartridge contains bound dsRNA. |
| A260/A280 ratio not in the 1.9-2.2 range | Sample was not washed with 1X RNA Wash Buffer | Wash the RNA Spin Cartridge containing bound d-siRNA twice with 1X RNA Wash Buffer (see Steps 9 and 10). |
| | RNA Spin Cartridge containing bound d-siRNA not centrifuged to remove residual 1X RNA Wash Buffer | Centrifuge RNA Spin Cartridge at 14,000 x g for 1 minute at room temperature to remove residual 1X RNA Wash Buffer and to dry the membrane (see Step 11). |

Purifying RNA Transcripts

This section provides guidelines and instructions to purify the single-stranded RNA transcripts (ssRNA) produced in the RNA transcription reaction. Use the BLOCK-iT™ RNA Purification reagents (Box 3) supplied with the kit. Remember that for each gene, you will perform 2 purification reactions to purify sense and antisense RNA transcripts.

Experimental Outline

To purify RNA transcripts, you will:

1. Add RNA Binding Buffer and ethanol to the transcription reaction to denature the proteins and to enable the ssRNA to bind to the column.
2. Add the sample to an RNA spin cartridge. The ssRNA binds to the silica-based membrane in the cartridge, and the digested DNA, free NTPs, and denatured proteins flow through the cartridge.
3. Wash the membrane-bound ssRNA to eliminate residual RNA Binding Buffer and any remaining impurities.
4. Elute the ssRNA from the RNA spin cartridge with water.

Advance Preparation

Before using the BLOCK-iT™ RNA Purification reagents for the first time, add 10 ml of 100% ethanol to the entire amount of 5X RNA Wash Buffer to generate a 1X RNA Wash Buffer (total volume = 12.5 ml). Place a check in the box on the 5X RNA Wash Buffer label to indicate that the ethanol was added. Store the 1X RNA Wash Buffer at room temperature.

The RNA Binding Buffer contains guanidine isothiocyanate. This chemical is harmful if it comes in contact with the skin or is inhaled or swallowed. Always wear a laboratory coat, disposable gloves, and goggles when handling solutions containing this chemical.

References

Anandalakshmi, R., Pruss, G. J., Ge, X., Marathe, R., Mallory, A. C., Smith, T. H., and Vance, V. B. (1998). A Viral Suppressor of Gene Silencing in Plants. Proc. Natl. Acad. Sci. USA 95, 13079-13084.

Andersson, S., Davis, D. L., Dahlbäck, H., Jörnvall, H., and Russell, D. W. (1989). Cloning, Structure, and Expression of the Mitochondrial Cytochrome P-450 Sterol 26-Hydroxylase, a Bile Acid Biosynthetic Enzyme. J. Biol. Chem. 264, 8222-8229.

Ausubel, F. M., Brent, R., Kingston, R. E., Moore, D. D., Seidman, J. G., Smith, J. A., and Struhl, K. (1994). Current Protocols in Molecular Biology (New York: Greene Publishing Associates and Wiley-Interscience).

Bernstein, E., Caudy, A. A., Hammond, S. M., and Hannon, G. J. (2001). Role for a Bidentate Ribonuclease in the Initiation Step of RNA Interference. Nature 409, 363-366.

Billy, E., Brondani, V., Zhang, H., Muller, U., and Filipowicz, W. (2001). Specific Interference with Gene Expression Induced by Long, Double-Stranded RNA in Mouse Embryonal Teratocarcinoma Cell Lines. Proc. Natl. Acad. Sci. USA 98, 14428-14433.

Boshart, M., Weber, F., Jahn, G., Dorsch-Häsler, K., Fleckenstein, B., and Schaffner, W. (1985). A Very Strong Enhancer is Located Upstream of an Immediate Early Gene of Human Cytomegalovirus. Cell 41, 521-530.

Bosher, J. M., and Labouesse, M. (2000). RNA Interference: Genetic Wand and Genetic Watchdog. Nature Cell Biol. 2, E31-E36.

Caplen, N. J., Fleenor, J., Fire, A., and Morgan, R. A. (2000). dsRNA-Mediated Gene Silencing in Cultured Drosophila Cells: A Tissue Culture Model for the Analysis of RNA Interference. Gene 252, 95-105.

Cogoni, C., and Macino, G. (1999). Gene Silencing in Neurospora crassa Requires a Protein Homologous to RNA-Dependent RNA Polymerase. Nature 399, 166-169.

Cogoni, C., and Macino, G. (1997). Isolation of Quelling-Defective (qde) Mutants Impaired in Posttranscriptional Transgene-Induced Gene Silencing in Neurospora crassa. Proc. Natl. Acad. Sci. USA 94, 10233-10238.

Cogoni, C., Romano, N., and Macino, G. (1994). Suppression of Gene Expression by Homologous Transgenes. Antonie Van Leeuwenhoek 65, 205-209.

Fire, A., Xu, S., Montgomery, M. K., Kostas, S. A., Driver, S. E., and Mello, C. C. (1998). Potent and Specific Genetic Interference by Double-Stranded RNA in Caenorhabditis elegans. Nature 391, 806-811.

Hammond, S. M., Bernstein, E., Beach, D., and Hannon, G. J. (2000). An RNA-Directed Nuclease Mediates Genetic Interference in Caenorhabditis elegans. Nature 404, 293-296.

Hannon, G. J. (2002). RNA Interference. Nature 418, 244-251.

Innis, M. A., Gelfand, D. H., Sninsky, J. J., and White, T. S. (1990) PCR Protocols: A Guide to Methods and Applications. Academic Press, San Diego, CA.

Jones, A. L., Thomas, C. L., and Maule, A. J. (1998). De novo Methylation and Co-Suppression Induced by a Cytoplasmically Replicating Plant RNA Virus. EMBO J. 17, 6385-6393.

Kaufman, R. J. (1999). Double-Stranded RNA-Activated Protein Kinase Mediates Virus-Induced Apoptosis: A New Role for an Old Actor. Proc. Natl. Acad. Sci. USA 96, 11693-11695.

Kennerdell, J. R., and Carthew, R. W. (1998). Use of dsRNA-Mediated Genetic Interference to Demonstrate that frizzled and frizzled 2 Act in the Wingless Pathway. Cell 95, 1017-1026.

Ketting, R. F., Fischer, S. E., Bernstein, E., Sijen, T., Hannon, G. J., and Plasterk, R. H. (2001). Dicer Functions in RNA Interference and in Synthesis of Small RNA Involved in Developmental Timing in C. elegans. Genes Dev. 15, 2654-2659.

Li, W. X., and Ding, S. W. (2001). Viral Suppressors of RNA Silencing. Curr. Opin. Biotechnol. 12, 150-154.

Misquitta, L., and Paterson, B. M. (1999). Targeted Disruption of Gene Function in Drosophila by RNA Interference (RNAi): A Role for Nautilis in Embryonic Muscle Formation. Proc. Natl. Acad. Sci. USA 96, 1451-1456.

Napoli, C., Lemieux, C., and Jorgensen, R. (1990). Introduction of a Chalcone Synthase Gene into Petunia Results in Reversible Co-Suppression of Homologous Genes in trans. Plant Cell 2, 279-289.

Nelson, J. A., Reynolds-Kohler, C., and Smith, B. A. (1987). Negative and Positive Regulation by a Short Segment in the 5'-Flanking Region of the Human Cytomegalovirus Major Immediate-Early Gene. Molec. Cell. Biol. 7, 4125-4129.

Ngo, H., Tschudi, C., Gull, K., and Ullu, E. (1998). Double-Stranded RNA Induces mRNA Degradation in Trypanosoma brucei. Proc. Natl. Acad. Sci. USA 95, 14687-14692.

Nykanen, A., Haley, B., and Zamore, P. D. (2001). ATP Requirements and Small Interfering RNA Structure in the RNA Interference Pathway. Cell 107, 309-321.

Plasterk, R. H. A., and Ketting, R. F. (2000). The Silence of the Genes. Curr. Opin. Genet. Dev. 10, 562-567.

Romano, N., and Macino, G. (1992). Quelling: Transient Inactivation of Gene Expression in Neurospora crassa by Transformation with Homologous Sequences. Mol. Microbiol. 6, 3343-3353.

Shuman, S. (1994). Novel Approach to Molecular Cloning and Polynucleotide Synthesis Using Vaccinia DNA Topoisomerase. J. Biol. Chem. 269, 32678-32684.

Shuman, S. (1991). Recombination Mediated by Vaccinia Virus DNA Topoisomerase I in Escherichia coli is Sequence Specific. Proc. Natl. Acad. Sci. USA 88, 10104-10108.
Smith, C. J., Watson, C. F., Bird, C. R., Ray, J., Schuch, W., and Grierson, D. (1990). Expression of a Truncated Tomato Polygalacturonase Gene Inhibits Expression of the Endogenous Gene in Transgenic Plants. Mol. Gen. Genet. 224, 477-481.
Svoboda, P., Stein, P., Hayashi, H., and Schult, R. M. (2000). Selective Reduction of Dormant Maternal mRNAs in Mouse Oocytes by RNA Interference. Development 127, 4147-4156.
van der Krol, A. R., Mur, L. A., Beld, M., Mol, J. N., and Stuitje, A. R. (1990). Flavonoid Genes in Petunia: Addition of a Limited Number of Gene Copies May Lead to a Suppression of Gene Expression. Plant Cell 2, 291-299.
Voinnet, O., Pinto, Y. M., and Baulcombe, D. C. (1999). Suppression of Gene Silencing: A General Strategy Used by Diverse DNA and RNA Viruses of Plants. Proc. Natl. Acad. Sci. USA 96, 14147-14152.
Wianny, F., and Zernicka-Goetz, M. (2000). Specific Interference with Gene Function by Double-Stranded RNA in Early Mouse Development. Nature Cell Biol. 2, 70-75.
Yang, S., Tutton, S., Pierce, E., and Yoon, K. (2001). Specific Double-Stranded RNA Interference in Undifferentiated Mouse Embryonic Stem Cells. Mol. Cell. Biol. 21, 7807-7816.
Zamore, P. D. (2001). RNA Interference: Listening to the Sound of Silence. Nat. Struct. Biol. 8, 746-750.

Example 11

Small Nucleic Acids Purification System

All catalog numbers provided below correspond to Invitrogen Corporation products, Carlsbad, CA, unless otherwise noted.

Small nucleic acid molecules, especially siRNA, is getting great attention with function in gene specific knockout or silencing of gene expression. Recently, many researchers demonstrated that gene specific siRNA can be generated *in vitro* via a combination of transcription and a ribonuclease enzyme. The digestion of long transcripts is accomplished with a ribonuclease called RNase III or Dicer and the digested sample is required to be purified from the non-processed template, intermediate and buffer component of enzyme reaction. If residual long dsRNA template and other intermediates remained in the sample and were transfected along with siRNA into cells, it might lead to non-specific response. Thus removal of this residual template and intermediate is required for accurate functional analysis of the gene specific siRNA. Pre-existing total RNA purification systems are not suitable and not designed to purify less than 30bp nucleic acids and only a size exclusion spin column has been utilized to select small size nucleic acid from mixtures.
We have developed a buffer formulation to purify dsRNA that is smaller than 30 bp using our pre-existing glass fiber filter. Both single-column and double-column method were developed to purify siRNAs generated using Dicer and RNase III. The purified siRNA can be used to assay cellular functional via gene specific knock out without non-specific interference by >30 bp dsRNA (complete buffer exchange; eluted in DEPC treated $H_2O$). This purification procedure can be utilized for other applications such as linker, aptamer, protein binding domain extraction, etc.

Introduction

Total RNA is composed of three main transcript categories. These are ribosomal RNAs (28S, 18S, and 5S in the case of mammalian cells), mRNA, and low molecular weight RNA species such as tRNA, snRNA, and others. The recent discovery and rudimentary elucidation of the mechanism of action of RNA interference and the identification of a new regulatory RNA termed short interfering RNA (siRNA) as well as micro RNA are receiving increasing attention by the scientific community. This increased interest is based on siRNA's ability to mediate down-regulation of gene expression by sequence specific, and hence gene specific, degradation of targeted mRNA. The popularity of the siRNA approach is justified as it has distinct advantages over anti-sense methods and knockout approaches. It appears that the siRNA approach is capable of down-regulating gene expression with higher efficiency and efficacy than the antisense approach and offers greater flexibility and ease of use compared to knockout approaches.

RNA interference is a cellular defense mechanism where a long double-stranded RNA molecule is processed by an endogenous (endo)ribonuclease resulting in the production of small interfering RNAs (siRNAs), which are generally 21 to 23 nucleotides in length. The siRNA molecules bind to a protein complex, RNA Induced Silencing Complex (RISC), which contains a helicase activity that unwinds siRNA molecules, allowing the anti-sense strand of siRNA to bind to complementary mRNA, thus triggering targeted mRNA degradation by endonucleases or blocking mRNA translation into protein (for a review see Denli and Hannon, 2003, Carrington and Ambros, 2003). In addition, siRNA does not trigger an immune response, because it is a natural cellular mechanism (Sledz *et. al.,* 2003)

Initial attempts of gene specific knockdown using long dsRNA transcripts failed in mammalian cells because of activation of protein kinase PKR and 2', 5'-oligoadenylate synthetase that trigger non-specific shutdown of protein synthesis and non-specific degradation of mRNA. Elbashir and coworkers demonstrated that transfection of chemically synthesized 21-23 nt dsRNA fragments could specifically suppress gene expression without triggering non-specific gene silencing effects in mammalian cells. However, different suppression levels are often observed with synthetic short siRNAs as they target a single specific site. Under these conditions site accessibility becomes an issue as mRNA containing high levels of secondary or tertiary structure may prevent siRNA/target/RISC complex formation and affect efficacy of the siRNA used. Thus, multiple double-stranded siRNA molecules, usually 4 - 5, need to be screened that target different sequences in a targeted mRNA to identify one siRNA construct with adequate potency for gene suppression in a given mRNA. Short interfering RNA constructs can also be generated by transcription *in vitro* from short DNA templates or by transcription *in vivo* from a transfected DNA construct. However, none of the latter methods are easily scaled up for multiple gene screens due to high cost of oligonucleotides and/or difficulties of target region selection. A new method was recently developed to generate gene specific functional siRNA pools using a combination of RNA transcription followed by digestion with Dicer enzyme. This method generates multiple functional siRNAs from long dsRNA target sequences which are correspond to the gene transcript of interest. With this new method, low cost and highly efficient screening of gene knockdown effects is possible and high throughput screening of multiple genes can be achieved. However, the latter methodology requires purification of functional siRNA after digestion of long dsRNA substrate with Dicer. Undigested, long dsRNA substrates as well as intermediate digestion products longer than approximately 30 bp elicit non-specific responses such as non-specific shutdown of translation and initiation of apoptosis (Kaufman, 1999). Others have used size exclusion columns for purification of functional siRNA. However, this purification is not efficient and does not provide high quality siRNA for transfection.

Our Small Nucleic Acids Purification System provides an efficient means of purification for functional, diced siRNA and other small dsRNA molecules. The purification is based on glass fiber purification technology. The small nucleic acids purification system eliminates dsRNA that exceeds 30 bp in length and selectively and specifically purifies dsRNA shorter than 30 base pairs. In the case of siRNA, the purified dsRNA is of high quality, highly functional for transcript specific gene suppression, and exhibits no cell toxicity. Currently, Invitrogen Block-iT™ Dicer RNAi Kits provide complete Dicer RNAi transfection kit, include RNAi purification kit, Dicer Enzyme kit, Lipofectamine™ 2000 Reagent and/or TOPO® transcription Kit, as bundle product. Small Nucleic Acids Purification kit is a stand-alone product of the siRNA purification module from Block-iT™ that accommodates not only siRNA purification generated by Dicer and RNaseIII but also other small nucleic acids applications. The Small Nucleic Acids Purification Kit, as related to the purification of enzymatically-generated siRNA (Dicer & RNaseIII), will generally meet the following criteria: (1) Purified siRNA expected not to contain dsRNA molecules greater than 30 bp in length, (2) Suppression levels observed with purified siRNA will be the same or higher than those observed with synthesized siRNA, (3) Recovery of purified material expected to exceed 80%.

Spin Column Purification Kit Components

1. 50 individual spin columns assembled in collection tubes in one bag
2. 50 individual recovery tubes in one bag
3. Binding Buffer (47-6001): 11 mL
4. 5x Wash Buffer (47-6003): 15 mL, EtOH (95-100%) added by end user
5. Elution Buffer (47-6002): 3 mL, 1.5 mL EtOH (95-100%) and 1.5 mL RNase-free water to be added by end user
6. DEPC water (47-0005): 10 mL
7. Manual
8. QRC

The components provided in the kit are sufficient for 50 purifications using the single-column protocol, in which a final ethanol precipitation step in the presence of glycogen as a co-precipitant is desired. The components provided in the kit are sufficient for 25 purifications when using the two-column protocol, in which the second column is used for selective binding of the short target nucleic acids followed by elution in DEPC-treated water to obtain the final, purified product (see Purification Protocol Flowchart)

Opitional Materials:

Crude small nucleic acids preparation for purification
Materials for generating long dsRNA template
Materials for digestion of long dsRNA template to generate crude siRNA product

Chemically synthesized siRNA
EtOH (95 or 100%)
UltraPure ™Glycogen (20 µg/µl) (Invitrogen cat #10814-010)

Purification Protocol Flowchart

The Small Nucleic Acids Purification System is designed to purify Micro-RNA molecules such as micro RNA, tiny RNA, small nuclear RNA, guide RNAs, telomerase RNA, small non-mRNA, catalytic RNA, and small regulatory RNAs (such as aptamer). Also, RNAi molecules RNase III-generated diced siRNA (15-16 bp), Dicer-generated siRNA (21-23 bp), other short hairpin RNA, and small temporary regulatory RNA can be purified with the Small Nucleic Acids Purification System.

| **Single-Column Protocol **** | **Two-Column Protocol*** |
|---|---|
| Add 150 µl of Binding Buffer to 50 µl of sample reaction volume* and mix it well (Total volume: 200 µl) | Add 50 µl of Binding Buffer to 50 µl of sample reaction volume* and mix it well. (Total volume 100 µl) |
| Add 600 µl of EtOH (95-100%) (Final EtOH concentration 71-75%, total volume: 800 µl) | Add 50 µl of EtOH (95-100%) (Final EtOH concentration 31-33%, sample volume: 150 µl) |
| Mix sample well and load onto spin column | |
| Centrifuge at 20,000 x g for 1 min | |
| Expected recovery volume: ca. 750 µl | Expected recovery volume: ca. 130 µl |
| | Remove spin column from collection tube |
| | Add 185 µl of EtOH (95 -100%) to pass-through and mix it well. (Final EtOH conc. ca. 70 - 74%) |
| | Load sample onto **2nd column** |
| | Centrifuge at 20,000 x g for 1 min |
| Wash spin column with 500 µl of 1X Wash Buffer | |
| Repeat the washing step (optional) | |
| Centrifuge at 20,000 x g for 1 min to dry the filter | |
| Add 100 µl of **Elution Buffer** to dried spin column and incubate at ambient temperature for 1 min | Add 100 µl of ***DEPC-treated water*** to dried spin column & incubate at ambient temperature for 1 min |
| **Single-Column Protocol **** | **Two-Column Protocol*** |
| Centrifuge at 20,000 x g for 1 min Expected elution volume: ca. 95 µl | |
| **The eluate contains the purified, short dsRNA** | |
| **EtOH precipitation of short nucleic acids:**<br>a. Add 200 µl of ice cold 100% EtOH and 1 µl glycogen solution (20 µg/µl).<br>b. Incubate at -20 °C for 15 min and centrifuge for 15 min at 20,000 x g<br>C. Discard supernatant carefully and wash pellet with 0.5 ml of 70% EtOH<br>d. Centrifuge for 10 min at 20,000 x g<br>e. Discard supernatant and air dry pellet<br>Resuspend pellet of **purified, short dsRNA** in 50 µl (or desirable amount) of DEPC-treated water | |
| *Higher sample reaction volumes may require proportionally increased Binding Buffer and EtOH volumes. (Two-Column protocol provide here is scaled down procedure from siRNA purification kit module of Block-iT(Dicer RNAi Kit). Either EtOH or isopropanol can be used to mixing step with Binding Buffer. | |

(continued)

| Single-Column Protocol ** | Two-Column Protocol* |
|---|---|
| **Single column purification will limit its reaction volume to 500μl reaction. (up to 10 μg of dsRNA reaction). | |

Please see detail description of Purification of Small Nucleic Acids-General Consideration in ***Results and Discussion*** section.

Materials and Methods

Generation of dsRNA and siRNA

Crude siRNA needed for purifications was generated in a two-step process. First, *in-vitro* T7 RNA polymerase transcription reaction was used to generate the individual strands that form dsRNA, which then, in a second reaction, served as a template for either Dicer or RNase III digestion yielding crude siRNA preparations that were used for purification with the new kit. The genes of LacZ (Accession number: AY150267) and Luciferase (Accession number: AAL30778.1) were selected as the target genes for siRNA inhibition. LacZ dsRNA template was generated as follows: (1) PCR was performed with lacZ gene-specific primer 1 (5'-ACC AGA AGC GGT GCC GGA AA -3' (SEQ ID NO: 2)) and primer 2 (5'- CCA CAG GGG ATG GTT CGG AT-3' (SEQ ID NO: 3)), (2) PCR was performed to incorporate T7 sequences at both ends of the amplicon generated in step 1 with Primer 3 (5'-GAC TCG TAA TAC GAC TCA CTA TAG GGA CCA GAA GCG GTG CCG GAA A -3' (SEQ ID NO: 8)) and primer 4 (5'- GAC TCG TAA TAC GAC TCA CTA TAG GGC CAC AGC GGA TGG TTC GGA T-3' (SEQ ID NO: 9)). The resulting amplicon was purified with Qiagen's PCR clean up kit (QIAquick PCR Purification Kit, cat # 28104) and used as template for the T7 RNA polymerase reverse transcription reaction to generate dsRNA. Long dsRNA was treated in a final step before Dicer or RNaseIII digestion with DNase I and RNaseA to remove template DNA and unhybridized single-stranded RNA. Luciferase specific dsRNA was generated analogously using the following primer sets: primer 5 (5'-TGA ACA TTT CGC AGC CTA CC-3' (SEQ ID NO: 4)) and primer 6 (5'- GCC ACC TGA TAT CCT TT- 3' (SEQ ID NO: 10)) for the first round of PCR, primer 7 (5'-GAC TCG TAA TAC GAC TCA CTA TAG GGT GAA CAT TTC GCA GCC TAC C-3' (SEQ ID NO: 11)) and primer 8 (5'-GAC TCG TAA TAC GAC TCA CTA TAG GGG CCA CCT GAT ATC CTT T -3' (SEQ ID NO: 12)) for the second round of PCR. Plasmids containing the LacZ and Lucifease gene used as templates (pcDNA1.2/V5/GW-lacZ and pcDNA5-FRT-luc). These two plasmids were also used for transfection to serve as reporter plasmid for functional testing. The two plasmids used are components of the BLOCK-iT™ Dicer RNAi Kit (Invitrogen, cat. # K3600-01). Double-stranded RNA, which was to serve as template for siRNA generation, was purified using the glass fiber filter columns developed for siRNA purification as well as with Ambion's purification columns and protocol. Purified dsRNA template was digested with either Dicer (Invitrogen) or RNase III (Ambion) to generate functional siRNA. The latter was purified using the single-column as well as the two-column protocol outlined above.

Mammalian Cell Culture and Transfection

For functional testing GripTite™ 293 MSR cells (Invitrogen, cat. # R79507) and F1 pIn 293 cells were used. GripTit™e 293 MSR cells were cultured in DMEM containing 4 mM L-glutamine, 10% FBS, and 600 μg/ml geneticin (Invitrogen, cat.# 11811-023). In co-transfection experiments 100 ng of each reporter plasmid (see above) was co-transfected with either unpurified siRNA, purified siRNA, or synthetic siRNA specific for lacZ or for Green Fluorescent Protein (GFP) into 90% confluent GripTite™ 293 cells plated at 2 x 105 cells/well. F1 pIn 293 cells (FlpIn 293 luc) expressing luciferase from a single integrated copy were used to test luciferase specific siRNA. LacZ activity was also monitored as a control to assess any general, non-specific changes in mRNA expression. F1 pIn 293 cells were cultured in DMEM containing 4 mM L-glutamine, 10% FBS, and 100 μg/ml hygromycin B (Invitrogen, cat. #10687-010). Cells were seeded in 24-well plates and grown to 30 - 50% confluence before transfection with siRNA.

β-Galactosidase and Luciferase Assays

Activity and specificity of siRNA transfected was assessed by monitoring the activity of the reporter gene products luciferase and β-galactosidase. One to two days after transfection the medium was removed from each well of the 24-well plates and replaced with 500 μl cold luciferase lysis buffer from Promega (25 mM Tris-HCl pH 8.0, 0.1 mM EDTA pH 8.0, 10% v/v glycerol, 0.1% v/v Triton X-100). Plates were then frozen at-80°C for at least 1 hour. Samples were thawed for 30 min at RT and 50 μl (for luciferase assay) or 10 μl (for β-galactosidase assay) were transferred to a black 96-well plate. For β-galactosidase, 90 μl of Reaction Dilution Buffer containing 1% (v/v) Galacton-Plus® (Applied Biosystems, cat # T1006) was added to each sample and incubated for 30 min at room temperature. Luminescence was measured on a MicroLumat Plus luminometer using Winglow v.1.24 software (EG&G Berthold). For luciferase, either 50 μl of Luciferase Assay Reagent (Promega, cat # E1483) or 100 μl Accelerator II (Tropix) were injected per well and readings were taken for 5 seconds after a 2-second delay.

Other Materials Used

i. Silencer siRNA Cocktail Kit (Cat. no. 1625, Ambion Inc.)
ii. RNA purification Column 1 and 2 (Cat. no., T510004, T510005, Gene Therapy Systems, Inc.)
iii. Yeast tRNA (Cat. no. 15401-011, Invitrogen Inc.)
iv. E-Gel 4% (Cat. no. G5018-04, Invitrogen Inc.)
v. 10 bp DNA ladder (Cat. no. 10821-015, Invitrogen Inc.)

Results and Discussion

Purification of Small Nucleic Acids - General Considerations

Commercially available kits for the isolation and purification of double-stranded nucleic acids, RNA as well as DNA, generally do not address the need for purification of short double-stranded nucleic acids. A notable exception is the use of size exclusion filtration technology. However, this technology suffers from several drawbacks (limited automation capabilities, broad cut-off size ranges, low recoveries, etc.) that have limited its use. Short double-stranded nucleic acids shall be defined here as nucleic acids that are shorter than about 100 bp in length. Ribonucleic acids falling into this category include, but are not limited to, RNA species that are described in the literature as tiny RNA, small RNA (sRNA), non-coding RNA (ncRNA), micro-RNA (miRNA), small non mRNA (snmRNA), functional RNA (fRNA), transfer RNA (tRNA), catalytic RNA such as ribozymes, small nucleolar RNA (snRNA), short hairpin RNA (shRNA), small temporally regulated RNA (strRNA), aptamers, and RNAi molecules including without limitation small interfering RNA (siRNA). With recent developments in the field of RNAi/siRNA technology, a particular need for the purification of siRNA from crude enzymatic preparations of siRNA has become apparent. Small interfering RNAs (siRNA) are small dsRNA molecules in the size range of approximately 12 to 25 bp, which can be generated either enzymatically or chemically (Elbashir *et. al.,* 2001). Short, deoxyribonucleic acids potentially requiring purification may comprise, but are not limited to, dsDNA molecules such as adapters, linkers, short restriction fragments and PCR products. The purification system described here is based on nucleic acids binding to a glass fiber filter under controlled conditions permitting size-dependant, efficient; high-recovery, high-purity purification of short nucleic acids.

Two general approaches for the purification of small nucleic acids are outlined in the purification protocol flowchart above pertaining to the purification of enzymatically-generated siRNA. Using a single-column protocol, all double-stranded nucleic acids exceeding a length of approximately 10 base pairs are bound to the glass fiber matrix during an initial step in the presence of a chaotropic salt, which is contained in the Binding Buffer, and EtOH in excess of 70% (v/v). The binding step is followed by a wash step, which removes non-nucleic acids components from the target nucleic acids bound to the glass fiber matrix. In a third step small nucleic acids are selectively eluted in Elution Buffer containing a controlled amount of EtOH that is specific for the release of the targeted nucleic acids size range. Nucleic acids exceeding the targeted size range will remain bound to the glass fiber filter. In the case of siRNA, either generated by Dicer or RNaseIII digestion of larger dsRNA template molecules, the optimal concentration of EtOH was determined to be 25% (v/v). Use of the single-column protocol for small nucleic acids purification typically employs a final EtOH precipitation step in order to remove chaotropic salts, which are present in the Elution Buffer, followed by resuspension of purified nucleic acids in a buffer of choice.

In the two-column protocol double-stranded nucleic acids fragments exceeding a length of approximately 30 base pairs are bound to the glass fiber matrix during the initial binding step, while fragments shorter than approximately 30 base pairs are washed through the glass fiber matrix and are recovered in the flow through. This size fractionation is achieved by applying the mixture of nucleic acids fragment of various sizes in a Binding Buffer containing a chaotropic salt and a controlled concentration of EtOH. In the case of siRNA the optimal concentration of EtOH was determined to be approximately 33% (v/v). The size cut-off for flow through of short nucleic acids can be fine tuned by adjusting the relative amount of EtOH contained in the binding solution. Increased EtOH concentrations result in retention of shorter nucleic acid fragments on the glass fiber filter, while decreased EtOH concentrations in the binding solution result in the elution of larger nucleic acids fragments. In a second step the EtOH concentration of the flow through from the first column containing the small nucleic acids of interest is increased to >70% (v/v) and applied to a second glass fiber filter column. Under these conditions small, double-stranded nucleic acids are bound to the matrix of the second filter column.

This second binding step is followed by a wash step, which removes any remaining non-nucleic acid components from the targeted small nucleic acids bound to the glass fiber matrix. In a final step the targeted small nucleic acids are eluted off the glass fiber matrix at low ionic strength with water.

The single-column and the two-column protocol provide two alternatives for the purification of small nucleic acids molecules. The single-column protocol is more economical as it uses only one filtration step. However, this protocol typically employs a final EtOH precipitation step, which is more time consuming than a filtration step and holds the risk of incomplete nucleic acid precipitation. On the other hand, EtOH precipitation is generally considered to yield a cleaner nucleic acid preparation. The two-column protocol, while more costly per sample purification, is generally faster than the single-column protocol by virtue of avoiding the EtOH precipitation step.

Functional Testing of Purified siRNA (Single-Column and Two-Column Protocol Comparison)

Experimental Setup

As outlined above in the Purification Protocol Flowchart, two alternative purification approaches are feasible depending mainly on individual preferences regarding ethanol precipitation and procedure time. In the following experiments, which are illustrated in Figures 19A-19C, lacZ siRNA was purified according to the single-column and two-column protocols described earlier. The siRNA obtained was tested for specificity and functionality in transfection experiments using GripTite™ 293 MSR cells, which contained either luciferase or β-galactosidase gene constructs in reporter plasmids, as described in detail above. In the case of the single-column purification protocol, elution was performed with Elution Buffer containing ethanol at final concentrations of between 5 and 30%. Transfection experiments were performed in duplicate.

Results and Discussion

Figure 19A shows gel analysis results of crude lacZ siRNA, siRNA purified using the two-column protocol, various fractions of the single-column purification protocol, as well as chemically synthesized siRNA analyzed on a 4% E-Gel, which were used for functional testing. Green Fluorescent Protein (GFP) siRNA, by virtue of being chemically synthesized, does not contain any long dsRNA impurities. The siRNA that was purified with the two-column protocol and siRNA fractions eluted with 20, 25, and 30% EtOH using the single-column protocol appear to be devoid of intermediate Dicer reaction products and full-length dsRNA template. Therefore, these siRNA preparations are expected to be potent and specific in the suppression of their target genes and are not expected to exhibit any of the adverse effects associated with the presence of long dsRNA. Unpurified lacZ siRNA contains significant amounts of undigested and partially digested long dsRNA molecules and is hence expected to result in cell death upon transfection. Likewise, siRNA purified with the single-column protocol and eluted with Elution Buffer containing 5, 15, and 20% EtOH is expected to result in cell death, albeit at decreasing degrees as ethanol concentration increases.

Figure 19 A:

Lane 1: 10 bp DNA Ladder (Invitrogen Cat #18021-015) - The 10-bp fragment only shows as a faint band.
Lane 2: Chemically synthesized, unpurified Green Fluorescent Protein (GFP) siRNA
Lane 3: Crude lacZ siRNA reaction mixture
Lane 4: LacZ siRNA purified using the two-column protocol (see flowchart above)
Lane 5: LacZ siRNA eluted with 5% EtOH-containing Elution Buffer according to the single-column protocol
Lane 6: LacZ siRNA eluted with 10% EtOH-containing Elution Buffer according to the single-column protocol
Lane 7: LacZ siRNA eluted with 15% EtOH-containing Elution Buffer according to the single-column protocol
Lane 8: LacZ siRNA eluted with 20% EtOH-containing Elution Buffer according to the single-column protocol
Lane 9: LacZ siRNA eluted with 25% EtOH-containing Elution Buffer according to the single-column protocol
Lane 10: LacZ siRNA eluted with 30% EtOH-containing Elution Buffer according to the single-column protocol

The effects of lacZ siRNA on luciferase activity are shown in Figure 19B. This is a negative control experiment. Since lacZ siRNA does not have sequence homology to the luciferase gene, its activity should remain unperturbed by the presence of lacZ siRNA. Any changes in luciferase activity will thus be attributed to nonspecific effects such as the effect that the presence of long dsRNA may have on the transfected cells or the effects of transfection itself. Cells that do not carry the reporter plasmid for luciferase (untransfected) do not exhibit luciferase activity. Cells transfected with the reporter plasmid for luciferase (reporters alone) exhibit baseline luciferase activity serving as a point of reference for the action of siRNA in the following experiments. Transfection of cells carrying luciferase reporter plasmid with chemically synthesized, unrelated GFP siRNA (GFP siRNA) did not alter luciferase activity, as expected. Unpurified, crude lacZ Dicer reaction containing undigested and partially digested long dsRNA resulted in cell death and a concomitant lack of luciferase activity (lacZ dicing reaction). Transfection of target cells with lacZ siRNA purified using the two-column purification protocol (lacZ d-siRNA) did not suppress luciferase activity as expected. However, nonspecific induction of luciferase activity by about 40% was apparent. LacZ siRNA obtained by elution with Elution Buffer containing 5, 10, or 15% ethanol using the single-column protocol (lacZ fract 5, lacZ fract 10, lacZ fract 15) resulted in suppression of luciferase activity. This observation, however, is attributed to residual long dsRNA template and partial digestion products thereof in these fractions eliciting cell death as observed for unpurified Dicer reactions. The observed suppression of luciferase activity in these cases is not the result of specific siRNA action. LacZ siRNA obtained by elution with Elution Buffer containing 20, 25, and 30% EtOH did not alter luciferase activity. Hence, these fractions of purified siRNA do not elicit nonspecific effects such as induction or suppression of luciferase activity upon transfection.

The effects of lacZ siRNA on its target transcripts, as evidenced and measurable through the activity of β-galactosidase, are shown in Figure 19C. Cells that do not carry the reporter plasmid for β-galactosidase (untransfected) do not exhibit β-galactosidase activity. Cells transfected with the reporter plasmid for β-galactosidase (reporters alone) exhibit baseline β-galactosidase activity serving as a point of reference for the action of siRNA in the following experiments. Transfection of cells carrying β-galactosidase reporter plasmid with chemically synthesized, unrelated GFP siRNA (GFP siRNA) did not alter β-galactosidase activity. Unpurified, crude lacZ Dicer reaction containing undigested and partially digested long dsRNA resulted in cell death and a concomitant lack of β-galactosidase activity (lacZ dicing reaction). Transfection of target cells with lacZ siRNA purified using the two-column purification protocol (lacZ d-siRNA) suppressed β-galactosidase activity by approximately 70%. LacZ siRNA obtained by elution with Elution Buffer containing 5, 10, or 15% ethanol using the single-column protocol (lacZ fract 5, lacZ fract 10, lacZ fract 15) resulted in suppression of β-galactosidase activity. This observation, however, may be attributed to residual long dsRNA template and partial digestion products thereof in these fractions, eliciting cell death as observed for unpurified Dicer reactions. In addition, β-galactosidase activity in surviving cells may further be suppressed by the presence of siRNA specific for the lacZ gene. Thus, while suppression appears to be efficient, it is mainly caused by cell death and not by the specific action of the siRNA used. LacZ siRNA obtained by elution with Elution Buffer containing 20, 25, and 30% EtOH did profoundly suppress the activity of the β-galactosidase enzyme by approximately 80%. In the latter case cells appeared healthy after transfection with purified siRNA. Hence, these fractions of purified siRNA are highly effective and specific in the suppression of their targeted mRNA.

Fractionated siRNA samples used were obtained using either the single-column or two-column protocol as a means of purification. The effects of lacZ siRNA are specific for the β-galactosidase gene due to sequence homologies and a reduction of β-galactosidase activity is expected as a result of the presence of lacZ siRNA.

Figures 19 B and C:

Untransfected: Cells have not been transfected with reporter plasmids carrying the luciferase or β-galactosidase gene
Reporters alone: Cells have been transfected with reporter plasmid only, but not with siRNA
GFP siRNA: Transfection with chemically synthesized, crude siRNA specific for the green fluorescent protein gene
LacZ dicing reaction: Transfection with crude, unpurified lacZ siRNA from Dicer reaction
LacZ d-siRNA: Transfection with lacZ siRNA purified using the two-column protocol
LacZ frac 5: Transfection with lacZ siRNA from 5% EtOH containing fraction (single-column protocol)
LacZ frac 10: Transfection with lacZ siRNA from 10% EtOH containing fraction (single-column protocol)
LacZ frac 15: Transfection with lacZ siRNA from 15% EtOH containing fraction (single-column protocol)
LacZ frac 20: Transfection with lacZ siRNA from 20% EtOH containing fraction (single-column protocol)
LacZ frac 25: Transfection with lacZ siRNA from 25% EtOH containing fraction (single-column protocol)
LacZ frac 30: Transfection with lacZ siRNA from 30% EtOH containing fraction (single-column protocol)

Purification of siRNA Generated by Dicer or RNase III

Experimental Setup

One-kb dsRNA transcript of either lacZ or luciferase was incubated with Dicer or RNaseIII to generate double-stranded siRNA products. Dicer reactions were carried out using a protocol as described in the BLOCK-iT™ Complete Dicer RNAi Kit (Invitrogen cat. # K3650-01). Digestion with RNaseIII (Ambion, cat. # 2290) was performed according to the manufacturer's suggestions. Crude RNase III and Dicer siRNA reactions were purified using the single-column and two-column purification protocol and subsequently tested for functionality.

Results and Discussion

The Dicer enzyme is a member of the RNaseIII family of ribonucleases and digests long dsRNA templates into 21-23 nucleotide, double-stranded siRNA that have been shown to function as key intermediates in triggering sequence specific RNA degradation during posttranscriptional gene silencing. Likewise, RNaseIII digests long dsRNA templates into short double-stranded siRNA molecules. However, the siRNA generated by RNaseIII is generally only approximately 12 - 15 base pairs long. Dicer enzyme is found in all eukaryotic cells and RNaseIII is mainly found in prokaryotes. Dicer enzyme is speculated to bind to the ends of long dsRNA and progressively cleave the template dsRNA. The mode of action of RNaseIII may involve random cleavage of template dsRNA into smaller, compared to the Dicer enzyme, 12-15 bp siRNA fragments. The RnaseIII enzyme is considerably more active than the corresponding Dicer enzyme, which leads to complete digestion of template dsRNA by RNaseIII enzyme, while Dicer enzyme, even after prolonged digestion, results in only incomplete digestion of template dsRNA. These findings are illustrated in Figure 20A. Neither enzyme requires ATP for function. However, both enzymes require divalent metal cations and a specific, optimal pH range for optimal activity, which are provided by enzyme-specific reaction buffers. The purification procedures for siRNA used here result in the removal of proteins and buffer components. The purified siRNA is resuspended in RNase-free water in the final purification step independent of the purification protocol used. Short interfering RNA generated by the action of RNase III as well as by Dicer enzyme were successfully purified using Invitrogen's spin columns applying either the single- or two-column purification protocol as shown in Figure 20B.

Figures 20A and 20B show purification of siRNA generated with Dicer and RNaseIII

Figure 20A:

Lane 2: unpurified lacZ siRNA cleaved by RNaseIII,
Lane 3 unpurified luciferase siRNA cleaved by RNaseIII,
Lane 4: unpurified lacZ siRNA cleaved by Dicer,
Lane 5: unpurified luciferase siRNA cleaved by Dicer

Figure 20B:

Lane 1, 5,9: lacZ siRNA cleaved by RNaseIII,
Lane 2, 6,10: luciferase siRNA cleaved by RNaseIII,
Lane 3, 7,11 lacZ siRNA cleaved by Dicer,
Lane 4, 8,12 luciferase siRNA cleaved by Dicer

Conclusion

Short interfering RNA generated by digestion of long dsRNA templates with either Dicer or RNaseIII enzyme can be efficiently purified using the single-column or two-column purification protocol.

Functional Testing of SiRNA Preparations with F1pIn 293 luc Cells

Experimental Setup

Short interfering RNA was generated by digestion of long dsRNA templates (1μg) with either RNase III or Dicer enzyme. Samples were purified using the single- and two-column purification protocol. Concentrations of purified siRNA were determined by A260 measurements and 20 ng of purified sample was used for each transfection into F1pIn 293 luc cells.

Results and Discussion

Transfection experiments were performed in 5 experimental groups with each experiment being conducted in duplicate. Experimental group 1 consisted of two control reactions of mock transfected (Mock), i.e. transfection with transfection agent but without siRNA, as well as F1pIn 293 luc cells expressing baseline levels of luciferase (Untransfected). The luciferase activity was measured in these latter two experiments and served as reference for luciferase activity that was determined in group 2-5 experiments. In experimental groups 2 and 3 the effect of luciferase activities by Dicer generated luciferase specific siRNA (luc siRNA) and β-galactosidase specific siRNA (lacZ siRNA) were assessed. Likewise, in groups 4 and 5 the effect of siRNA generated with RNaseIII enzyme on luciferase activity was assessed.

siRNA (20 ng luc Dicer reaction) resulted in cell death and nonspecific lack of luciferase activity. SiRNA that was purified using the single-column purification protocol, where siRNA was eluted with Elution Buffer containing 25 or 30% EtOH (20 ng luc Dicer 25% pur. & 20 ng luc Dicer 30% pur.), resulted in efficient suppression of luciferase activity by more than 80%. Equally efficient suppression was achieved with siRNA purified using the two-column purification protocol (20 ng luc Dicer 2 col. pur.) and with the latter siRNA that was subjected to an additional step of EtOH precipitation (20 ng luc d-siRNA (EtOH)). Thus, luciferase specific siRNA purified with either the single-column or two-column purification protocol is highly potent in suppressing the activity of luciferase.

All purified and β-galactosidase-specific siRNA samples generated with the Dicer enzyme failed, as expected, to significantly change expression levels of the luciferase gene as determined by assessing luciferase activity. Variations in the activity of the luciferase enzyme caused by β-galactosidase-specific siRNA were generally less than 10%. As observed earlier, unpurified Dicer-generated siRNA (20 ng luc Dicer reaction) resulted in cell death and nonspecific lack of luciferase activity. Thus, β-galactosidase-specific siRNA purified with the single-column or two-column protocol does not cause any significant nonspecific induction or suppression of bystander proteins, in this case luciferase.

In experimental groups 4 and 5 the effect of luciferase specific siRNA (luc siRNA) as well as β-galactosidase enzyme specific siRNA (lacZ siRNA) generated with RNaseIII enzyme (Ambion) on luciferase activity was assessed. Unlike unpurified Dicer reactions, which contain significant amounts of undigested or partially digested long dsRNA template, unpurified RNaseIII reactions do not contain significant amounts of undigested or partially digested long dsRNA template as previously shown in Figure 20A. Consequently, transfection with unpurified RNaseIII reaction products (20 ng luc RNaseIII reaction & 20 ng lacZ RNaseIII reaction) does not lead to cell death and concomitant nonspecific reduction of luciferase activity. RNase III digestion products are in the 13 - 15 bp size range, which is well below the size range reported for potent siRNA (~20-23bp). Consequently, purified RNaseIII-generated luciferase specific siRNA (20 ng luc RNaseIII 25% pur., 20 ng luc RNaseIII 30% pur, and 20 ng luc RNaseIII 2 col. pur.) suppressed luciferase activity by only approximately 25% under the conditions used here. This lack of efficient suppression at the siRNA concentrations used may be attributable to a lack of functional siRNA present after digestion with RNaseIII since the siRNA size generated by RNaseIII is less than 20 base pairs (see Figures 20A and 20B). Czaudema et al.(Nucleic Acids Research 2003) reported that synthetic siRNAs shorter than 19 base pairs in size were not effective in suppressing gene expression. Concentrations of up to 200 ng/transfection of RNaseIII-generated siRNA were tested. However, even at these elevated amounts no significant suppression was observed. As shown in experimental group 5, neither unpurified nor purified lacZ siRNA that was generated by digestion of long dsRNA templates with RNaseIII had any effect on luciferase activity.

Functional Testing of siRNA Preparations with GripTite™ MSR Cells

Experimental setup

Two reporter plasmids (see above) expressing luciferase and β-galactosidase, respectively, were co-transfected into GripTite™ 293 MSR cells with siRNA specific for luciferase mRNA (luc) or β-galactosidase mRNA (lacZ) generated by Dicer or RNaseIII using the experimental scheme described in the previous experiment. Luciferase and β-galactosidase activity was determined as described above to assess the effect of specific siRNA preparations on the expression of the two gene transcripts under investigation.

Results and Discussion

Results demonstrate the effect of different luc siRNA and lacZ siRNA preparations generated with Dicer and RNaseIII enzyme on β-galactosidase activity. The results obtained are in good agreement with the previous results. In brief, GripTite 293 MSR cells transfected with the reporter plasmid alone (Reporters Only) exhibited reference levels of β-galactosidase activity. Cells not transfected with the reporter plasmid (Mock) did not yield any β-galactosidase activity. As seen previously, crude Dicer reactions (20 ng luc Dicer reaction & 20 ng lacZ Dicer reaction) caused cell death and nonspecific suppression of β-galactosidase activity, while this effect was not observed with crude RNaseIII reactions (20 ng luc RNaseIII reaction & 20 ng lacZ RNaseIII reaction). All preparations of purified, Dicer-generated lacZ siRNA efficiently suppressed expression of β-galactosidase activity by more than 80%. On the other hand, neither preparation of the negative control luc siRNA affected β-galactosidase activity to any significant degree. SiRNA generated by digestion with RNaseIII elicited similar responses to those observed above. Suppression of β-galactosidase activity by lacZ siRNA preparations was inefficient with maximum suppressions of 40%. However, from the results it is apparent that luciferase specific siRNA preparations generated with RNaseIII enzyme caused significant nonspecific induction of the β-galactosidase gene.

Results also demonstrate the effect of different luc siRNA and lacZ siRNA preparations generated with Dicer and RNaseIII enzyme on luciferase activity. The results obtained are in good agreement with the previous results. In brief, GripTite 293 MSR cells transfected with the reporter plasmid alone (Reporters Only) exhibited reference levels of luciferase activity. Cells not transfected with the reporter plasmid (Mock) did not yield any luciferase activity. Crude Dicer reactions (20 ng luc Dicer reaction & 20 ng lacZ Dicer reaction) caused cell death and nonspecific suppression of luciferase activity, while this effect was not observed with crude RNaseIII reactions (20 ng luc RNaseIII reaction & 20 ng lacZ RNaseIII reaction). All preparations of purified, Dicer-generated luc siRNA efficiently suppressed expression of luciferase activity by more than 90%. On the other hand, neither preparation of the negative control lacZ siRNA suppressed luciferase activity. However, in the series of experiments shown here, luciferase activity was stimulated by up to 40% by β-galactosidase specific lacZ siRNA. SiRNA generated by digestion with RNaseIII elicited similar responses to those observed above. The suppression of luciferase activity by luc siRNA preparations was inefficient with maximum suppressions of approximately 20%. The effects of lacZ siRNA preparations generated with RNaseIII on luciferase activity were inconsistent with both induction (20 ng lacZ RNaseIII 25% pur.) and suppression (20 ng lacZ RNaseIII 30% pur. & 20 ng lacZ RNaseIII 2 col. pur.) being observed.

Conclusion

SiRNA generated by digestion of long dsRNA templates with Dicer enzyme and purified using either the single-column or two-column purification protocol efficiently suppressed gene specific expression with minimal nonspecific induction of bystander proteins. Short interfering RNA generated by digestion of long dsRNA templates with RNaseIII enzyme, while efficiently purified with either the single-column or two-column purification protocol, did not perform well under the experimental conditions used here.

Column Capacity and Recovery Determination

Experimental setup.

These experiments were intended to determine the recovery of RNA, tRNA and a 1-kb dsRNA fragment, after binding to the glass fiber matrix of the spin column as a function of elution volume. The experiments were also designed to provide information about the general loading capacity of the spin column for short double-stranded nucleic acids and long dsRNA fragments, the latter are used as templates for RNase digestion assays. In order to assess the column capacity for siRNA, yeast tRNA was used, because it was available in the quantities needed. Yeast tRNA constitutes a sensible alternative for column testing to siRNA as its linear, single-stranded size is approximately 75 nucleotides that are involved in extensive secondary structure formation, i.e. tRNA is present predominantly in dsRNA form. The tRNA used here migrates like a 40-bp double-stranded nucleic acid fragment on agarose gels. The efficiency of recovery and approximate loading capacity was also determined for a 1-kb dsRNA fragment. These long dsRNA fragments serve as templates for Dicer and RNaseIII digestion and require purification after clean up of the transcription reaction with DNaseI and RNaseA and prior to Dicer/RNaseIII digestion for generating siRNA. Purifications were carried out using the single-column purification protocol with 10 - 1000 μg of yeast tRNA or 4 - 240 μg of the 1-kb dsRNA fragment. Bound dsRNA was eluted from the spin columns with either a single elution of 100 μl DEPC-treated water or two successive elutions of 50 μl DEPC-treated water. Amounts of eluted RNA were quantified by A260 measurements and compared to the initial amount of RNA loaded.

Results and Discussion

Ten μg of tRNA were eluted with either a single 100-μl elution or two 50-μl elutions with an efficiency exceeding 90% (Figure 23A). Amounts of tRNA of up to 1 mg can be eluted with efficiencies of approximately 80%, independent of whether a single 100-μl elution or two 50-μl elutions were used. Recovery can be further increased to about 95% with a second 100-μl elution or a third 50-μl elution. It shall be noted that for yeast tRNA amounts in excess of about 100 μg the addition of Binding Buffer and EtOH to the sample results in precipitation of presumably tRNA. The results shown in Figure 23A demonstrate that tRNA, and by correlation siRNA, can be recovered almost quantitatively from the spin column matrix by elution with DEPC-treated water. Also, the results show that the column capacity exceeds 1 mg for tRNA/siRNA.

Figure 23B shows the recovery results obtained with a 1-kb dsRNA fragment loaded at amounts ranging from 4 - 240 μg. Independent of whether a single 100-μl elution or two 50-μl elutions were used recovery efficiency was about 90%. It shall be noted that the long dsRNA fragment was more susceptible to form a precipitate after the addition of Binding Buffer and EtOH than tRNA. Loading of dsRNA amounts exceeding about 500 μg resulted in progressively decreasing recoveries with either a single 100-μl elution or two 50-μl elutions, which could be improved with additional elutions.

Conclusion

Short dsRNA, *e.g.*, siRNA or tRNA, as well as long dsRNA fragments can be efficiently eluted after binding to the spin column with DEPC-treated water. No major differences in recovery were observed for either a single elution with 100 μl or two successive 50-μl elutions.

References

Kaufman, R.J., Proc Natl. Acad. Sci. USA 96:11693-11695 (1999).
Denli, A.M. and Hannon, G.J., Trends Biochem. Sci. 28:196-201 (2003).
Carrington ,J.C. and Ambros, V., Science. 301:336-338 (2003).
Sledz, C.A, et al., Nat Cell Biol. 5:834-839 (2003).
Illangasekare, M. and Yarus, M., RNA. 5:1482-1489 (1999).
Elbashir, S.M., et al., Nature 411:494-498 (2001).
Czaudema, F., et al., Nucleic Acids Res. 31:2705-2716 (2003).
Elbashir, S.M., et al., EMBO J. 20:6877-6888 (2001).

It is that various modifications are possible within the scope of the invention claimed. Thus, it should be understood that although the present invention has been specifically disclosed herein, optional features, modification and variation of the concepts herein disclosed may be resorted to by those skilled in the art, and that such modifications and variations are considered to be within the scope of this invention as defined by the appended claims.
Other aspects of the invention are within the following claims.

**Claims**

**1.** A method for purifying short RNA molecules from about 18 to about 50 nucleotides or base pairs in length, the method comprising:

(a) adding a first fluid mixture or, in any order, fluids or combinations of fluids that contain the components of said first fluid mixture, to a sample comprising said short RNA molecules and other nucleic acids having a size other than said short RNA molecules to produce a first binding mixture,

wherein the first fluid mixture or fluids or combinations of fluids that contain the components of the fluid mixture, comprises guanidine isothiocyanate, and
wherein the first binding mixture comprises m% (v/v) ethanol or isopropanol wherein m is any whole integer from 30 to 45;

(b) filtering said first binding mixture through a first silica based column, wherein said short RNA molecules pass through a composition within the first column, and said other nucleic acids are retained, to produce a first flow-through solution;

(c) adding a second fluid mixture or, in any order, fluids or combinations of fluids that contain the components of said second fluid mixture to said first flow-through solution, to produce a second binding mixture,

wherein the second binding mixture comprises m% (v/v) ethanol or isopropanol wherein m is any whole integer from 65 to 75;

(d) filtering the second binding mixture through a second silica based column, wherein said short RNA molecules bind to a composition within the second column and, optionally, washing the bound nucleic acids; and

(e) eluting the said short RNA molecules with a third fluid mixture, to produce an eluate, wherein said short RNA molecules are present in said eluate.

**2.** The method of claim 1, wherein said RNA molecules are double-stranded.

**3.** The method of any one of claims 1 to 2, wherein said RNA molecules are micro-RNA molecules.

**4.** The method of any one of claims 1 to 2, wherein said RNA molecules are RNAi molecules.

**5.** The method of claim 4, wherein said RNAi molecules are selected from the group consisting of shRNA, stRNA, siRNA, e-siRNA, and d-siRNA.

**6.** The method of any one of claims 1 to 5, wherein the size of said short RNA molecules ranges from about 20 to about 30 nucleotides or base pairs in length, and preferably from about 20 to 25 nucleotides or base pairs in length.

**7.** The method of any one of the preceding claims, wherein said other nucleic acids are RNA molecules.

**8.** The method of claim 7, wherein said short RNA molecules and said other RNA molecules are double-stranded molecules.

**9.** The method of any one of the preceding claims, wherein said sample is from a biological system.

**10.** The method of any one of claims 1 to 9, wherein the first and second columns are glass fibre columns.

## Patentansprüche

**1.** Verfahren zum Reinigen kurzer RNA-Moleküle in einer Länge von etwa 18 bis 50 Nucleotiden oder Basenpaaren, welches Verfahren umfasst:

(a) Hinzufügen einer ersten Fluidmischung oder, in beliebiger Reihenfolge, Fluids oder Kombinationen von Fluids, welche die Komponenten der genannten ersten Fluidmischung enthalten, zu einer Probe, umfassend die genannten kurzen RNA-Moleküle und andere Nucleinsäuren, die eine andere Größe als die kurzen RNA-Moleküle haben, um eine erste bindende Mischung zu erzeugen,
wobei die erste Fluidmischung oder Fluids oder Kombinationen von Fluids, welche die Komponenten der ersten Fluidmischung enthalten, Guanidinisothiocyanat aufweisen, und
wobei die erste bindende Mischung m% (v/v) Ethanol oder Isopropanol umfasst, wobei m eine beliebige ganze Zahl von 30 bis 45 ist;

(b) Filtern der ersten bindenden Mischung durch eine erste, auf Silica basierende Säule, wobei die genannten kurzen RNA-Moleküle eine Zusammensetzung innerhalb der ersten Säule passieren und die genannten anderen Nucleinsäuren zurückgehalten werden, um eine erste Durchflusslösung zu erzeugen;

(c) Hinzufügen einer zweiten Fluidmischung oder, in einer beliebigen Reihenfolge, von Fluids oder Kombinationen von Fluids, welche die Komponenten der genannten zweiten Fluidmischung enthalten, zur ersten Durchflusslösung, um eine zweite bindende Mischung zu erzeugen,
wobei die zweite bindende Mischung m% (v/v) Ethanol oder Isopropanol umfasst, wobei m eine beliebige ganze Zahl von 65 bis 75 ist;

(d) Filtern der zweiten bindenden Mischung durch eine zweite, auf Silica basierende Säule, wobei sich die kurzen RNA-Moleküle an eine Zusammensetzung innerhalb der zweiten Säule binden, und wahlweise Waschen der gebundenen Nucleinsäuren;
und
(e) Eluieren der kurzen RNA-Moleküle mit einer dritten Fluidmischung, um ein Eluat zu erzeugen, wobei die kurzen RNA-Moleküle im diesem Eluat vorliegen.

**2.** Verfahren nach Anspruch 1, wobei die RNA-Moleküle doppelsträngig sind.

**3.** Verfahren nach einem der Ansprüche 1 bis 2, wobei die RNA-Moleküle Mikro-RNA-Moleküle sind.

**4.** Verfahren nach einem der Ansprüche 1 bis 2, wobei die RNA-Moleküle RNAi-Moleküle sind.

**5.** Verfahren nach Anspruch 4, wobei die RNAi-Moleküle ausgewählt sind aus der Gruppe, bestehend aus shRNA, stRNA, siRNA, e-siRNA und d-siRNA.

**6.** Verfahren nach einem der Ansprüche 1 bis 5, wobei die Größe der kurzen RNA-Moleküle im Bereich einer Länge von etwa 20 bis etwa 30 Nucleotiden oder Basenpaaren liegt und bevorzugt einer Länge von etwa 20 bis 25 Nucleotiden oder Basenpaaren liegt.

**7.** Verfahren nach einem der vorgenannten Ansprüche, wobei die genannten anderen Nucleinsäuren RNA-Moleküle sind.

**8.** Verfahren nach Anspruch 7, wobei die genannten kurzen RNA-Moleküle und die genannten anderen RNA-Moleküle doppelsträngige Moleküle sind.

**9.** Verfahren nach einem der vorgenannten Ansprüche, wobei die genannte Probe aus einem biologischen System stammt.

**10.** Verfahren nach einem der Ansprüche 1 bis 9, wobei die ersten und zweiten Säulen Glasfasersäulen sind.

**Revendications**

**1.** Procédé de purification de molécules courtes d'ARN de longueur comprise entre environ 18 et environ 50 paires de base, le procédé comprenant :

(a) l'ajout d'un premier mélange de fluide ou dans un ordre quelconque, de fluides ou de combinaisons de fluides qui contiennent les composants dudit premier mélange de fluides, dans un échantillon contenant lesdites molécules courtes d'ARN et d'autres acides nucléiques qui ont une taille autre que lesdites molécules courtes d'ARN pour produire un premier mélange de liaison,
le premier mélange de liaison ou les fluides ou les combinaisons de fluides qui contiennent les composants du mélange de fluide comprenant de l'isothiocyanate de guanidine, et
le premier mélange de liaison comprenant m % (en vol.) d'éthanol ou d'isopropanol, m étant un nombre entier compris entre 30 et 45 ;
(b) la filtration dudit premier mélange de liaison à travers une première colonne à base de silice, lesdites molécules courtes d'ARN traversant une composition dans la première colonne, et lesdits autres acides nucléiques étant retenus, pour produire une première solution d'écoulement ;
(c) l'ajout d'un deuxième mélange fluide ou dans un ordre quelconque, de fluides ou de combinaisons de fluides qui contiennent les composants dudit deuxième mélange de fluides avec ladite première solution d'écoulement, pour produire un deuxième mélange de liaison,
le deuxième mélange de liaison comprenant m% (en vol.) d'éthanol ou d'isopropanol, m représentant tout entier compris entre 65 et 75 ;
(d) la filtration du deuxième mélange de liaison à travers une deuxième colonne à base de silice, lesdites molécules courtes d'ARN se liant à une composition dans la deuxième colonne et en option, le lavage des acides nucléiques liés ; et
(e) l'élution desdites molécules courtes d'ARN avec un troisième mélange de fluides, pour produire un éluat, lesdites molécules courtes d'ARN étant présentes dans ledit éluat.

2. Procédé selon la revendication 1, dans lequel lesdites molécules d'ARN sont à double brin.

3. Procédé selon l'une quelconque des revendications 1 à 2, dans lequel lesdites molécules d'ARN sont des molécules de micro-ARN.

4. Procédé selon l'une quelconque des revendications 1 à 2, dans lequel lesdites molécules d'ARN sont des molécules d'ARNi.

5. Procédé selon la revendication 4, dans lequel lesdites molécules d'ARN sont choisies dans le groupe constitué d'ARNsh, d'ARNst, d'ARNsi, d'ARNe-si et d'ARNd-si.

6. Procédé selon l'une quelconque des revendications 1 à 5, dans lequel la taille desdites molécules courtes d'ARN est comprise entre environ 20 et environ 30 nucléotides ou paires de base en longueur, et de préférence d'environ 20 à 25 nucléotides ou paires de base en longueur.

7. Procédé selon l'une quelconque des revendications précédentes, dans lequel lesdits autres acides nucléiques sont des molécules d'ARN.

8. Procédé selon la revendication 7, dans lequel lesdites molécules courtes d'ARN et lesdites autres molécules d'ARN sont des molécules à double brin.

9. Procédé selon l'une quelconque des revendications précédentes, dans lequel ledit échantillon provient d'un système biologique.

10. Procédé selon l'une quelconque des revendications 1 à 9, dans lequel les première et deuxième colonnes sont des colonnes de fibre de verre.

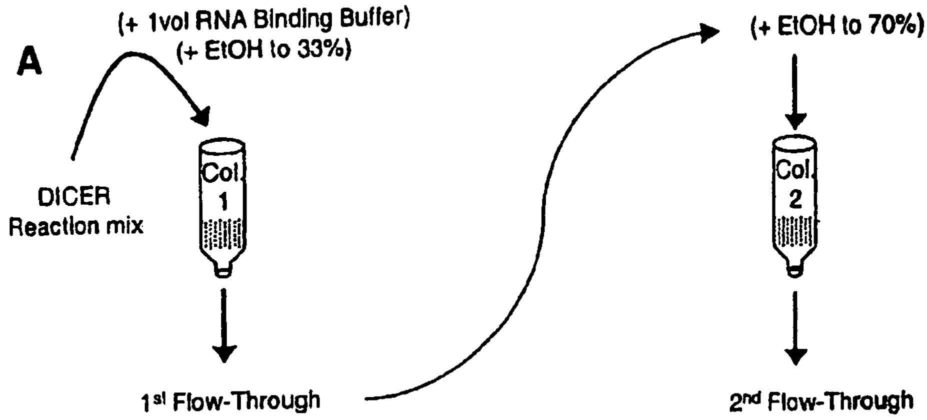
A
(+ 1vol RNA Binding Buffer)
(+ EtOH to 33%)
DICER
Reaction mix
Col
1
1st Flow-Through
(+ EtOH to 70%)
Col
2
2nd Flow-Through

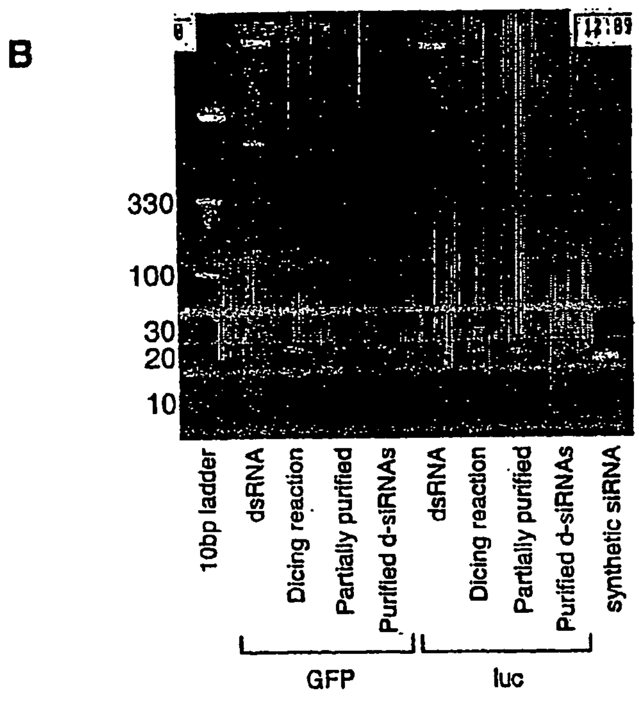
B
330
100
30
20
10
10bp ladder
dsRNA
Dicing reaction
Partially purified
Purified d-siRNAs
dsRNA
Dicing reaction
Partially purified
Purified d-siRNAs
synthetic siRNA
GFP
luc

Figure 3

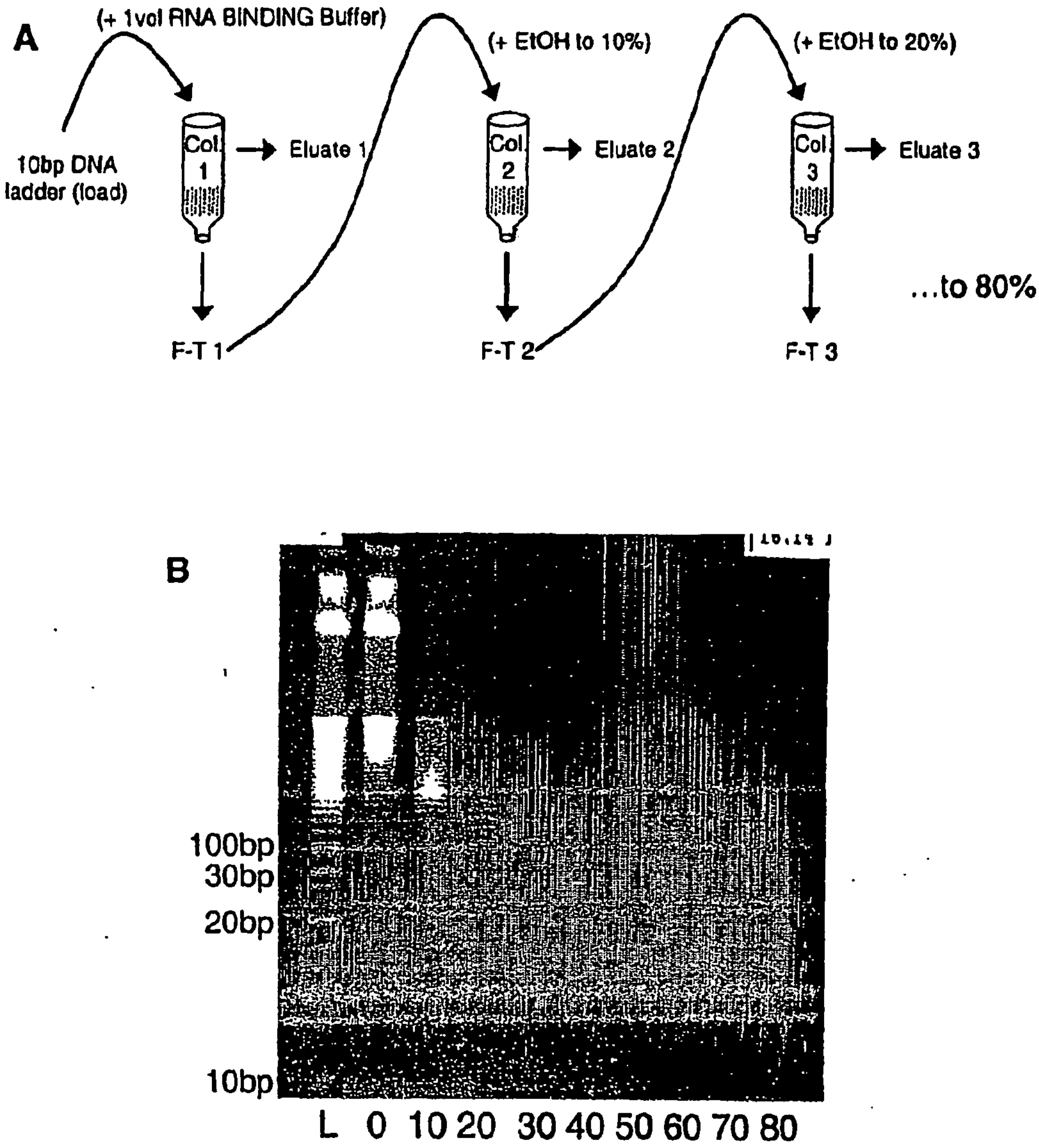
A
(+ 1vol RNA BINDING Buffer)
(+ EtOH to 10%)
(+ EtOH to 20%)
10bp DNA ladder (load)
Col 1
Col 2
Col 3
Eluate 1
Eluate 2
Eluate 3
F-T 1
F-T 2
F-T 3
...to 80%
B
100bp
30bp
20bp
10bp
L 0 10 20 30 40 50 60 70 80

Figure 4

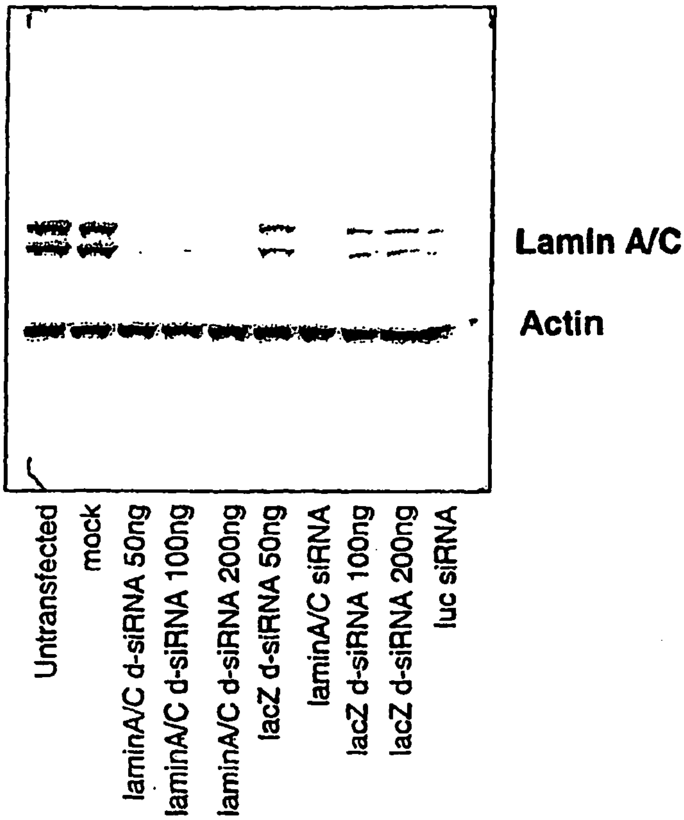
Lamin A/C
Actin
Untransfected
mock
laminA/C d-siRNA 50ng
laminA/C d-siRNA 100ng
laminA/C d-siRNA 200ng
lacZ d-siRNA 50ng
laminA/C siRNA
lacZ d-siRNA 100ng
lacZ d-siRNA 200ng
luc siRNA

Figure 5

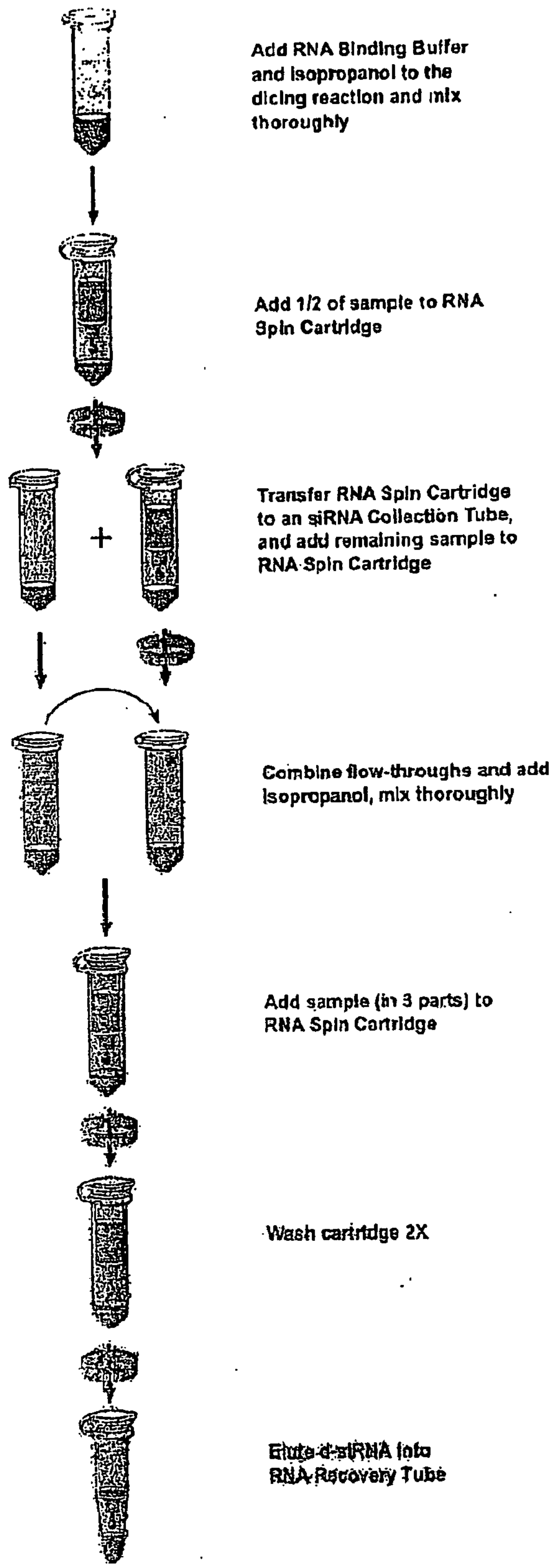
Add RNA Binding Buffer and Isopropanol to the dicing reaction and mix thoroughly
Add 1/2 of sample to RNA Spin Cartridge
Transfer RNA Spin Cartridge to an siRNA Collection Tube, and add remaining sample to RNA Spin Cartridge
Combine flow-throughs and add Isopropanol, mix thoroughly
Add sample (in 3 parts) to RNA Spin Cartridge
Wash cartridge 2X
Elute d-siRNA into RNA Recovery Tube

FIG. 8

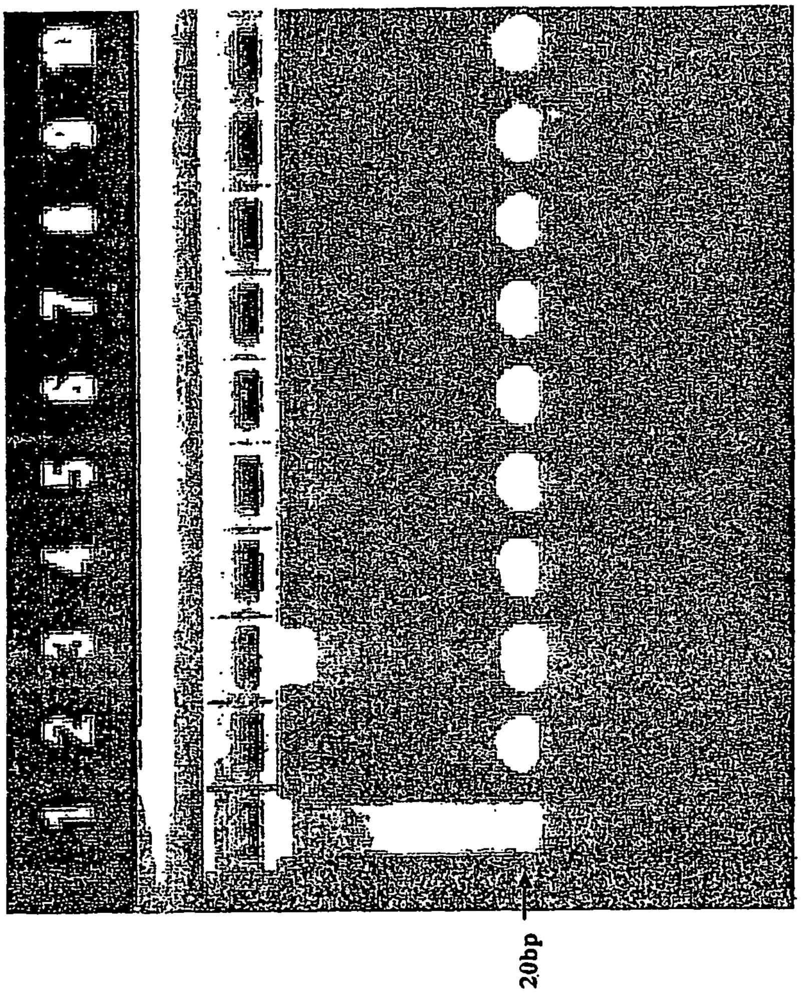


FIG. 19A

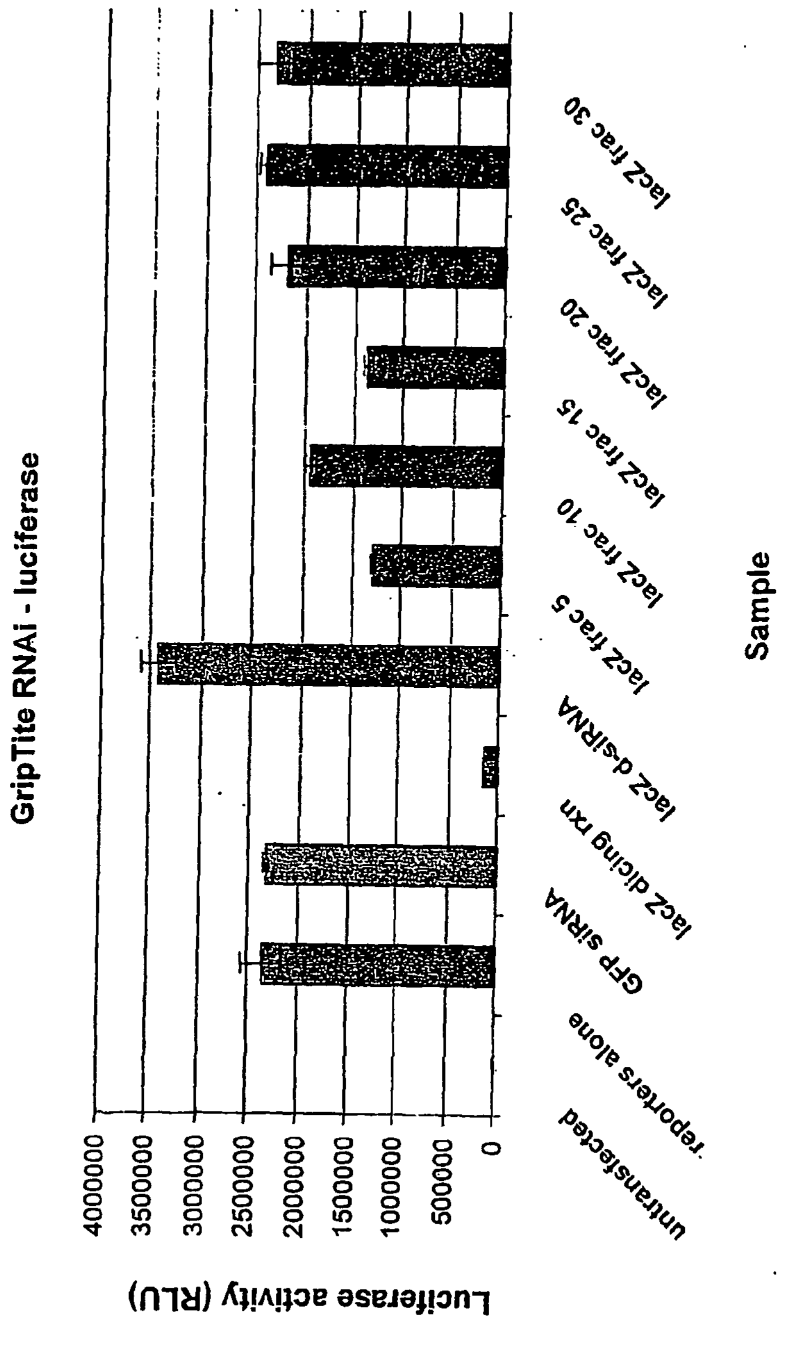
GripTite RNAi - luciferase
Luciferase activity (RLU)
4000000
3500000
3000000
2500000
2000000
1500000
1000000
500000
0
untransfected
reporters alone
GFP siRNA
lacZ dicing rxn
lacZ d-siRNA
lacZ frac 5
lacZ frac 10
lacZ frac 15
lacZ frac 20
lacZ frac 25
lacZ frac 30
Sample

FIG. 19B

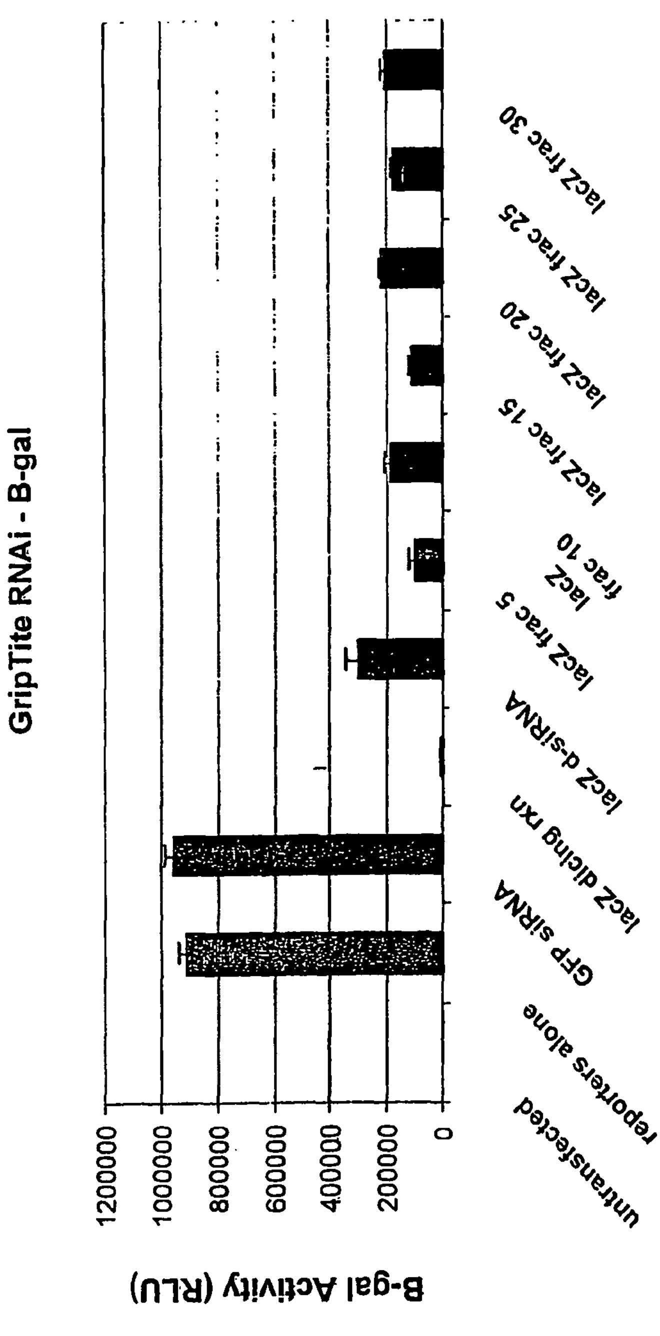
GripTite RNAi - B-gal
B-gal Activity (RLU)
1200000
1000000
800000
600000
400000
200000
0
untransfected
reporters alone
GFP siRNA
lacZ dicing rxn
lacZ d-siRNA
lacZ frac 5
lacZ frac 10
lacZ frac 15
lacZ frac 20
lacZ frac 25
lacZ frac 30
Sample

FIG. 19C

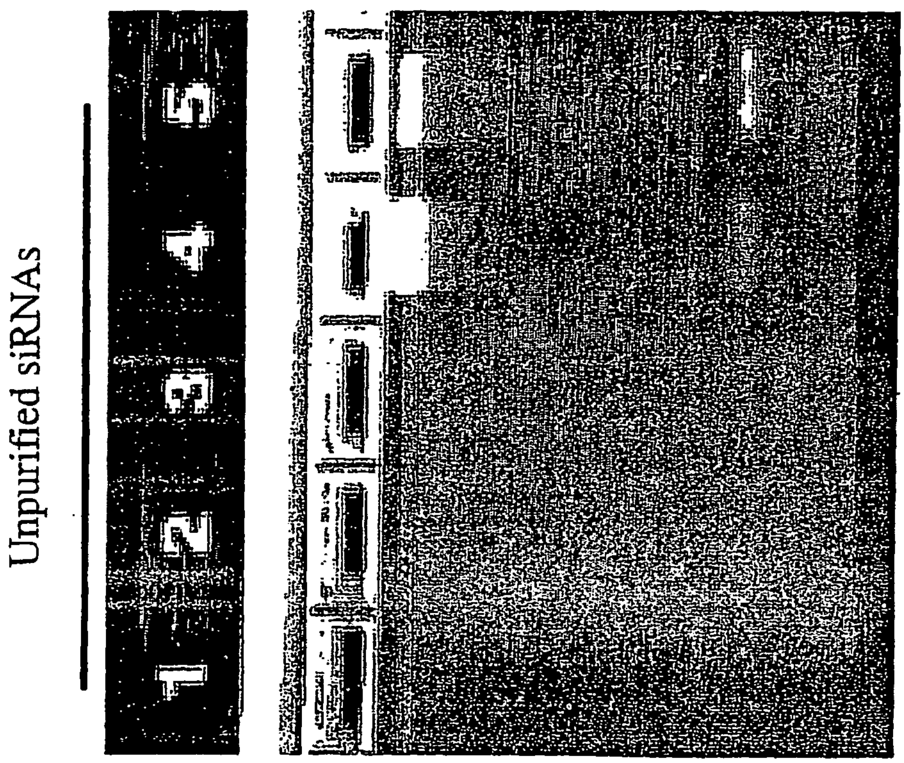


FIG. 20A

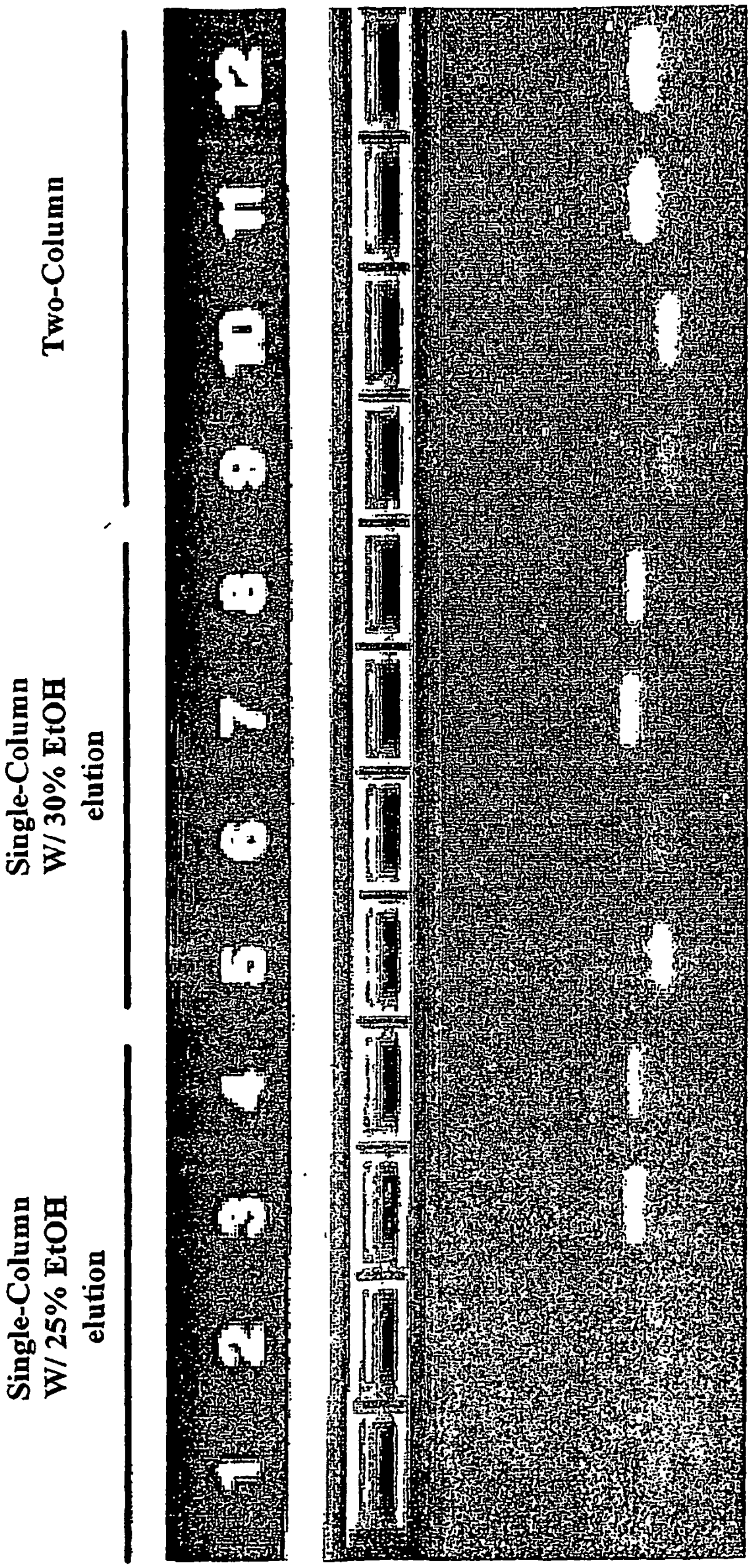
Single-Column
W/ 25% EtOH
elution
Single-Column
W/ 30% EtOH
elution
Two-Column
1
2
3
4
5
6
7
8
9
10
11
12

FIG. 20B

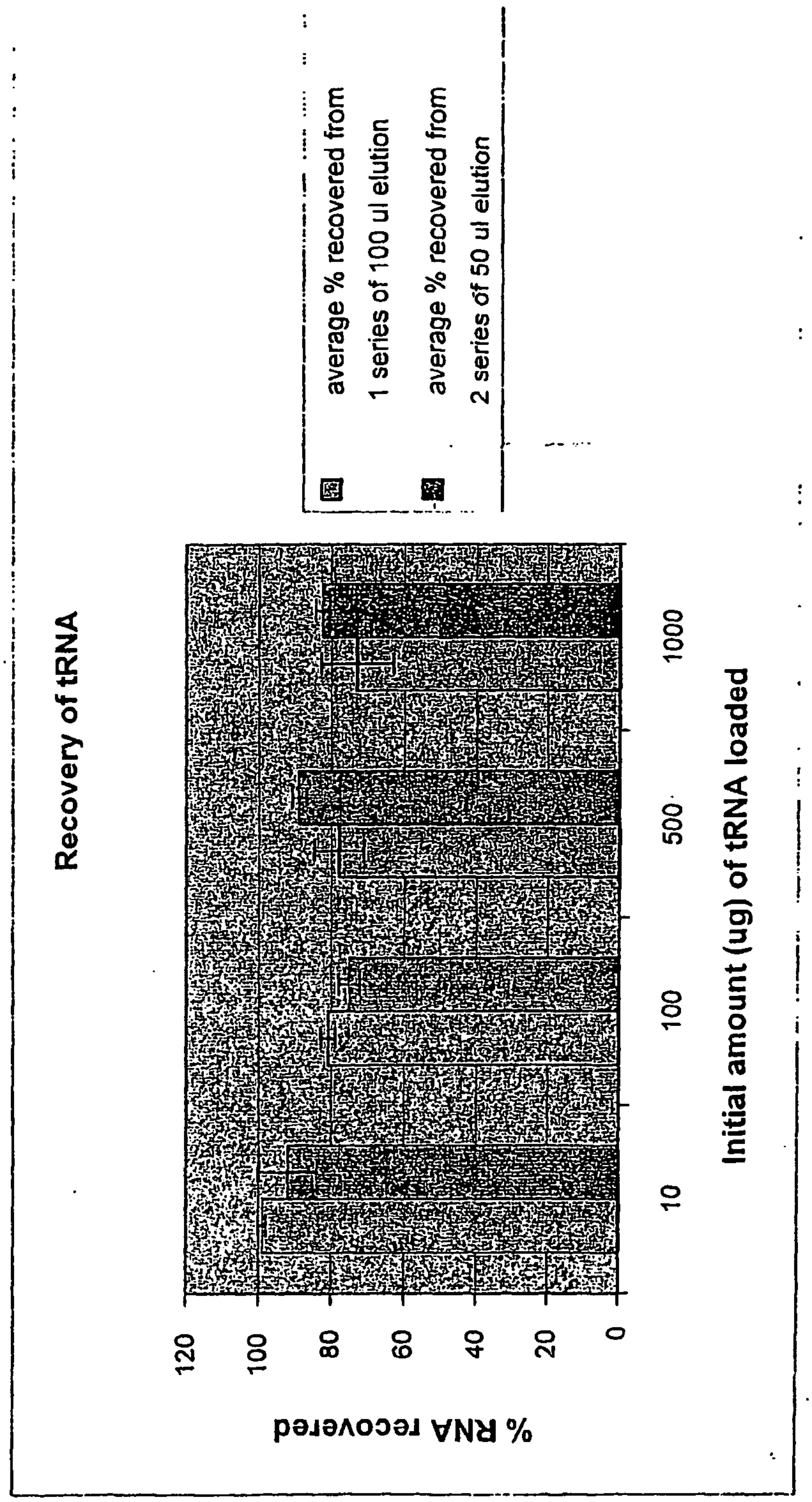
Recovery of tRNA
% RNA recovered
120
100
80
60
40
20
0
10
100
500
1000
Initial amount (ug) of tRNA loaded
average % recovered from 1 series of 100 ul elution
average % recovered from 2 series of 50 ul elution

FIG. 23A

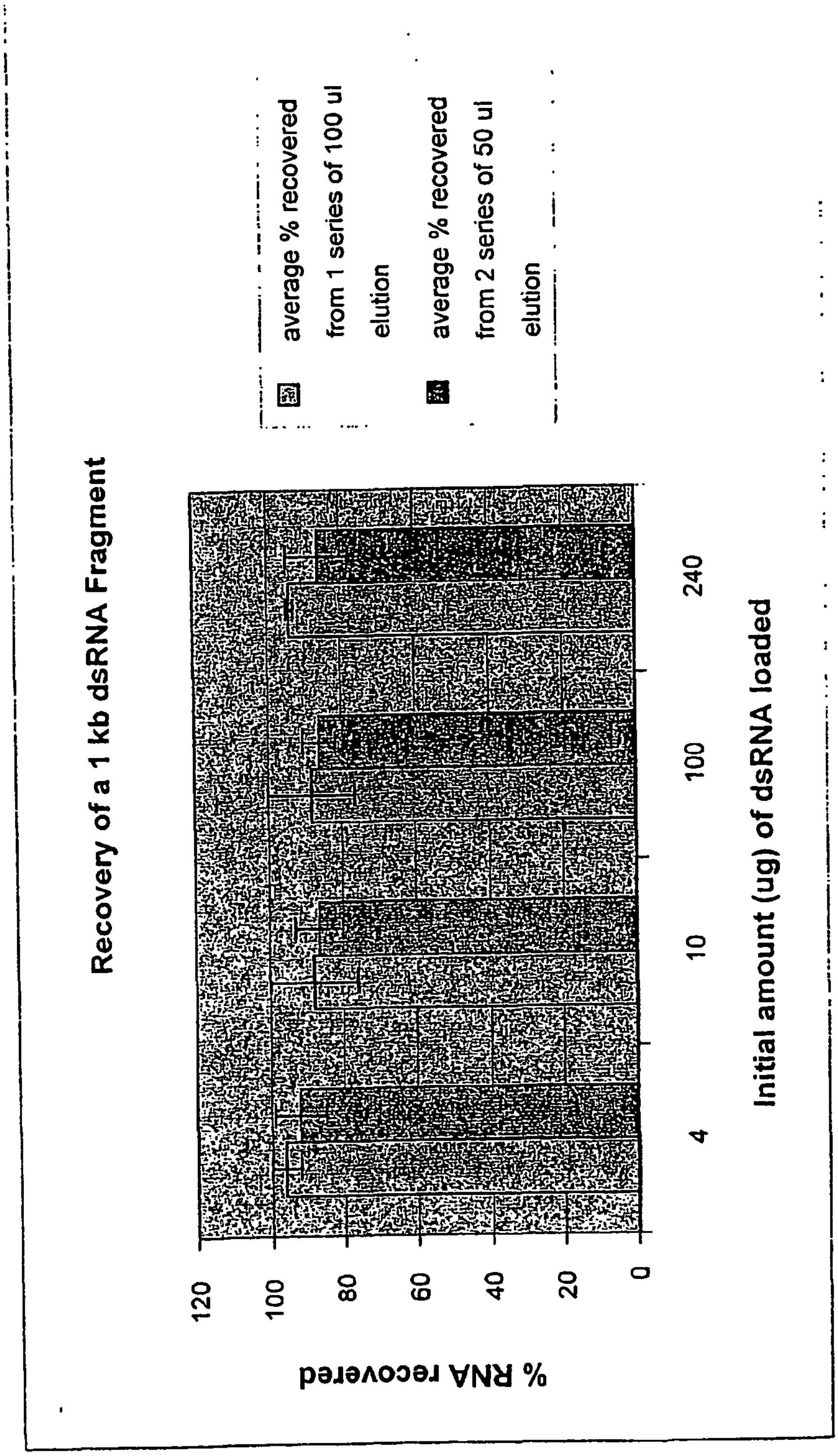
Recovery of a 1 kb dsRNA Fragment
% RNA recovered
120
100
80
60
40
20
0
4
10
100
240
Initial amount (ug) of dsRNA loaded
average % recovered from 1 series of 100 ul elution
average % recovered from 2 series of 50 ul elution

FIG. 23B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6180778 B1
- US 5789573 A
- US 6197584 B, Baker
- WO 9623569 A
- US 4987071 A
- US 5877021 A
- WO 02061034 A
- US 25451000 P
- US 32609201 P
- US 01412801 A
- US 52094603 P
- WO 9119813 A
- US 5270163 A
- US 4517338 A
- US 4458066 A
- US 20020146826 A
- US 5674908 A
- US 5834439 A
- US 6110916 A
- EP 0394111 A
- US 5994317 A
- US 5459127 A
- US 5264618 A, Felgner
- US 4897355 A
- US 5208066 A
- US 5550289 A
- US 5334761 A
- US 5736392 A
- US 6051429 A
- US 5861397 A
- US 6022874 A
- WO 9319768 A
- WO 9502397 A
- WO 04779463 A
- WO 60491758 A
- WO 60520383 A


### Non-patent literature cited in the description


- **SAMBROOK et al.** Molecular Cloning: A Laboratory Manual. Cold Spring Harbor Laboratory Press, 2001
- Labeling and Purification of RNA Synthesized by In Vitro Transcription. **CLARKE.** RNA-Protein Interaction Protocols. Humana Press, 1999
- **PUTTARAJU et al.** *Nucleic Acids Symp Ser.,* 1995, vol. 33, 49-51
- **MCLAUGHLIN et al.** *J Chromatogr,* 1987, vol. 418, 51-72
- **MANDELL et al.** *Anal Biochem,* 1960, vol. 1, 66
- **LOESER et al.** *Biochemistry,* 1970, vol. 9, 2364-6
- **MODAK et al.** *Anal Biochem.,* 1970, vol. 34, 284-6
- **FIRE et al.** *Nature,* 1998, vol. 391, 806-811
- **DER et al.** *Proc. Natl. Acad. Sci. USA,* 1997, vol. 94, 3279-3283
- **COLBY et al.** *Annu. Rev. Microbiol.,* 1971, vol. 25, 333
- **KLEINSCHMIDT ET AL.** *Annu. Rev. Biochem.,* 1972, vol. 41, 517
- **LAMPSON et al.** *Proc. Natl. Acad. Sci. USA,* 1967
- **LOMNICZI et al.** *J. Gen. Virol.,* 1970, vol. 8, 55
- **YOUNGER et al.** *J. Bacteriol.,* 1966, vol. 92, 862
- **GEHRIG et al.** *Plant Molecular Biology Reporter,* 2000, vol. 18, 369-376
- **JOHANSEN et al.** *Plant Physiol.,* 2001, vol. 2, 110-117
- **MEYERS et al.** *Nature Biotech.,* 2003, vol. 21, 324-328
- **CAPLEN et al.** *Proc. Natl. Acad. Sci. USA,* 2001, vol. 98, 9742-9747
- **ELBASHIR et al.** *Nature,* 2001, vol. 411, 494-498
- **YANG et al.** *Proc Natl Acad Sci USA,* 2002, vol. 99, 9942-7
- **CALEGARI et al.** *Proc Natl Acad Sci USA,* 2002, vol. 99, 14236-40
- **PADDISON et al.** *Proc. Natl. Acad. Sci. USA,* 2002, vol. 99, 1443-8
- **LAMONTAGNE et al.** *Curr Issues Mol Biol.,* 2001, vol. 3, 71-78
- **CONRAD et al.** *Int JBiochem Cell Biol.,* 2002, vol. 34, 116-29
- **SRIVASTAVA et al.** *Indian JBiochem Biophys.,* 1996, vol. 33, 253-60
- **PROVOST et al.** *Proc Natl Acad Sci USA,* 2002, vol. 99, 16648-53
- **PARK et al.** *Curr Biol,* 2002, vol. 12, 1484-95

- **GOLDEN et al.** *Plant Physiol.,* 2002, vol. 130, 808-22
- **SCHAUER et al.** *Trends Plant Sci.,* 2002, vol. 7, 487-91
- **KETTING et al.** *Genes Dev.,* 2001, vol. 15, 2654-9
- **NICHOLSON et al.** *Mamm Genome,* 2002, vol. 13, 67-73
- **BERNSTEIN et al.** *Nature,* 2001, vol. 409, 363-6
- **MATSUDA et al.** *Biochim Biophys Acta,* 2000, vol. 1490, 163-9
- **PROVOST et al.** *EMBO J.,* 2002, vol. 21, 5864-74
- **MYERS et al.** *Nat Biotechnol.,* 2003, vol. 21, 324-8
- **KAWASAKI et al.** *Nucleic Acids Res.,* 2003, vol. 31, 981-7
- **YANG et al.** *Proc Natl Acad Sci USA,* 2002, vol. 99, 9942-9947
- **FORTIN et al.** *BMC Genomics,* 2002, vol. 3, 26
- **RAUHUT et al.** *Nucleic Acids Res.,* 1996, vol. 24, 1246-1251
- **STORZ.** *Science,* 2002, vol. 296, 1260-3
- **ILLANGASEKARE et al.** *RNA,* 1999, vol. 5, 1482-1489
- **WASSARMAN et al.** *Trends Microbiol.,* 1999, vol. 7, 37-45
- **ILLANGASKARE et al.** *RNA,* 1999, vol. 5, 1482-1489
- **MUTO et al.** *Trends Biochem Sci.,* 1998, vol. 23, 25-29
- **GILLET et al.** *Mol Microbiol.,* 2001, vol. 42, 879-885
- **SAMBROOK et al.** Molecular Cloning: A Laboratory Manual. Cold Spring Harbor Laboratory Press, 1989
- **ELLINGTON et al.** Current Protocols in Molecular Biology. Wiley Interscience, 1992
- **TUSCHL.** *Chembiochem.,* 2001, vol. 2, 239-245
- **CULLEN.** *Nat Immunol.,* 2002, vol. 3, 597-599
- **PARRISH et al.** *Mol Cell,* 2000, vol. 6, 1077-1087
- **TABARA et al.** *Cell,* 1999, vol. 99, 123-132
- **NAPOLI et al.** *Plant Cell,* 1990, vol. 2, 279-289
- **HAMILTON et al.** *Science,* 1999, vol. 286, 950-952
- **HAMILTON et al.** *EMBO J,* 2002, vol. 21, 4671-4679
- **ROMANO et al.** *Mol Microbiol,* 1992, vol. 6, 3343-3353
- **CAPLEN et al.** *Proc Natl Acad Sci USA,* 2001, vol. 98, 9742-9747
- **BERNSTEIN et al.** *Nature,* 2001, vol. 409, 363-366
- **BOUTLA et al.** *Curr Biol,* 2001, vol. 11, 1776-1780
- **ELBASHIR et al.** *Methods,* 2002, vol. 26, 199-213
- **HARBORTH et al.** *J Cell Sci,* 2001, vol. 114, 4557-4565
- **KRICHEVSKY et al.** *Proc Natl Acad Sci USA,* 2002, vol. 99, 11926-11929
- **GITLIN et al.** *Nature,* 2002, vol. 418, 379-380
- **CAPODICI et al.** *J Immunol,* 2002, vol. 169, 5196-5201
- **SCHERR et al.** *Blood*
- **CALEGARI et al.** *Proc Natl Acad Sci USA,* 2002, vol. 99, 14236-14240
- **ZHOU et al.** *Nucleic Acids Res,* 2002, vol. 30, 1664-1669
- **LEWIS et al.** *Nat Genet,* 2002, vol. 32, 107-108
- **MCCAFFREY et al.** *Nature,* 2002, vol. 418, 38-39
- **BERTRAND et al.** *Biochem Biophys Res Commun,* 2002, vol. 296, 1000-1004
- **LASSUS et al.** *Sci STKE 2002,* 2002, vol. 147
- **LEIRDAL et al.** *Biochem Biophys Res Commun,* 2002, vol. 295, 744-748
- **HOHJOH.** *FEBS Lett,* 2002, vol. 521, 195-199
- **DONZEET et al.** *Nucleic Acids Res,* 2002, vol. 30, e46
- **YU et al.** *Proc Natl Acad Sci USA,* 2002, vol. 99, 6047-6052
- **NAGASE et al.** *DNA Res.,* 1999, vol. 6, 63-70
- **NICHOLSON et al.** *Mamm. Genome,* 2002, vol. 13, 67-73
- **PADDISON et al.** *Genes & Dev.,* 2002, vol. 16, 948-958
- **BANERJEE et al.** *Bioessays,* 2002, vol. 24, 119-129
- **BLACKWELL et al.** *Science,* 1990, vol. 250, 1104-1110
- **BLACKWELL et al.** *Science,* 1990, vol. 250, 1149-1152
- **TUERK et al.** *Science,* 1990, vol. 249, 505-510
- **JOYCE.** *Gene,* 1989, vol. 82, 83-87
- **TUERK ; GOLD.** *Science,* 1990, vol. 249, 505-510
- **KINZLER et al.** *Nucleic Acids Res.,* 1989, vol. 17, 3645-3653
- **ELLINGTON et al.** *Nature,* 1990, vol. 346, 818-822
- **ECKER et al.** *Nuc. Acids Res.,* 1993, vol. 21, 1853
- **TUERK et al.** *Science,* 1990, vol. 249, 505
- **PADMANABHAN et al.** *J. Biol. Chem.,* 1993, vol. 24, 17651
- **WANG et al.** *Biochemistry,* 1993, vol. 32, 1899

- **MACAYA et al.** *Proc. Nat'l. Acad. Sci. USA,* 1993, vol. 90, 3745
- **LYER et al.** Modified oligonucleotides--synthesis, properties and applications. *Curr Opin Mol Ther,* 1999, vol. 1, 344-358
- **VERMA et al.** Modified oligonucleotides: synthesis and strategy for users. *Annu Rev Biochem.,* 1998, vol. 67, 99-134
- **PFLEIDERER et al.** Recent progress in oligonucleotide synthesis. *Acta Biochim Pol.,* 1996, vol. 43, 37-44
- **WARREN et al.** Principles and methods for the analysis and purification of synthetic deoxyribonucleotides by high-performance liquid chromatography. *Mol Biotechnol.,* 1995, vol. 4, 179-199
- **SPROAT.** Chemistry and applications of oligonucleotide analogues. *JBiotechnol.,* 1995, vol. 41, 221-238
- **DE MESMAEKER et al.** Backbone modifications in oligonucleotides and peptide nucleic acid systems. *Curr Opin Struct Biol.,* 1995, vol. 5, 343-355
- **CHARUBALA et al.** Chemical synthesis of 2',5'-oligoadenylate analogues. *Prog Mol Subcell Biol.,* 1994, vol. 14, 114-138
- **SONVEAUX.** Protecting groups in oligonucleotide synthesis. *Methods Mol Biol.,* 1994, vol. 26, 1-71
- **GOODCHILD.** Conjugates of oligonucleotides and modified oligonucleotides: a review of their synthesis and properties. *Bioconjug Chem.,* 1990, vol. 1, 165-187
- **THUONG et al.** Chemical synthesis of natural and modified oligodeoxynucleotides. *Biochimie,* 1985, vol. 67, 673-684
- **ITAKURA et al.** Synthesis and use of synthetic oligonucleotides. *Annu Rev Biochem.,* 1984, vol. 53, 323-356
- **CARUTHERS et al.** Deoxyoligonucleotide synthesis via the phosphoramidite method. *Gene Amplif Anal.,* 1983, vol. 3, 1-26
- **OHTSUKA et al.** Recent developments in the chemical synthesis of polynucleotides. *Nucleic Acids Res.,* 1982, vol. 10, 6553-6560
- **KOSSEL.** Recent advances in polynucleotide synthesis. *Fortschr Chem Org Naturst,* 1975, vol. 32, 297-508
- **LIM et al.** *Science,* 2003, vol. 299, 1540
- **MELI et al.** *Int Microbiol.,* 2001, vol. 4, 5-11
- **PECULIS et al.** *Curr. Opin. Cell Biol.,* 1996, vol. 6, 1413-1415
- **GERBI.** *Biochem. Cell. Biol.,* 1995, vol. 73, 845-858
- **MAXWELL et al.** *Annu. Rev. Biochem.,* 1995, vol. 35, 897-934
- **BALAKIN et al.** *Cell,* 1996, vol. 86, 823-834
- **GANOT et al.** *Genes Dev.,* 1997, vol. 11, 941-956
- **TOLLERVEY et al.** *Curr. Opin. Cell Biol.,* 1997, vol. 9, 337-342
- **SAMARSKY et al.** *EMBO J.,* 1998, vol. 17, 3747-3757
- **WILLIAMS.** *Nucleic Acids Res,* 1999, vol. 27, 165-166
- **WILLIAMS et al.** *Nucleic Acids Res,* 1998, vol. 26, 163-165
- **BRAASCH et al.** *Biochemistry,* 2003, vol. 42, 7967-75
- **ZHANG et al.** *EMBO J,* 2002, vol. 21, 5875-5885
- **PROVOST et al.** *EMBO J,* 2002, vol. 21, 5864-5874
- **TUSCHL et al.** *Genes Dev.,* 1999, vol. 13, 3191-3197
- **ZAMORE et al.** *Cell,* 2000, vol. 101, 25-33
- **LI et al.** *Nucleic Acids Res,* 1993, vol. 21, 1919-1925
- **FRANCH et al.** *J Biol Chem,* 1999, vol. 274, 26572-26578
- **BELLOFATTO et al.** *J Biol Chem,* 1983, vol. 258, 5467-5476
- **MARCH et al.** *Nucleic Acids Res,* 1990, vol. 18, 3293-3298
- **SAMBROOK, J. et al.** Molecular Cloning: A Laboratory Manual. Cold Spring Harbor Laboratory Press, 1989
- **WALZEM et al.** *Poult Sci.,* 1997, vol. 76, 882-6
- **GAO et al.** *Biochim. Biophys. Res. Comm.,* 1991, vol. 179, 280-285
- **KIKUCHI et al.** *Hum Gene Ther,* 1999, vol. 10, 947-55
- **BEHR et al.** *Proc. Natl. Acad. Sci. USA,* 1989, vol. 86, 6982-6986
- **MAI et al.** *J Biol Chem.,* 2002, vol. 277, 30208-30218
- **ROSENZWEIG et al.** *Bioconjug Chem,* 2001, vol. 12, 258-63
- **FELGNER et al.** *Ann N Y Acad Sci,* 1995, vol. 772, 126-39
- **KONOPKA et al.** *Biochim Biophys Acta,* 1996, vol. 1312, 186-96
- **SAN et al.** *Hum Gene Ther,* 1993, vol. 4, 781-8
- **BACCAGLINI et al.** *J Gene Med,* 2001, vol. 3, 82-90
- **BUCHBERGER et al.** *Biochemica,* 1996, vol. 2, 7-10
- **ZELLMER et al.** *Histochem Cell Biol,* 2001, vol. 115, 41-7
- **WIESENHOFER et al.** *J Neurosci Methods,* 1999, vol. 92, 145-52
- **STEPHAN et al.** *Hum Gene Ther,* 1996, vol. 7, 1803-12
- **LEWIS et al.** *Proc Natl Acad Sci USA,* 1996, vol. 93, 3176-81
- **BANERJEE et al.** *J Med Chem,* 2001, vol. 44, 4176-85

- **LEE et al.** *Gene Ther,* 2002, vol. 9, 859-66
- **SORGI et al.** *Gene Ther,* 1997, vol. 4, 961-8
- **TANG et al.** *Bioconjugate Chem.,* 1996, vol. 7, 703
- **OUAHABI et al.** *FEBS Lett,* 1997, vol. 414, 187-92
- **ANANDALAKSHMI, R. ; PRUSS, G. J. ; GE, X. ; MARATHE, R. ; MALLORY, A. C. ; SMITH, T. H. ; VANCE, V. B.** A Viral Suppressor of Gene Silencing in Plants. *Proc. Natl. Acad. Sci. USA,* 1998, vol. 95, 13079-13084
- **ANDERSSON, S. ; DAVIS, D. L. ; DAHLBÄCK, H. ; JÖRNVALL, H. ; RUSSELL, D. W.** Cloning, Structure, and Expression of the Mitochondrial Cytochrome P-450 Sterol 26-Hydroxylase, a Bile Acid Biosynthetic Enzyme. *J. Biol. Chem.,* 1989, vol. 264, 8222-8229
- **AUSUBEL, F. M. ; BRENT, R. ; KINGSTON, R. E. ; MOORE, D. D. ; SEIDMAN, J. G. ; SMITH, J. A. ; STRUHL, K.** Current Protocols in Molecular Biology. Greene Publishing Associates and Wiley-Interscience, 1994
- **BERNSTEIN, E. ; CAUDY, A. A. ; HAMMOND, S. M. ; HANNON, G. J.** Role for a Bidentate Ribonuclease in the Initiation Step of RNA Interference. *Nature,* 2001, vol. 409, 363-366
- **BILLY, E. ; BRONDANI, V. ; ZHANG, H. ; MULLER, U. ; FILIPOWICZ, W.** Specific Interference with Gene Expression Induced by Long, Double-Stranded RNA in Mouse Embryonal Teratocarcinoma Cell Lines. *Proc. Natl. Acad. Sci. USA,* 2001, vol. 98, 14428-14433
- **BOSHART, M. ; WEBER, F. ; JAHN, G. ; DORSCH-HÄSLER, K. ; FLECKENSTEIN, B. ; SCHAFFNER, W.** A Very Strong Enhancer is Located Upstream of an Immediate Early Gene of Human Cytomegalovirus. *Cell,* 1985, vol. 41, 521-530
- **BOSHER, J. M. ; LABOUESSE, M.** RNA Interference: Genetic Wand and Genetic Watchdog. *Nature Cell Biol.,* 2000, vol. 2, E31-E36
- **CAPLEN, N. J. ; FLEENOR, J. ; FIRE, A. ; MORGAN, R. A.** dsRNA-Mediated Gene Silencing in Cultured Drosophila Cells: A Tissue Culture Model for the Analysis of RNA Interference. *Gene,* 2000, vol. 252, 95-105
- **COGONI, C. ; MACINO, G.** Gene Silencing in Neurospora crassa Requires a Protein Homologous to RNA-Dependent RNA Polymerase. *Nature,* 1999, vol. 399, 166-169
- **COGONI, C. ; MACINO, G.** Isolation of Quelling-Defective (qde) Mutants Impaired in Posttranscriptional Transgene-Induced Gene Silencing in Neurospora crassa. *Proc. Natl. Acad. Sci. USA,* 1997, vol. 94, 10233-10238
- **COGONI, C. ; ROMANO, N. ; MACINO, G.** Suppression of Gene Expression by Homologous Transgenes. *Antonie Van Leeuwenhoek,* 1994, vol. 65, 205-209
- **FIRE, A. ; XU, S. ; MONTGOMERY, M. K. ; KOSTAS, S. A. ; DRIVER, S. E. ; MELLO, C. C.** Potent and Specific Genetic Interference by Double-Stranded RNA in Caenorhabditis elegans. *Nature,* 1998, vol. 391, 806-811
- **HAMMOND, S. M. ; BERNSTEIN, E. ; BEACH, D. ; HANNON, G. J.** An RNA-Directed Nuclease Mediates Genetic Interference in Caenorhabditis elegans. *Nature,* 2000, vol. 404, 293-296
- **HANNON, G. J.** RNA Interference. *Nature,* 2002, vol. 418, 244-251
- **INNIS, M. A. ; GELFAND, D. H. ; SNINSKY, J. J. ; WHITE, T. S.** PCR Protocols: A Guide to Methods and Applications. Academic Press, 1990
- **JONES, A. L. ; THOMAS, C. L. ; MAULE, A. J.** De novo Methylation and Co-Suppression Induced by a Cytoplasmically Replicating Plant RNA Virus. *EMBO J.,* 1998, vol. 17, 6385-6393
- **KAUFMAN, R. J.** Double-Stranded RNA-Activated Protein Kinase Mediates Virus-Induced Apoptosis: A New Role for an Old Actor. *Proc. Natl. Acad. Sci. USA,* 1999, vol. 96, 11693-11695
- **KENNERDELL, J. R. ; CARTHEW, R. W.** Use of dsRNA-Mediated Genetic Interference to Demonstrate that frizzled and frizzled 2 Act in the Wingless Pathway. *Cell,* 1998, vol. 95, 1017-1026
- **KETTING, R. F. ; FISCHER, S. E. ; BERNSTEIN, E. ; SIJEN, T. ; HANNON, G. J. ; PLASTERK, R. H.** Dicer Functions in RNA Interference and in Synthesis of Small RNA Involved in Developmental Timing in C. elegans. *Genes Dev.,* 2001, vol. 15, 2654-2659
- **LI, W. X. ; DING, S. W.** Viral Suppressors of RNA Silencing. *Curr. Opin. Biotechnol.,* 2001, vol. 12, 150-154
- **MISQUITTA, L. ; PATERSON, B. M.** Targeted Disruption of Gene Function in Drosophila by RNA Interference (RNAi): A Role for Nautilis in Embryonic Muscle Formation. *Proc. Natl. Acad. Sci. USA,* 1999, vol. 96, 1451-1456
- **NAPOLI, C. ; LEMIEUX, C. ; JORGENSEN, R.** Introduction of a Chalcone Synthase Gene into Petunia Results in Reversible Co-Suppression of Homologous Genes in trans. *Plant Cell,* 1990, vol. 2, 279-289
- **NELSON, J. A. ; REYNOLDS-KOHLER, C. ; SMITH, B. A.** Negative and Positive Regulation by a Short Segment in the 5'-Flanking Region of the Human Cytomegalovirus Major Immediate-Early Gene. *Molec. Cell. Biol.,* 1987, vol. 7, 4125-4129
- **NGO, H. ; TSCHUDI, C. ; GULL, K. ; ULLU, E.** Double-Stranded RNA Induces mRNA Degradation in Trypanosoma brucei. *Proc. Natl. Acad. Sci. USA,* 1998, vol. 95, 14687-14692
- **NYKANEN, A. ; HALEY, B. ; ZAMORE, P. D.** ATP Requirements and Small Interfering RNA Structure in the RNA Interference Pathway. *Cell,* 2001, vol. 107, 309-321

- **PLASTERK, R. H. A. ; KETTING, R. F.** The Silence of the Genes. *Curr. Opin. Genet. Dev.,* 2000, vol. 10, 562-567
- **ROMANO, N. ; MACINO, G.** Quelling: Transient Inactivation of Gene Expression in Neurospora crassa by Transformation with Homologous Sequences. *Mol. Microbiol.,* 1992, vol. 6, 3343-3353
- **SHUMAN, S.** Novel Approach to Molecular Cloning and Polynucleotide Synthesis Using Vaccinia DNA Topoisomerase. *J. Biol. Chem.,* 1994, vol. 269, 32678-32684
- **SHUMAN, S.** Recombination Mediated by Vaccinia Virus DNA Topoisomerase I in Escherichia coli is Sequence Specific. *Proc. Natl. Acad. Sci. USA,* 1991, vol. 88, 10104-10108
- **SMITH, C. J. ; WATSON, C. F. ; BIRD, C. R. ; RAY, J. ; SCHUCH, W. ; GRIERSON, D.** Expression of a Truncated Tomato Polygalacturonase Gene Inhibits Expression of the Endogenous Gene in Transgenic Plants. *Mol. Gen. Genet.,* 1990, vol. 224, 477-481
- **SVOBODA, P. ; STEIN, P. ; HAYASHI, H. ; SCHULT, R. M.** Selective Reduction of Dormant Maternal mRNAs in Mouse Oocytes by RNA Interference. *Development,* 2000, vol. 127, 4147-4156
- **VAN DER KROL, A. R. ; MUR, L. A. ; BELD, M. ; MOL, J. N. ; STUITJE, A. R.** Flavonoid Genes in Petunia: Addition of a Limited Number of Gene Copies May Lead to a Suppression of Gene Expression. *Plant Cell,* 1990, vol. 2, 291-299
- **VOINNET, O. ; PINTO, Y. M. ; BAULCOMBE, D. C.** Suppression of Gene Silencing: A General Strategy Used by Diverse DNA and RNA Viruses of Plants. *Proc. Natl. Acad. Sci. USA,* 1999, vol. 96, 14147-14152
- **WIANNY, F. ; ZERNICKA-GOETZ, M.** Specific Interference with Gene Function by Double-Stranded RNA in Early Mouse Development. *Nature Cell Biol.,* 2000, vol. 2, 70-75
- **YANG, S. ; TUTTON, S. ; PIERCE, E. ; YOON, K.** Specific Double-Stranded RNA Interference in Undifferentiated Mouse Embryonic Stem Cells. *Mol. Cell. Biol.,* 2001, vol. 21, 7807-7816
- **ZAMORE, P. D.** RNA Interference: Listening to the Sound of Silence. *Nat. Struct. Biol.,* 2001, vol. 8, 746-750
- **CZAUDEMA et al.** *Nucleic Acids Research,* 2003
- **KAUFMAN, R.J.** *Proc Natl. Acad. Sci. USA,* 1999, vol. 96, 11693-11695
- **DENLI, A.M. ; HANNON, G.J.** *Trends Biochem. Sci.,* 2003, vol. 28, 196-201
- **CARRINGTON ,J.C. ; AMBROS, V.** *Science,* 2003, vol. 301, 336-338
- **SLEDZ, C.A et al.** *Nat Cell Biol.,* 2003, vol. 5, 834-839
- **ILLANGASEKARE, M. ; YARUS, M.** *RNA.,* 1999, vol. 5, 1482-1489
- **ELBASHIR, S.M. et al.** *Nature,* 2001, vol. 411, 494-498
- **CZAUDEMA, F. et al.** *Nucleic Acids Res.,* 2003, vol. 31, 2705-2716
- **ELBASHIR, S.M. et al.** *EMBO J.,* 2001, vol. 20, 6877-6888

SEQUENCE LISTING

<110> Invitrogen Corporation

<120> Composition and Methods for Preparing Short RNA Molecules and Other Nucleic Acids

<130> 0942.563PC02

<140> 04779463.1
<141> 2004-07-30

<150> 60/491,758
<151> 2003-08-01

<150> 60/520,383
<151> 2003-11-17

<160> 16

<170> PatentIn version 3.2

<210> 1
<211> 24
<212> DNA
<213> Artificial Sequence

<220>
<223> Primer T7amp1

<400> 1
gatgactcgt aatacgactc acta 24

<210> 2
<211> 20.
<212> DNA
<213> Artificial Sequence

<220>.
<223> Primer lacZ-fwd2

<400> 2
accagaagcg gtgccggaaa 20

<210> 3
<211> 20
<212> DNA
<213> Artificial Sequence

<220>
<223> Primer lacZ-rev2

<400> 3
ccacagcgga tggttcggat 20

<210> 4
<211> 20
<212> DNA
<213> Artificial Sequence

<220>
<223> Primer LucFor2

<400> 4
tgaacatttc gcagcctacc 20

<210> 5
<211> 20
<212> DNA
<213> Artificial Sequence

<220>
<223> Primer LucRev2

<400> 5
ggggccacct gatatccttt 20

<210> 6
<211> 20
<212> DNA
<213> Artificial Sequence

<220>
<223> Primer laminAC-fwd

<400> 6
aggagaagga ggacctgcag 20

<210> 7
<211> 20
<212> DNA
<213> Artificial Sequence

<220>
<223> Primer laminAC-rev

<400> 7
agaagctcct ggtactcgtc 20

<210> 8
<211> 45
<212> DNA
<213> Artificial Sequence

<220>
<223> Primer 3 to generate lacZ dsRNA fragments

<400> 8
gactcgtaat acgactcact atagggacca gaagcggtgc cggaaa 46

<210> 9
<211> 45
<212> DNA
<213> Artificial Sequence

<220>
<223> Primer 4 to generate lacZ dsRNA fragments

<400> 9
gactcgtaat acgactcact atagggccac agcggatggt tcggat 46

<210> 10
<211> 17
<212> DNA
<213> Artificial Sequence

<220>
<223> Primer 6 to generate lacZ dsRNA fragments

<400> 10
gccacctgat atccttt 17

<210> 11
<211> 46
<212> DNA
<213> Artificial Sequence

<220>
<223> Primer 7 to generate lacZ dsRNA fragments

<400> 11
gactcgtaat acgactcact atagggtgaa catttcgcag cctacc 46

<210> 12
<211> 43
<212> DNA
<213> Artificial Sequence

<220>
<223> Primer 8 to generate lacZ dsRNA fragments

<400> 12
gactcgtaat acgactcact ataggggcca cctgatatcc ttt 43

<210> 13
<211> 18
<212> DNA
<213> Artificial Sequence

<220>
<223> Primer 9 to generate lacZ dsRNA fragments

<400> 13
gcatcgtaac cgtgcatc 18

<210> 14
<211> 16
<212> DNA
<213> Artificial Sequence

<220>
<223> Primer 10 to generate lacZ dsRNA fragments

<400> 14
gcgagtgcca acatgg 16

<210> 15
<211> 47
<212> DNA
<213> Artificial Sequence

<220>
<223> Primer 11 to generate lacZ dsRNA fragments

<400> 15
gactcgtaat acgactcact ataggtactg catcgtaacc gtgcatc 47

<210> 16
<211> 45
<212> DNA
<213> Artificial Sequence

<220>
<223> Primer 12 to generate lacZ dsRNA fragments

<400> 16
gactcgtaat acgactcact ataggtactg cgagtggcaa catgg 45